# EUROPEAN PATENT APPLICATION

(11) **EP 4 737 448 A1**
(43) Date of publication of application: **06.05.2026**
(21) Application number: 24830675.5
(22) Date of filing: 24.06.2024
(51) Int. Cl.: C07D 401/10, C07D 401/14, A61K 31/4178, A61P 9/00

(54) **SARM1 ENZYME ACTIVITY INHIBITOR AND USE THEREOF**

(30) Priority: 28.06.2023 CN 202310781783
(71) Applicant: Artivila (ShenZhen) Innovation Center, Ltd., Guangdong, 518172 (CN)
(72) Inventor: NIU, Deqiang, Shenzhen, Guangdong 518172 (CN); ZHU, Zhendong, Shenzhen, Guangdong 518172 (CN); FAN, Ming, Shenzhen, Guangdong 518172 (CN); ZHENG, Zihua, Shenzhen, Guangdong 518172 (CN)
(74) Representative: Boult Wade Tennant LLP
(86) International application number: PCT/CN2024/100860
(87) International publication number: WO 2025/002030

(57) **Abstract**

Provided in the present invention is the use of an SARMI enzyme activity inhibitor in treatment of neurodegenerative diseases or neurological diseases or disorders. Particularly provided in the present invention are compounds of formula (I) serving as SARMI enzyme activity inhibitors and a pharmaceutical composition thereof

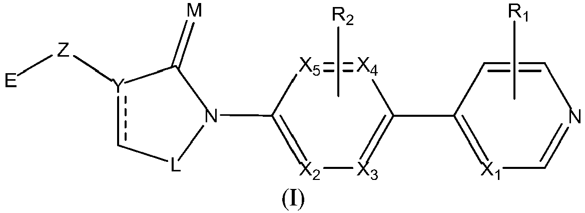

## Description

### FIELD

The present disclosure relates to a compound for inhibiting SARM1 enzymatic activity, and/or use of the compound in treating and/or preventing a neurodegenerative or neurological disease or condition associated with SARM1 enzymatic activity.

### BACKGROUND

Neurodegenerative diseases are a type of disorder that can seriously harm human health. They may result in a progressive disease, which can cause devastating damage, such as death of nerve cells. Central nervous system diseases such as Alzheimer's disease, Parkinson's disease, amyotrophic lateral sclerosis (ALS) and Huntington's disease, and peripheral neurological diseases such as diabetic peripheral neuropathy (DPN) are already known to be the major neurodegenerative diseases. Most of these are related to aging. Actually, the onset risk of these diseases increases with age. However, they also affect middle-aged people or even younger people.

With deepening researchinto the brain's structure and function, the roles of neurotransmitters and neurotrophic factors have been gradually elucidated, but many local causes of neurodegeneration remain unclear. Only for Parkinson's disease, the relationship between the disease and dopamine (a special neurotransmitter) has been clarified, and L-dopa(a precursor of dopamine) has been used as a drug to alleviate neurological symptoms and restore neurological functions. However, L-dopa cannot prevent the progression of neurodegeneration, and gradually loses effectiveness as the disease advances, leading to the degeneration and defects of dopamine-based nerve cells. Similarly, Alzheimer's disease is also caused by the degeneration and defects of various nerve cells, including acetylcholine-based nerve cells and monoamine-based nerve cells. As drugs to treat this disease, cholinesterase inhibitors have been put on the market or are in the process of being developed. However, L-dopa for treating Parkinson's disease is still limited to symptomatic treatment to temporarily improve neurological symptoms.

Therefore, there is currently a lack of effective therapeutic drugs for neurodegenerative diseases.

It has been found from studies that nerve axon damage occurs in a variety of neurological diseases such as neurodegenerative diseases and accidental injuries. Axonal degeneration can cause structural necrosis and dysfunction of the peripheral nervous system, ultimately leading to acquired or inherited degenerative diseases of the central nervous system.

Although there is currently no highly effective pharmacological method that can accurately assess the weight ofthe incidence rate of the disease by axonal degeneration, it has been found in histopathological studies that significant axonal damage and degradation are observed in the early stages of various neuropathies such as Alzheimer's disease, Parkinson's disease, multiple sclerosis, amyotrophic lateral sclerosis and peripheral neuropathy, indicating that axonal degeneration plays an important role in the development of neuropathy (Fischer et al., Neuro-degenerative Diseases, 2007, 4:431-442). Therefore, maintaining the integrity of neuronal structure and function by attenuating or even blocking axonal degeneration may be a therapeutic method that can benefit a variety of neurological diseases.

In the absence of effective therapeutic drugs for neurodegenerative diseases in the prior art, the research and development of new compounds is urgently required, especially small chemical molecules, including compounds that have effect on neural axon degeneration.

### SUMMARY

After long-term research, the inventors unexpectedly found a compound with significant inhibitory effect on SARM1 enzymatic activity, and found that the compound can improve axonal degeneration and be used to treat or prevent neurodegenerative diseases and related conditions.

SARM1 (Sterile alpha and TIR motif containing 1) consists of three domains, namely ARM (Armadillo/HEAT repeat) domain at the N-terminal, two tandem SAM (Sterile alpha motif) domains, and TIR (Toll/Interleukin receptor) domain at the C-terminal. In addition, there is also a mitochondrial targeting signal peptide at the N-terminal.

It is known that in wild-type neurons, axonal injury induces NAD⁺ depletion and axonal degeneration. Knocking out SARM1 in neurons can inhibit axonal degeneration, and maintain NAD⁺ at normal levels, indicating that SARM1 promotes NAD⁺ consumption and aggravates axonal degeneration.

Milbrandt's research group at Washington University School of Medicine, USA, has synthesized TIR domain of SARM1 (SARM1-TIR) and found that it has NAD⁺ hydrolase activity. High-purity SARM1-TIR was further obtained through strict protein expression and purification in E. coli and a cell-free protein expression system. It has been finally demonstrated that SARM1-TIR can catalyze NAD⁺ to produce adenosine 5'-diphosphate ribose (ADPR) and cyclic adenosine 5'-diphosphate ribose (cADPR).

SARM1 is a multifunctional signaling enzyme that can catalyze a variety of substrates such as NAD⁺, NADP⁺ and NA to generate signaling molecules such as cADPR, ADPR and NAADP. In a variety of neurodegenerative diseases, SARM1 is activated, which leads to NAD⁺ depletion and thereby initiates a distinct mechanism of cell death. Knocking out SARM1 can inhibit axonal degeneration and disease progression. Therefore, SARM1 is considered a potential drug target for related neurological diseases, including TBI (traumatic brain injury), AD (Alzheimer's disease), CIPN (chemotherapy induced peripheral neuropathy) and ALS (amyotrophic lateral sclerosis).

In the present disclosure, the inventor produces full-length SARM1 for NADase activity assays and for screening to obtain compounds herein that inhibit SARM1 enzymatic activity.

Therefore, based on the above findings, in a first aspect, the present disclosure provides use of a SARM1 enzymatic activity inhibitor in the manufacture of a medicament for treating or preventing a neurodegenerative disease or a neurological disease or condition.

In another aspect, the present disclosure provides use of a SARM1 enzymatic activity inhibitor in the manufacture of a medicament for treating or preventing an axonal degeneration-related disease or condition.

In particular, the present disclosure provides a compound represented by formula I that serves as a SARM1 enzymatic activity inhibitor, or a racemate, enantiomer, diastereoisomer, deuterated compound or pharmaceutically acceptable salt thereof: 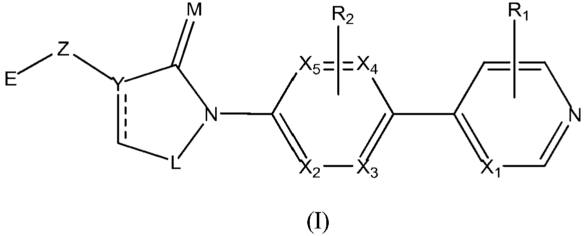 wherein,
X₁, X₂, X₃, X₄ and X₅ are each independently selected from the group consisting of CH and N;
Z represents -CH₂-, -CH₂(CH₃)- or -O-; 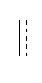 represents a single bond or a double bond;
Y represents a carbon atom or a nitrogen atom;
R₁ is independently selected from the group consisting of H, halogen, C₁-C₃ alkyl, cyano, trifluoromethyl, amino, hydroxy, methoxy and -C(O)NH₂, preferably selected from the group consisting of H, F, Cl, methyl, cyano, and -C(O)NH₂;
R₂ is independently selected from the group consisting of H, halogen, C₁-C₃ alkyl, cyano, trifluoromethyl, amino, hydroxy, methoxy, C₁-C₃ alkyl-OC(O)- and C₁-C₃ alkyl-NHC(O)-, preferably selected from the group consisting of H, cyano, Cl, methyl and methoxy;
M represents O or S;
L is selected from the group consisting of -CH₂-CH₂-, -CH₂-, -O- and -CO-, wherein either -CH₂- moiety in -CH₂-CH₂- is optionally substituted by -O-, -S-, -CO- or -NH-; preferably, L is selected from the group consisting of -CH₂-, -CH₂-CH₂-, -CH-O-, -O- and -CO-; and
E is selected from the group consisting of 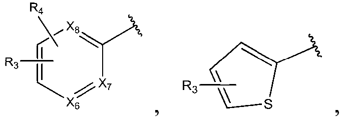 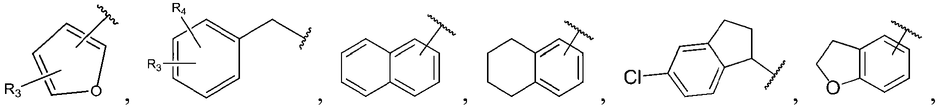 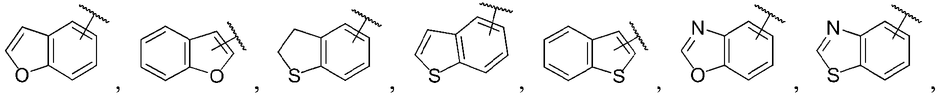 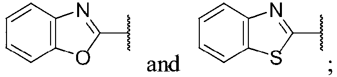
wherein, X₆, X₇ and X₈ are each independently selected from the group consisting of CH and N,
R₃ is independently selected from the group consisting of H, halogen, methyl, cyano, trifluoromethyl and nitro, preferably selected from the group consisting of H, F, Cl, Br, cyano, trifluoromethyl and nitro, and
R₄ is independently selected from the group consisting of H, halogen, methyl, cyano, trifluoromethyl and nitro, preferably selected from the group consisting of H, F, Cl, Br, cyano, trifluoromethyl and nitro.

In some embodiments, R₃ and R₄ are each independently selected from the group consisting of H, halogen, methyl, cyano, trifluoromethyl, nitro, acetyl, methoxycarbonyl, carboxyl, ethynyl, ClCH=CH- and

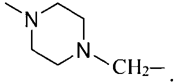

In some preferred embodiments, E is selected from the group consisting of 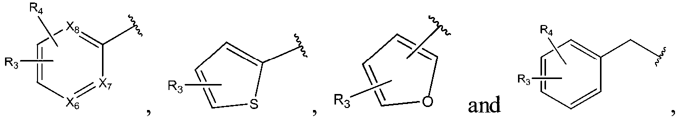 wherein X₆, X₇, X₈, R₃ and R₄ are as defined above.

In some preferred embodiments, the compound represented by formula (I) has a structure represented by formula (II): 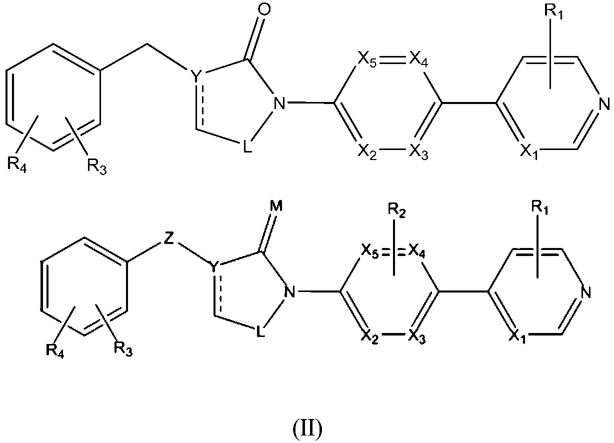 wherein X₁, X₂, X₃, X₄, X₅, L, Y, R₁, R₃ and R₄ are as defined above.

In some preferred embodiments, the compound of the present disclosure is a compound selected from the group consisting of: 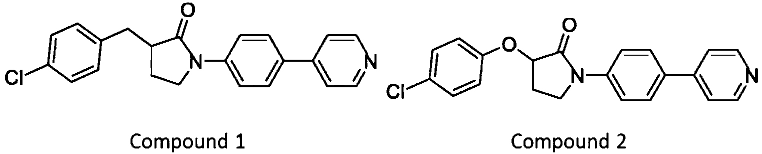 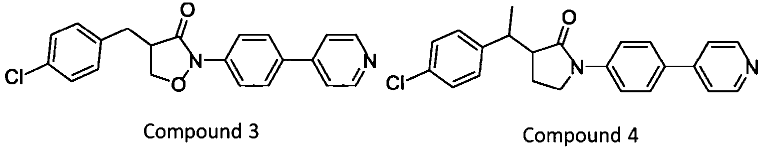 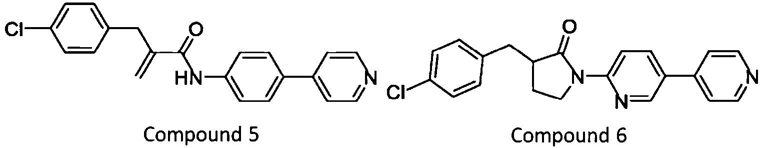 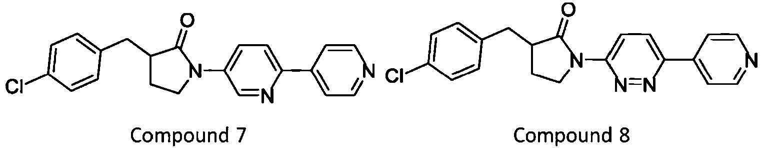 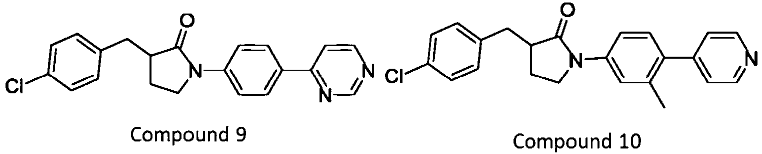 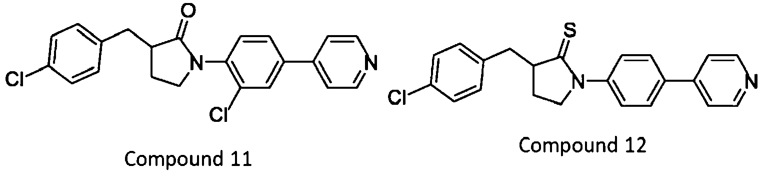 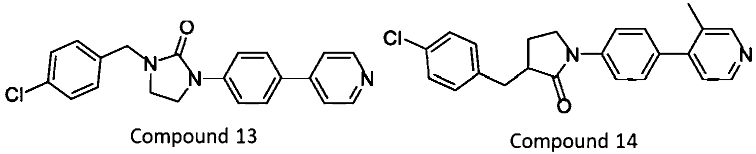 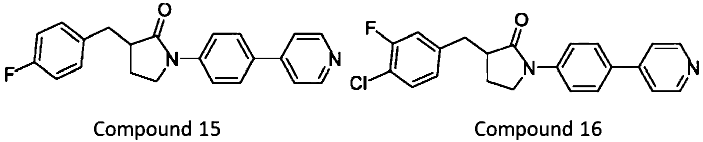 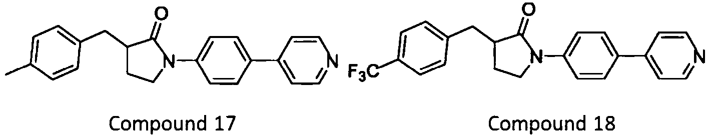 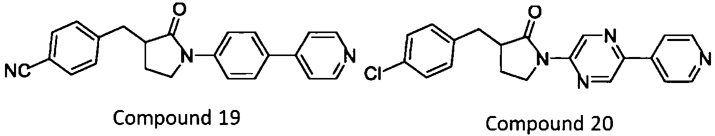 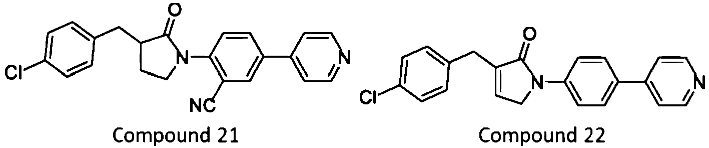 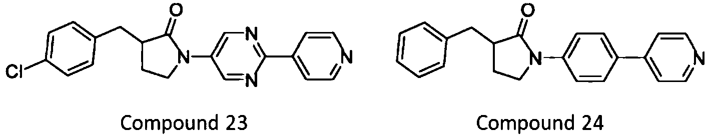 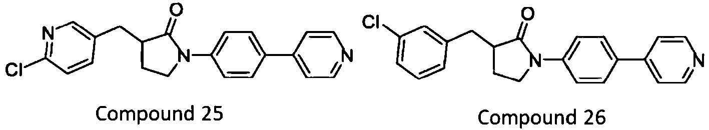 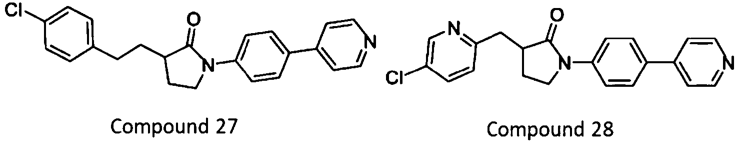 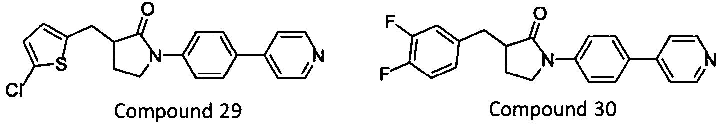 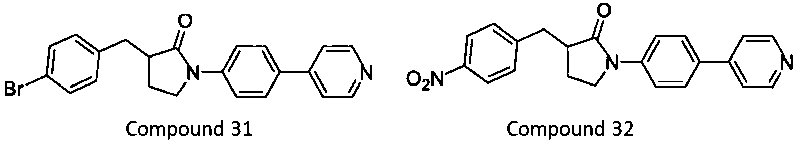 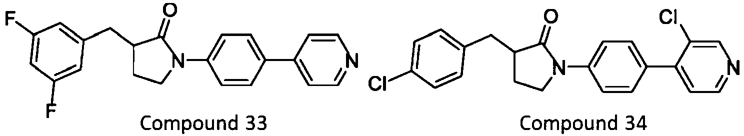 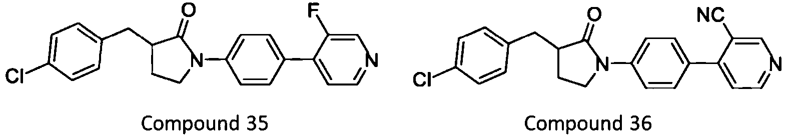 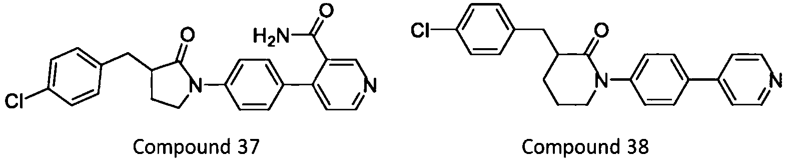 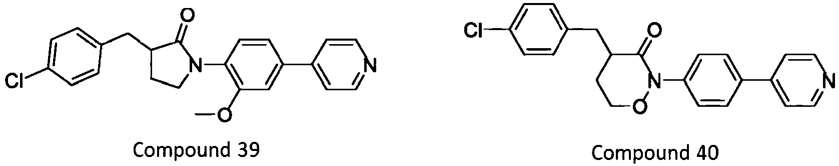 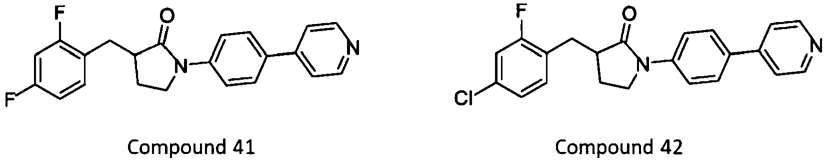 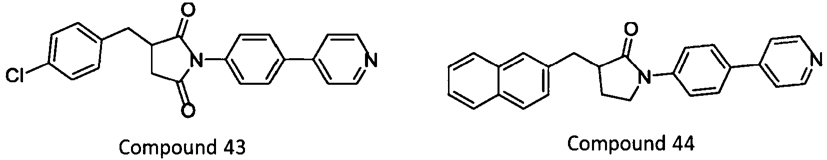 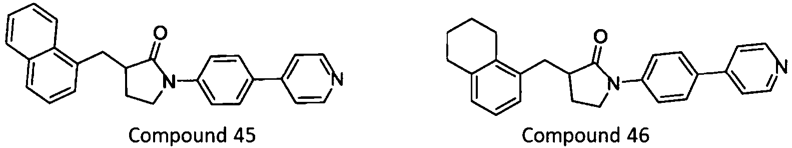 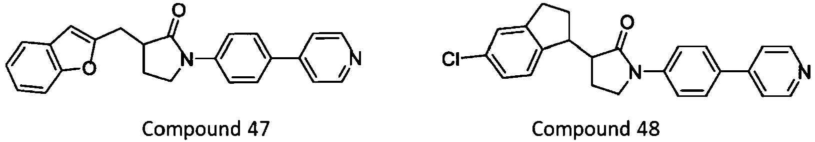 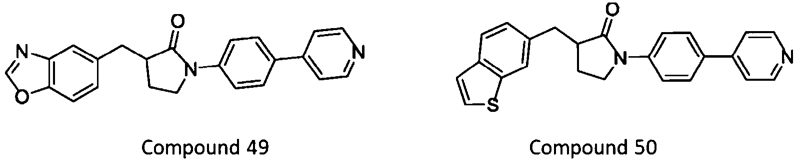 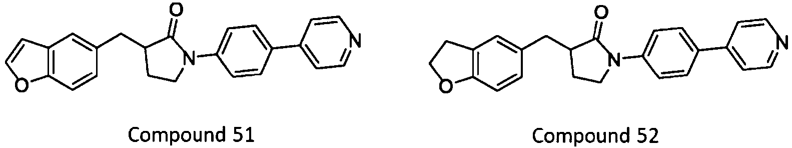 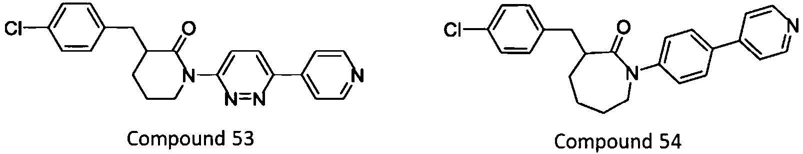 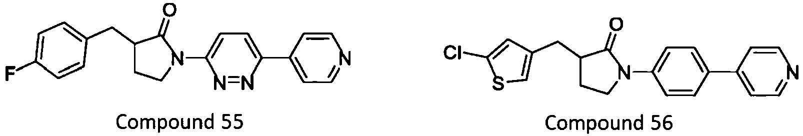 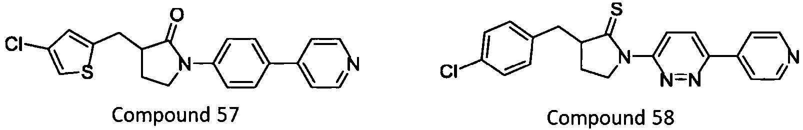 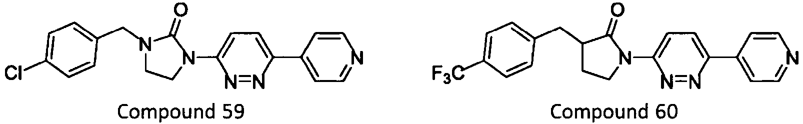 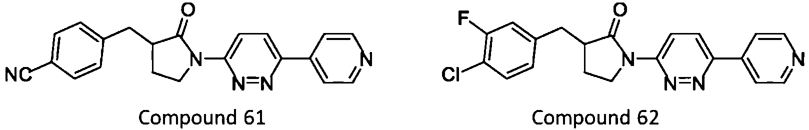 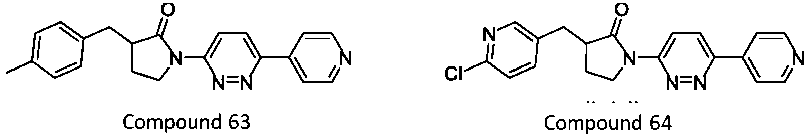 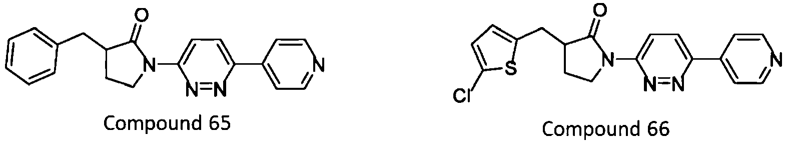 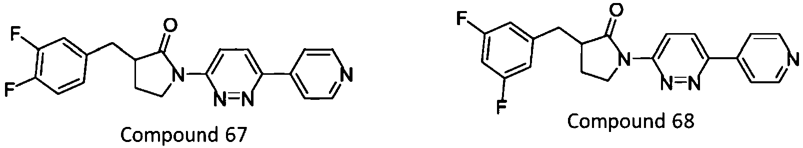 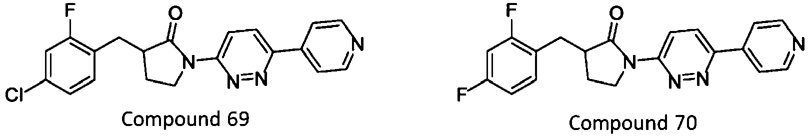 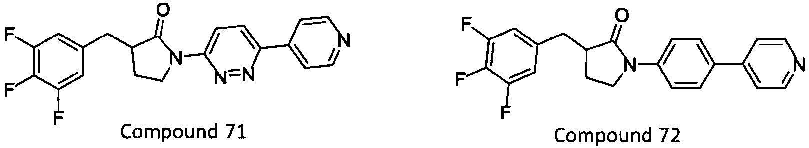 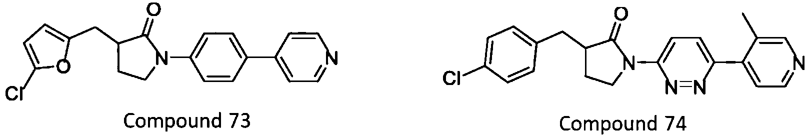 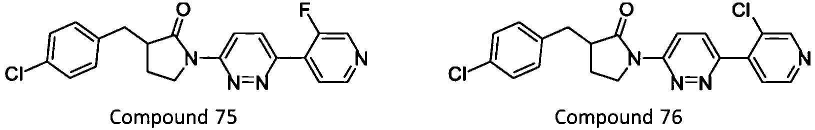 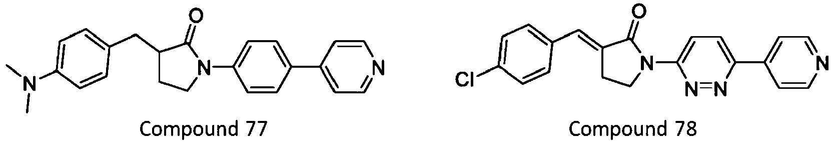 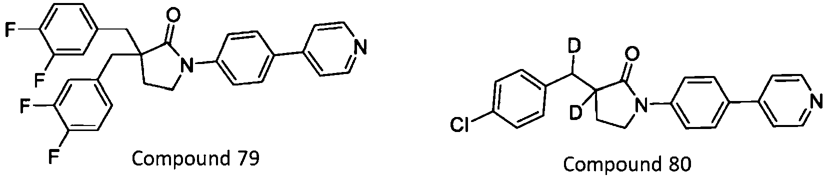 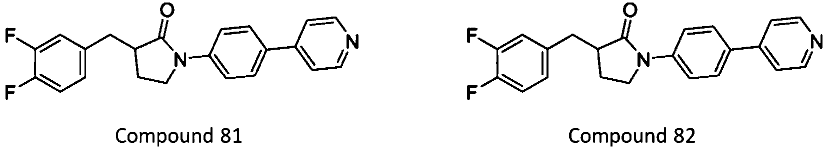 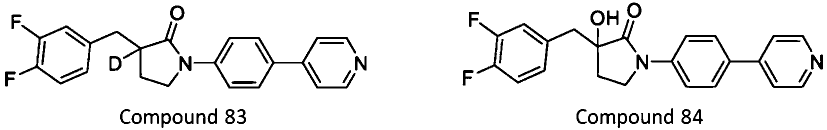 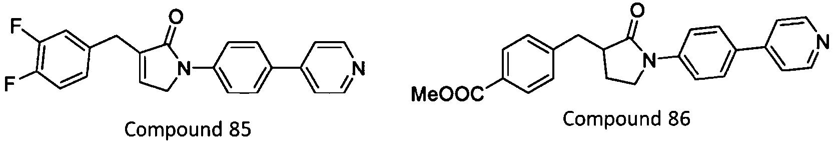 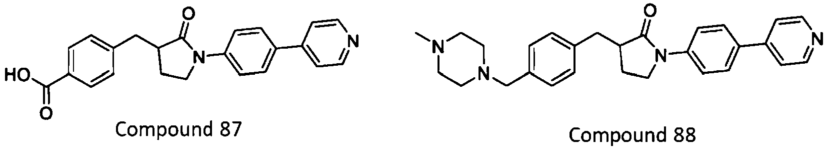 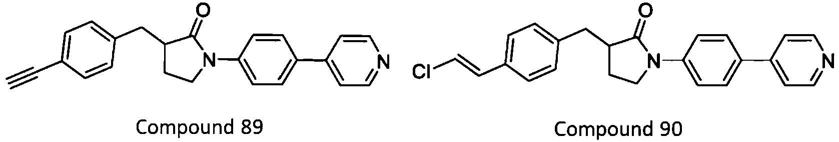 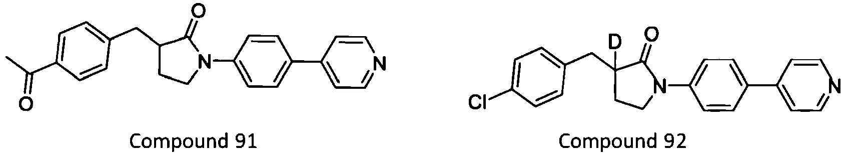 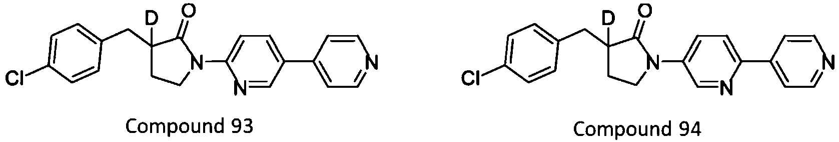 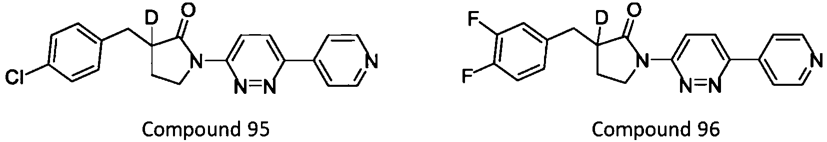 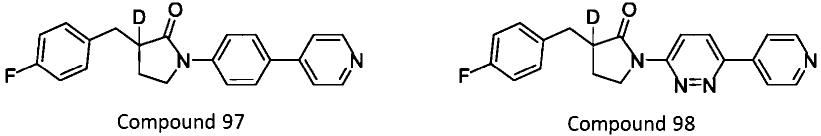 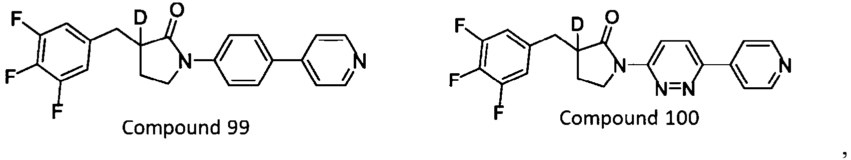 or a racemate, enantiomer, diastereoisomer, deuterated compound or pharmaceutically acceptable salt thereof

As used herein, when reference is made to the compounds represented by formula (I) or formula (II), it also includes pharmaceutically acceptable salts or stereoisomers of the compounds represented by formula (I) or formula (II).

The present disclosure further relates to a method for treating or preventing a neurodegenerative disease or a neurological disease or condition associated therewith, comprising administering to a subject in need thereof the compound of the present disclosure as a SARM1 enzymatic activity inhibitor. In particular, the present disclosure relates to a method for treating or preventing an axonal degeneration-related disease or condition, comprising administering to a subject in need thereof the compound of the present disclosure as a SARM1 enzymatic activity inhibitor. More particularly, the present disclosure relates to a method for inhibiting SARM1 enzymatic activity, comprising administering to a subject in need thereof the compound of the present disclosure. More particularly, the present disclosure relates to a method for inhibiting axonal degeneration, comprising administering to a subject in need thereof the compound of the present disclosure. The compound or composition of the present disclosure can be administered to a subject or patient in need thereof in an effective amount.

Accordingly, the present disclosure further relates to use of the compound of the present disclosure or a pharmaceutically acceptable salt or stereoisomer thereof in the manufacture of a medicament for treating or preventing a neurodegenerative disease or a neurological disease or condition. The present disclosure further relates to use of the compound of the present disclosure or a pharmaceutically acceptable salt or stereoisomer thereof in the manufacture of a SARM1 enzymatic activity inhibitor. The present disclosure further relates to use of the compound of the present disclosure or a pharmaceutically acceptable salt or stereoisomer thereof in the manufacture of a medicament for treating or preventing an axonal degeneration-related disease or condition. Preferably, the neurodegenerative disease, neurological disease or condition, or the axonal degeneration-related disease or condition is selected from the group consisting of Alzheimer's disease, Parkinson's disease, multiple sclerosis, amyotrophic lateral sclerosis and peripheral neuropathy.

### DETAILED DESCRIPTION

### Terms

As used herein, reference to a "compound" having a particular structural formula generally also encompasses pharmaceutically acceptable salts, stereoisomers, diastereoisomers, enantiomers, racemic mixtures and isotope derivatives thereof

The pharmaceutically acceptable salt of the present disclosure may be formed using, for example, the following inorganic or organic acids. "Pharmaceutically acceptable salts" refer to salts which, within the scope of reasonable medical judgment, are suitable for contact with humans and mammals tissue without undue toxicity, irritation, allergic reactions, etc., and have a reasonable benefit/risk ratio. The salts may be prepared in situ during the final isolation and purification of the compounds of the present disclosure, or prepared separately by reacting a free base or free acid with a suitable reagent. For example, a free base can be reacted with a suitable acid. Examples of pharmaceutically acceptable acid addition salts are salts formed by amino (amine) with inorganic acids (such as hydrochloric acid, hydrobromic acid, phosphoric acid, sulfuric acid, and perchloric acid) or organic acids (such as acetic acid, oxalic acid, maleic acid, tartaric acid, citric acid, succinic acid or malonic acid), or salts formed by using other methods in the art such as ion exchange. Other pharmaceutically acceptable salts include sodium alginate, ascorbate, benzenesulfonate, adipate, camphorsulfonate, aspartate, benzoate, bisulfate, borate, butyrate, camphorate, citrate, dodecyl sulfate, ethanesulfonate, formate, fumarate, glucoheptonate, glycerophosphate, gluconate, enanthate, hexanoate, hydroiodide, lactobionate, lactate, laurate, lauryl sulfate, malate, maleate, malonate, methanesulfonate, 2-naphthalenesulfonate, nicotinate, nitrate, oleate, oxalate, palmitate, pamoate, pectate, persulfate, 3-phenylpropionate, phosphate, picrate, pivalate, propionate, stearate, succinate, sulfate, tartrate, thiocyanate, p-toluenesulfonate, undecanoate, valerate, etc.

The pharmaceutically acceptable salt of the present disclosure can be prepared by conventional methods, for example, by dissolving the compound of the present disclosure in a water-miscible organic solvent (such as methanol, ethanol, acetone, and acetonitrile), adding an excess amount of organic acid or inorganic acid aqueous solution thereto, to precipitate salt from the resulting mixture, removing the solvent and the remaining free acid therefrom, and isolating the precipitated salt.

"Stereoisomerism" as used herein is divided into conformational isomerism and configurational isomerism. Configurational isomerism can further be divided into cis-trans isomerism and optical isomerism. Conformational isomerism refers to a stereoisomerism phenomenon in which each atom or group of atoms of an organic molecule with a certain configuration are arranged differently in space due to the rotation or distortion of carbon-carbon single bonds. Common structures include alkanes and cycloalkanes, such as the chair conformation and boat conformation that appear in the cyclohexane structure. "Stereoisomer" means that the compound of the present disclosure contains one or more asymmetric centers, which is thus available to be a racemate and racemic mixture, single enantiomer, diastereomeric mixture and single diastereomer. The compound of the present disclosure can have an asymmetric center, and each asymmetric center will produce two optical isomers. The scope of the present disclosure includes all possible optical isomers and diastereomeric mixtures and pure or partially pure compounds.

In some cases, the compound of the present disclosure may present as a tautomer having different positions of attaching to hydrogen by displacement of one or more double bonds. For example, a ketone and its enol form are keto-enol tautomers. Amide and imine can present a tautomeric form. Each tautomer and mixtures thereof are included in the compound of the present disclosure. All enantiomers, diastereomers, racemates, mesomers, cis-trans isomers, tautomers, mixtures thereof, etc. are encompassed within the scope of the present disclosure.

The compound of the present disclosure may be used in combination with an additional SARM1 enzymatic activity inhibitor for treating or preventing a neurodegenerative disease or a related neurological disease or condition, or may be used in combination with an additional active drug for treating or preventing a neurodegenerative disease or a related neurological disease or condition, for treating or preventing a neurodegenerative disease or a related disease or condition.

As used herein, 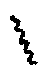 represents a position at which a group or substituent group is attached to a parent compound.

The compound of the present disclosure, or the mesomer, racemate, enantiomer, diastereomer or pharmaceutically acceptable salt thereof can be administered orally or parenterally as an active ingredient, at an effective amount ranging from 0.1 to 2000 mg/kg body weight/day, preferably, 0.1 to 100 mg/kg body weight/day in the case of mammals including humans (body weight about 70 kg), in single or divided doses per day, or with/without following a predetermined time. The administration amount of the active ingredient may be adjusted based on a number of relevant factors such as the condition of the subject to be treated, the type and severity of the disease, the rate of administration and physician opinion. In some cases, amounts less than the above amounts may be appropriate.

The compounds of the present disclosure, or the mesomer, racemate, enantiomer, diastereomer, or pharmaceutically acceptable salt thereof can be prepared into a form of pharmaceutical composition. The pharmaceutical composition may comprise the compound of the present invention, or the mesomer, racemate, enantiomer, diastereomer, or pharmaceutically acceptable salt thereof, and a pharmaceutically acceptable carrier.

The pharmaceutical composition of the present disclosure can be formulated into a dosage form for oral administration or parenteral administration (including intramuscular administration, intravenous and subcutaneous administration, and intratumoral injection) according to any of conventional methods, such as tablets, granules, powders, capsules, syrups, emulsions, microemulsions, solutions or suspensions.

The pharmaceutical composition of the present disclosure for oral administration can be prepared by mixing the active ingredient with a pharmaceutically acceptable carrier, for example, cellulose, calcium silicate, magnesium stearate, calcium stearate, corn starch, lactose, sucrose, dextrose, calcium phosphate, stearic acid, surfactants, suspending agents, gelatin, talc, emulsifiers or diluents. Examples of carriers used in the injectable compositions of the present disclosure include water, glycerides, salt solutions, alcohols, glycols, glucose solutions, ethers (such as polyethylene glycol 400), oils, fatty acids, fatty acid esters, surfactants, suspending agents and emulsifiers.

Unless otherwise stated, mass spectrometry, nuclear magnetic resonance, HPLC, protein chemistry, biochemistry, recombinant DNA technology, and pharmacological conventional methods are used. In the present disclosure, "or" or "and" are used to mean "and/or" unless stated otherwise.

In the description and claims, a given chemical formula or name will cover all stereo and optical isomers as well as racemates in which the above isomers are present. Unless otherwise indicated, all chiral (enantiomeric and diastereomeric) and racemic forms are within the scope of the present disclosure. Many geometric isomers of C=C double bonds, C=N double bonds, ring systems, etc., may also exist in the compounds, and all the above stable isomers are encompassed by the present disclosure.

The compounds of the present disclosure can be isolated in optically active or racemic forms. All methods for preparing the compounds of the present disclosure and the intermediates prepared therein are considered to be part of the present disclosure. In the preparation of enantiomeric or diastereomeric products, they can be isolated by conventional methods, for example by chromatography or fractional crystallization. It should be understood that all isomeric forms which may exist are included in the present disclosure.

Unless otherwise defined, when a substituent is designated as "optionally substituted", the substituent is selected from, for example, substituents such as alkyl, cycloalkyl, aryl, heterocyclyl, halogen, hydroxyl, alkoxy, nitro, cyano, oxo, alkanoyl, aryloxy, alkanoyloxy, amino, alkylamino, arylamino, and alkylthio.

The term "alkyl" or "alkylene" as used herein is intended to include both branched and straight chain saturated aliphatic hydrocarbon groups having the specified number of carbon atoms. The alkyl in the present disclosure is preferably C₁-C₁₂ alkyl, C₁-C₁₀ alkyl, C₁-C₈ alkyl, more preferably C₁-C₆ alkyl, particularly preferably C₁-C₄ alkyl, particularly C₁-C₃ alkyl. For example, "C₁-C₆ alkyl" represents an alkyl having 1 to 6 carbon atoms. Examples of alkyl include, but are not limited to, methyl (Me), ethyl (Et), propyl (such as n-propyl and isopropyl), butyl (such as n-butyl, isobutyl and tert-butyl), and pentyl (such as n-pentyl, isopentyl and neopentyl). In the C₁-C₁₂ alkyl in the present disclosure, 1 to 4 -CH₂- units are optionally replaced by O atoms, S atoms or -NH-.

The term "carbonyl" refers to an organic functional group consisting of two atoms of carbon and oxygen connected by a double bond (C=O or C(O)).

The term "aromatic ring" refers to an aromatic ring structure. A carbocyclic aromatic ring refers to a carbocyclic aromatic ring structure in which all ring members are carbon atoms. A carbocyclic non-aromatic ring refers to a carbocyclic non-aromatic ring structure in which all ring members are carbon atoms.

The term "halogen" or "halo" includes fluorine, chlorine, bromine or iodine, preferably fluorine or chlorine.

The term "heterocycle" refers to a monocyclic heterocyclic alkyl system or bicyclic heterocyclic alkyl system, which may also include spiroheterocyclic or bridged heterocyclic alkyl. A monocyclic heterocyclic alkyl refers to a 3- to 8-membered, saturated or unsaturated, non-aromatic cyclic alkyl system containing at least one heteroatom selected from the group consisting of O, N, S and P. A bicyclic heterocyclic alkyl system refers to a heterocyclic alkyl fused to a phenyl, cycloalkyl, cycloalkylene, heterocyclic alkyl or heteroaryl. An aromatic heterocycle refers to an aromatic heterocyclic system, and a non-aromatic heterocycle refers to a non-aromatic heterocyclic system.

When any variable occurs more than once in any composition or formula of a compound, its definition on each occurrence is independent of its definition on every other occurrence. Thus, for example, if a group is shown substituted with 0-3 R, then the group may be optionally substituted with up to three R groups, with R on each occurrence being independently selected from the definition of R. Furthermore, combinations of substituents and/or variables are permitted only if such combinations result in stable compounds.

As used herein, the term "effective amount" refers to an amount of a drug or agent (i.e., the compound of the present disclosure) that will elicit the biological or medical response in a tissue, system, animal, or human that is sought by, for example, a researcher or clinician. Furthermore, the term "therapeutically effective amount" refers to an amount that results in improved treatment, cure, prevention, or alleviation of a disease, condition, or side effect, or reduction in rate at which the disease or condition progresses, compared to a corresponding subject that does not receive such amount. An effective amount may be administered in one or more administrations, applications or doses and is not intended to be limited by a particular formulation or route of administration. The term further includes an amount effective to enhance normal physiological functions within its scope.

The term "treatment" as used herein includes any effect resulting in amelioration of a condition, disease, disorder, etc., such as alleviation, reduction, regulation, amelioration or elimination, or compromise of symptoms thereof

The term "pharmaceutically acceptable" as used herein refers to those compounds, substances, compositions and/or dosage forms which, within the scope of reasonable medical judgment, are suitable for use in contact with human and animal tissue without excessive toxicity, irritation, allergic reactions and/or other problems or complications, and proportionate to a reasonable benefit/risk ratio.

The phrase "pharmaceutically acceptable carrier" as used herein refers to a pharmaceutical substance, composition or vehicle such as a liquid or solid filler, diluent, excipient, manufacturing auxiliary (e.g., lubricant, talc, magnesium stearate, calcium stearate or zinc stearate or stearic acid) or a solvent encapsulated substance, which involves carrying or transporting the subject compound from one organ or part of the body to another. Each carrier must be "acceptable" in the sense of being compatible with the other ingredients of the formulation and harmless to the patient.

The term "pharmaceutical composition" refers to a composition comprising a compound of the present disclosure and at least one additional pharmaceutically acceptable carrier. "Pharmaceutically acceptable carrier" refers to a medium generally accepted in the art for the delivery of biologically active agents to animals, particularly mammals, including (i.e.) adjuvants, excipients or vehicles, such as diluents, preservatives, fillers, flow regulators, disintegrants, wetting agents, emulsifiers, suspending agents, sweeteners, flavoring agents, aromatics, antibacterial agents, antifungal agents, lubricants and dispersants, depending on the mode of administration and characteristics of dosage form.

The compound or pharmaceutical composition of the present disclosure, after administration, can ameliorate a disease, symptom or condition, especially the severity thereof, delay the onset of the disease, slow down the progression of the disease, or reduce the duration of the disease. Whether the administration is fixed or temporary, continuous or intermittent may be attributed to or related to the administration circumstances.

### Route of Administration

Suitable administration routes of the compound or pharmaceutical composition of the present disclosure include, but are not limited to, oral administration, intravenous injection, rectal administration, aerosol, and parenteral, ocular, pulmonary, transdermal, vaginal, ear canal, nasal and topical administration. In addition, by way of example only, parenteral administration includes intramuscular injection, subcutaneous injection, intravenous injection, intramedullary injection, ventricular injection, intraperitoneal injection, intralymphatic injection, and intranasal injection.

In some embodiments, the compound of the present disclosure is administered topically. In some other specific embodiments, the compound of the present disclosure is administered via implantation (e.g., subcutaneously or intramuscularly) or via intramuscular injection. Furthermore, in still other specific embodiments, the compound of the present disclosure is administered by a targeted drug delivery system.

In the pharmaceutical composition of the present disclosure, pharmaceutical carriers can be formulated according to many factors within the scope of knowledge of those skilled in the art. These factors include, but are not limited to, the type and characteristics of the active agent to be formulated; the subject to whom the composition containing the active agent is to be administered; the intended route of administration of the composition; and the targeted therapeutic indication. Pharmaceutical carriers include aqueous and non-aqueous liquid media and various solid and semi-solid dosage forms.

The carriers described above may include many other different ingredients and additives in addition to the active agent. The other ingredients are included in the formulation for various reasons known to those skilled in the art, such as an activity-stabilizing agent and a binding agent. Descriptions of suitable pharmaceutical carriers and factors involved in carrier selection can be found in several readily available sources, such as Allen L. V .Jr. et al. Remington: The Science and Practice of Pharmacy (2 Volumes), 22nd Edition (2012), Pharmaceutical Press.

The compound is typically administered in the form of a mixture formulated with a suitable pharmaceutical diluent, excipient or carrier (herein, collectively referred to as pharmaceutical carriers), which is appropriately selected in accordance with the intended mode of administration (such as oral tablets, capsules, elixirs, and syrups) and consistent with conventional pharmacy practice.

Although the compound of the present disclosure can be administered alone, it is preferred to be administered in the form of a pharmaceutical formulation (composition).

### Kit/Product Packaging

For use in the treatment of the above indications, the kit/product packaging is also described herein. These kits can be composed of a conveyor, a drug pack or a container box, wherein the container box can be divided into multiple compartments to accommodate one or more containers, such as a vial, a test tube and the like, and each container contains a single ingredient in the method described. Suitable containers include bottles, vials, syringes and test tubes. Containers are made of acceptable materials such as glass or plastic.

For example, a container may contain one or more compounds described herein, where the compound presents either as a pharmaceutical component or in a mixture with other ingredients described herein. The container may have a sterile outlet (for example, the container may be an intravenous infusion bag or bottle, and the stopper can be pierced by a hypodermic needle). Such kit may contain a compound and an instruction for the use method as described herein, a label, or an operation instruction.

A typical kit may include one or more containers. Each container contains one or more materials (such as a reagent, or a concentrated stock solution, and/or equipment) to accommodate commercial promotion and demand of users for the use of the compound. These materials include, but are not limited to, a buffer, a diluent, a filter, a needle, a syringe, a delivery device, a bag, a container, a bottle and/or a test tube, accompanied by a list of contents and/or an instruction for use, as may the built-in packaging. The entire set of instruction must be included.

The above features described in the present disclosure or the features described in the embodiments may be combined in any combination. All features disclosed in the specification of the present disclosure may be used in conjunction with any combination form. Each feature disclosed in the specification may be replaced by any alternative feature that serves the same, equivalent or similar purpose. Therefore, unless otherwise stated, the disclosed features are only general examples of equivalent or similar features.

The present disclosure will be further described below in conjunction with specific examples. It should be understood that these examples are only used to illustrate the present disclosure and are not intended to limit the scope of the present disclosure. In the following examples, experimental methods without specifying specific conditions are usually conducted under conventional conditions or conditions recommended by the manufacturer. Unless otherwise stated, all percentages, ratios, proportions, or parts are by weight.

The unit of weight-volume percentage in the present disclosure is well known to those skilled in the art, for example, referring to the weight of the solute in 100 ml of solution. Unless otherwise defined, all technical and scientific terms used herein have the same meaning as familiar to those skilled in the art. In addition, any methods and materials similar or equivalent to those described can be used in the method of the present disclosure. The preferred implementation methods and materials described herein are for demonstration purposes only.

In the terms used in the present disclosure, "neurodegenerative disease" and "neural degenerative disease" have the same meaning; "axonal degeneration" and "axon degeneration" have the same meaning. Those skilled in the art will understand that the terms have commonly understood meanings.

The present disclosure includes the description provided in the examples, which is not intended to limit the scope of any claim. The following non-limiting examples are provided to further illustrate the present disclosure. In light of the present disclosure, those skilled in the art will appreciate that many changes can be made to the specific embodiments disclosed and the same or similar results can still be obtained without departing from the spirit and scope of the present teachings.

Unless otherwise stated, all materials/reagents were obtained from commercial suppliers and used without further purification. Reactions were monitored during the experiments by LC-MS and/or thin layer chromatography (TLC) on silica 60 F254 (0.2 mm) precoated glass backings and visualized using UV light. The structures of the compounds in the following examples were characterized by nuclear magnetic resonance (NMR) and/or mass spectrometry (MS).

¹HNMR (400 MHz) spectra were recorded on Bruker spectrometer at room temperature with TMS or residual solvent peaks as internal standards. Chemical shift values or peak shape multiples are given in (δ), and the coupling constant (J) is given as an absolute value in Hertz (Hz). The abbreviations for multiplicity in ¹HNMR spectra are as follows: s (singlet), d (doublet), t (triplet), q (quartet), m (multiplet), br or broad (broadened).

Preparative HPLC purification was performed on Shimadzu LC-6AD. All purification work was performed using Shim-pack PREP-DDS(H)KIT chromatographic columns. The mobile phase was water (containing 0.1% HCO2H) and acetonitrile; all reagents used were HPLC grade. The flow rate was 10 ml/min.

LC-MS was performed on Agilent 1260 infinityII under the following conditions: mobile phase: A: water (0.1% trifluoroacetic acid), B: ACN; 3.5 min of running program; column: YMC-Triart C18, 50×3 mm, 3 m; flow rate: 1.8 ml/min; oven temperature: 40°C.; gradient: 5-100 (ACN %).

Preparative TLC was performed on a Whatman LK6F Silica Gel 60A plate with a size of 20×20 cm and a thickness of 500 µm.

The following examples are intended to illustrate embodiments of the present disclosure without limiting it in any way.

### Synthesis route of intermediate BB1

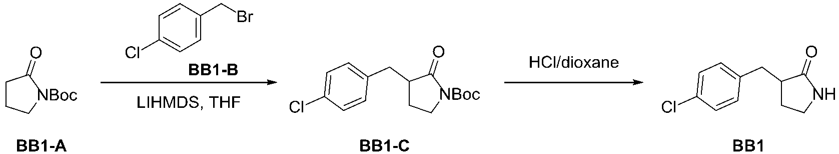

### Step 1: Compound BB1-C tert-butyl 3-(4-chlorobenzyl)-2-oxopyrrolidin-1-carboxylate

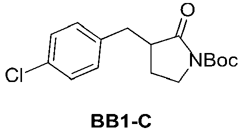

Under nitrogen protection at -60°C, LiHMDS (21.6 mL, 21.6 mmol) was slowly added to a solution of compound BB1-A tert-butyl 2-oxopyrrolidine-1-carboxylate (4 g, 21.6 mmol) in tetrahydrofuran (60 mL). After 1 h of reaction at -60°C, a solution of compound BB1-B 4-chlorobenzyl bromide (4.44 g, 21.6 mmol) in tetrahydrofuran (10 mL) was slowly added dropwise. After 2 h of reaction at -60°C, the reaction solution was heated to room temperature and stirred for 18 h. After the reaction was completed, as monitored by TLC, the reaction solution was added to the saturated ammonium chloride solution (50 mL) to quench reaction, and extracted with ethyl acetate. The organic phases were combined, dried over anhydrous sodium sulfate and concentrated under reduced pressure. The resulting crude product was separated and purified by preparative thin layer chromatography (eluent: a solution of 10-15% ethyl acetate in petroleum ether) to obtain compound BB1-C tert-butyl 3-(4-chlorobenzyl)-2-oxopyrrolidin-1-carboxylate as a colorless oil (1.7 g).

¹H NMR (400 MHz, CDCl₃) δ 7.30 - 7.27 (m, 2H), 7.15 (d, *J* = 8.4 Hz, 2H), 3.69 (ddd, *J* = 11.2, 8.7, 2.7 Hz, 1H), 3.59 - 3.51 (m, 1H), 3.23 (dd, *J* = 13.6, 3.9 Hz, 1H), 2.83 - 2.74 (m, 1H), 2.68 (t, *J* = 6.8 Hz, 1H), 2.06 - 1.99 (m, 1H), 1.71 - 1.65 (m, 1H), 1.55 (s, 9H).

### Step 2: Compound BB1 3-(4-chlorobenzyl)pyrrolidin-2-one

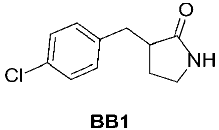

Compound BB1-C tert-butyl 3-(4-chlorobenzyl)-2-oxopyrrolidin-1-carboxylate (1.7 g, 5.488 mmol) was added to a solution of 4 M hydrochloric acid in dioxane (20 mL) at room temperature, and stirred for 1 h. After the reaction was completed, as monitored by TLC, the reaction system was concentrated under reduced pressure to obtain compound BB1 3-(4-chlorophenzyl) pyrrolidine-2-one (1.15 g) as a white solid.

LC_MS: (ES⁺): m/z 210.06 [M+H] ⁺

### Synthesis route of intermediate BB2

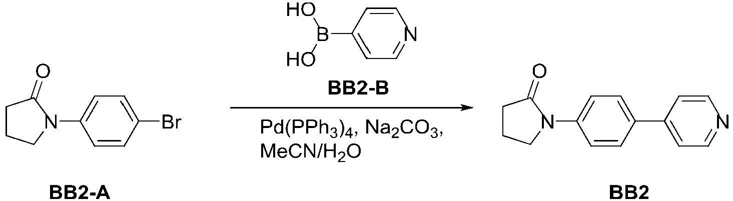

### Step 1: Compound BB2 1-(4-(pyridin-4-yl)phenyl) pyrrolidin-2-one

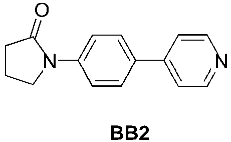

To a solution of compound BB2-A 1-(4-bromophenyl)pyrrolidin-2-one (2.5 g, 10.41 mmol), compound BB2-B pyridin-4-boronic acid (1.92 g, 15.62 mmol) and sodium carbonate (2.76 g, 26.03 mmol) in acetonitrile (50 mL) and water (10 mL), Pd(PPh₃)₄ (1.2 g, 1.04 mmol) was added. The reaction system was subjected to nitrogen replacement 3 times, and heated to 90°C for 18 h. After the reaction was completed, as monitored by TLC, the reaction solution was cooled to room temperature and extracted with ethyl acetate and water. The organic phases were combined, dried over anhydrous sodium sulfate and concentrated under reduced pressure. The resulting crude product was purified by silica gel column chromatography (eluent: a solution of 0-0.047% methanol in dichloromethane) to obtain compound BB2 1-(4-(pyridin-4-yl)phenyl)pyrrolidin-2-one as a yellow solid (1.9 g).

LC_MS: (ES⁺): m/z 239.05 [M+H] ⁺.

### Synthesis route of intermediate BB3

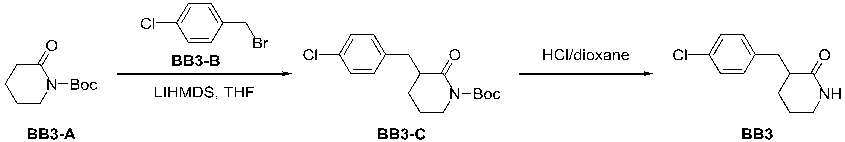

### Step 1: Compound BB3-C tert-butyl 3-(4-chlorobenzyl)-2-oxopiperidin-1-carboxylate

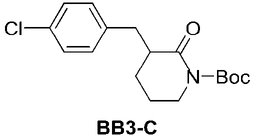

This step was carried out referring to step 1 of the synthesis of intermediate BB1.

LC_MS: (ES⁺): m/z 324.13 [M+H] ⁺.

### Step 2: Compound BB3 3-(4-chlorobenzyl)piperidin-2-one

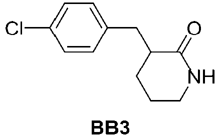

This step was carried out referring to step 2 of the synthesis of intermediate BB1.

LC_MS: (ES⁺): m/z 224.10 [M+H] ⁺.

### Example 1: Synthesis route of compound 1

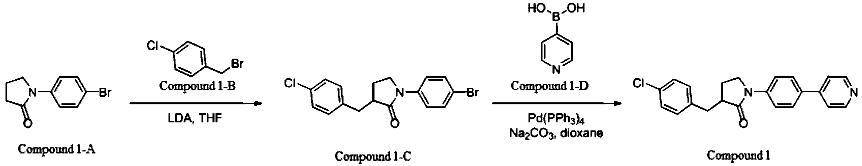

### Step 1: Compound 1-C 1-(4-bromophenyl)-3-(4-chlorobenzyl)pyrrolidin-2-one

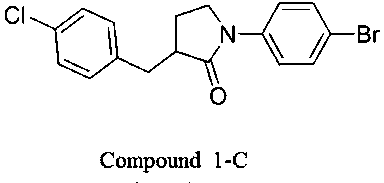

Compound 1-A (1-(4-bromophenyl)pyrrolidin-2-one (100 mg, 0.42 mmol) was dissolved in anhydrous tetrahydrofuran (4 mL). The system was cooled to -65°C under nitrogen protection. LDA (2 M in THF, 0.46 mL) was then added dropwise at -65°C. The reaction system was slowly warmed to -30°C within 0.5 h and then cooled to -65°C. A solution of compound 1-B 4-chlorobenzyl bromide (257 mg, 1.25 mmol) in THF (1 mL) was added. The reaction system was allowed to slowly return to room temperature for 1 h of reaction. After the reaction was completed, as monitored by TLC, the reaction solution was quenched with saturated ammonium chloride solution (10 mL) and extracted with ethyl acetate (10 mL×2). The organic phases were combined, backwashed once with saturated saline (20 mL), and evaporated under reduced pressure to dryness. The resulting crude product was purified by silica gel column chromatography (eluent: a solution of 6.7-16.7% ethyl acetate in petroleum ether) to obtain compound 1-C 1-(4-bromophenyl)-3-(4-chlorobenzyl)pyrrolidin-2-one as a white solid (108 mg).

LC_MS: (ES⁺): m/z 364.0 [M+H] ⁺

¹H NMR (400 MHz, CDCl₃): δ 7.53-7.46 (m, 4H), 7.28-7.26 (m, 2H), 7.18-7.16 (m, 2H), 3.76-3.56 (m, 2H), 3.25-3.21 (m, 1H), 2.91-2.77 (m, 2H), 2.22-2.15 (m, 1H), 1.88-1.80 (m, 1H).

### Step 2: Compound 1 3-(4-chlorobenzyl)-1-(4-( pyridin-4-yl) phenyl)pyrrolidin-2-one

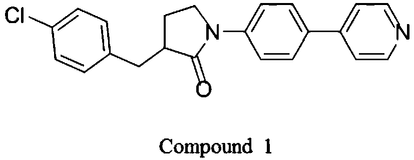

Compound 1-C 1-(4-bromophenyl)-3-(4-chlorobenzyl)pyrrolidin-2-one (50 mg, 0.137 mmol), compound 1-D pyridine-4-boronic acid (21 mg, 0.164 mmol), sodium carbonate (29 mg, 0.27 mmol), and tetrakis(triphenylphosphine)palladium (16 mg, 0.014 mmol) were added to a 10 mL reaction flask, and then dioxane (1 mL) and H₂O (0.5 mL) were added. The system was subjected to nitrogen replacement 3 times and the reaction was performed overnight at 90°C. The system was then cooled to room temperature. After the reaction was completed, as monitored by TLC, water was added to quench the reaction, and the reaction system was extracted with ethyl acetate (10 mL×2). The organic phases were combined, backwashed once with saturated saline (20 mL), and evaporated under reduced pressure to dryness. The resulting crude product was purified by Pre-TLC (eluent: a solution of 4% methanol in dichloromethane) to obtain compound 1 3-(4-chlorobenzyl)-1-(4-(pyridin-4-yl)phenyl)pyrrolidin-2-one as a white solid (30.4 mg).

LC_MS: (ES⁺): m/z 363.5 [M+H] ⁺

¹H NMR (400 MHz, CDCl₃): δ 8.66 (d, *J* = 5.2 Hz, 2H), 7.79-7.76 (m, 2H), 7.69-7.67 (m, 2H), 7.55 (d, *J* = 6.4 Hz, 2H), 7.29-7.27 (m, 2H), 7.20-7.18 (m, 2H), 3.80-3.65 (m, 2H), 3.29-3.24 (m, 1H), 2.98-2.91 (m, 1H), 2.86-2.81 (m, 1H), 2.27-2.19 (m, 1H), 1.92-1.82 (m, 1H).

### Example 2: Synthesis route of compound 2

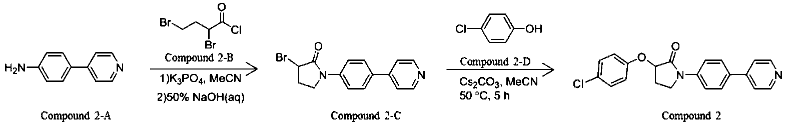

### Step 1: Compound 2-C 3-bromo-1-(4-(pyridin-4-yl)phenyl)pyrrolidin-2-one

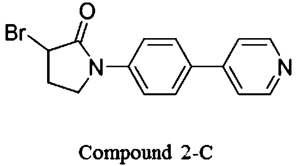

To a mixture of compound 2-A4-(pyridin-4-yl)aniline (50 mg, 0.294 mmol) and K₃PO₄ (32 mg, 0.147 mmol) in acetonitrile (2 mL) in an ice bath, compound 2-B 2,4-dibromobutyryl chloride (78 mg, 0.294 mmol) was added. The ice bath was then removed. The reaction solution was stirred at room temperature for 1 h and then aqueous 50% NaOH solution (0.6 mL) was added. The reaction solution was stirred overnight and filtered. The filter cake was washed with acetonitrile, and the filtrate was evaporated to dryness. The resulting crude product was purified by silica gel column chromatography (eluent: a solution of 20-30% ethyl acetate in petroleum ether) to obtain compound 2-C 3-bromo-1-(4-(pyridin-4-yl)phenyl)pyrrolidin-2-one as a yellow solid (20 mg).

LC_MS: (ES⁺): m/z 317.00 [M+H] ⁺.

### Step 2: Compound 2 3-(4-chlorophenoxy)-1-(4-(pyridin-4-yl)phenyl)pyrrolidin-2-one

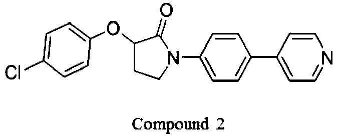

A solution of compound 2-C 3-bromo-1-(4-(pyridin-4-yl)phenyl)pyrrolidin-2-one (20 mg, 0.063 mmol), compound 2-D 4-chlorophenol (9 mg, 0.063 mmol) and Cs₂CO₃ (41 mg, 0.126 mmol) in acetonitrile (2 mL) was heated to 60°C and stirred for 5 h. After the reaction was completed, the reaction solution was evaporated to dryness. 20 mL of a mixed solvent CH₂Cl₂/MeOH (V/V=20/1) was added and dissolved completely. The reaction solution was filtered, and the filtrate was evaporated to dryness. The resulting crude product was purified by Prep-TLC (eluent: CH₂Cl₂/MeOH=20/1) to obtain compound 2 3-(4-chlorophenoxy)-1-(4-(pyridin-4-yl)phenyl)pyrrolidin-2-one as a yellow solid (11 mg).

LC_MS: (ES⁺): m/z 365.40 [M+H] ⁺.

### Example 3: Synthesis route of compound 3

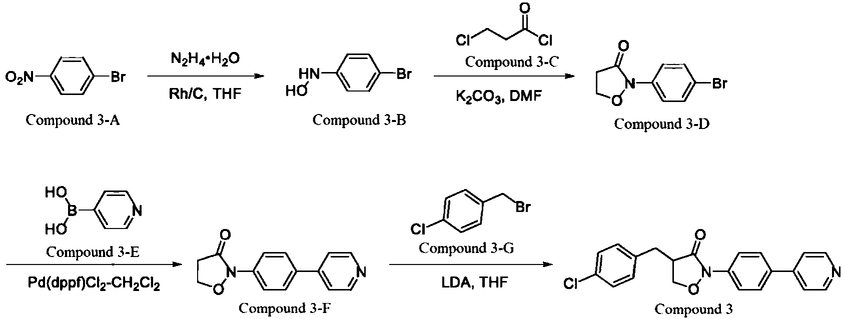

### Step 1: Compound 3-B N-(4-bromophenyl)hydroxylamine

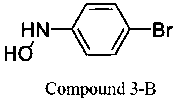

To a solution of compound 3-A 1-bromo-4-nitrobenzene (1.0 g, 4.95 mmol) in tetrahydrofuran (20 mL), rhodium on carbon (100 mg) was added. The reaction solution was cooled to 0°C. Hydrazine hydrate (309.77 mg, 4.95 mmol, 80% wt) was added dropwise in batches while maintaining the temperature at 25°C or less. The reaction solution was heated to room temperature and stirred for 2 h. After the reaction was completed, as monitored by TLC, the reaction solution was filtered through diatomaceous earth and concentrated under reduced pressure to obtain the crude compound 3-B N-(4-bromophenyl)hydroxylamine (930.78 mg). The obtained crude product was used directly in the next step without further purification.

LC_MS: (ES⁺): m/z 187.96 [M+H] ⁺.

### Step 2: Compound 3-D 2-(4-bromophenyl)isoxazolin-3-one

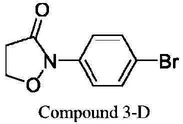

To a solution of compound 3-B N-(4-bromophenyl)hydroxylamine (930.78 mg, 4.95 mmol) in N,N-dimethylformamide (10 mL), potassium carbonate (684.46 mg, 4.95 mmol) and compound 3-C 3-chloropropionyl chloride (628.51 mg, 4.95 mmol) were added. The reaction solution was stirred at 25°C for 1 h. After the reaction was completed, as monitored by LCMS, the reaction solution was diluted with water (20 mL) and extracted with ethyl acetate (20 mL×2). The organic layers were combined, washed with saturated saline (20 mL), dried over anhydrous sodium sulfate, filtered and concentrated. The residue was purified by silica gel column chromatography (eluent: a solution of 25% ethyl acetate in petroleum ether) to obtain compound 3-D 2-(4-bromophenyl)isoxazolidin-3-one as a yellow solid (680 mg).

LC_MS: (ES⁺): m/z 241.97 [M+H] ⁺.

¹H NMR (400 MHz, CDCl₃) δ 7.64 - 7.58 (m, 2H), 7.53 - 7.47 (m, 2H), 4.56 (t, *J* = 8.0 Hz, 2H), 3.04 (t, *J* = 8.0 Hz, 2H).

### Step 3: Compound 3-F 2-(4-(pyridin-4-yl)phenyl)isoxazolidin-3-one

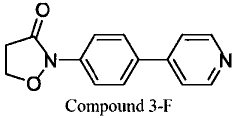

To a solution of compound 3-D 2-(4-bromophenyl)isoxazolidin-3-one (200 mg, 0.826 mmol), compound 3-E pyridin-4-boronic acid (152.44 mg, 1.24 mmol) and sodium carbonate (262.67 mg, 2.48 mmol) in 1,4-dioxane (5 ml) and water (1 ml), Pd(dppf)Cl₂-CH₂Cl₂ (67.45mg, 0.0826 mmol) was added. The reaction system was subjected to nitrogen replacement 3 times and heated to 85°C for 18 h. After the reaction was completed, as monitored by TLC, the reaction solution was extracted with water (20 mL) and ethyl acetate (20 mL). The organic phases were combined, dried over anhydrous sodium sulfate, and concentrated under reduced pressure. The crude product was purified by silica gel column chromatography (eluent: a solution of 0%-0.047% methanol in dichloromethane) to obtain compound 3-F 2-(4-(pyridin-4-yl)phenyl)isoxazolidin-3-one as a yellow solid (150 mg).

LC_MS: (ES⁺): m/z 241.10 [M+H] ⁺.

### Step 4: Compound 3 4-(4-chlorobenzyl)-2-(4-(pyridin-4-yl)phenyl)isoxazolidin-3-one

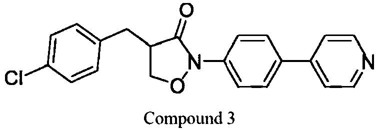

Under nitrogen protection at -78°C, LDA (208 µl, 0.416 mmol) was slowly added to a solution of compound 3-F 2-(4-(pyridin-4-yl)phenyl)isoxazolidin-3-one (50 mg, 0.208 mmol) in tetrahydrofuran (2.5 ml). After 1 h of reaction, compound 3-G 4-chlorobenzyl bromide (42.74 mg, 0.208 mmol) was added and the reaction solution was stirred at -78°C for 2 h of reaction. After the reaction was completed, as monitored by TLC, the reaction solution was added to saturated ammonium chloride solution (10 ml) to quench reaction, and extracted with ethyl acetate. The organic phases were combined, dried over anhydrous sodium sulfate, and concentrated under reduced pressure. The resulting crude product was purified by preparative thin layer chromatography (eluent: a solution of 0.047% methanol in dichloromethane) to obtain compound 3 4-(4-chlorobenzyl)-2-(4-(pyridin-4-yl)phenyl)isoxazolidin-3-one as a yellow solid (8 mg).

LC_MS: (ES⁺): m/z 365.50 [M+H] ⁺.

### Example 4: Synthesis route of compound 4

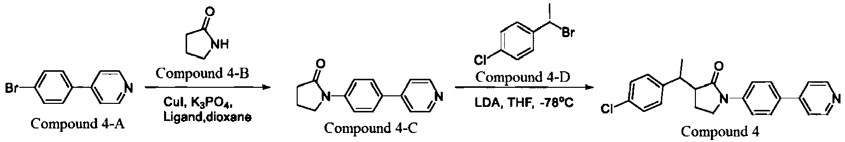

### Step 1: Compound 4-C 1-(4-(pyridin-4-yl)phenyl)pyrrolidin-2-one

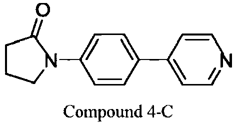

Under nitrogen atmosphere, compound 4-A 4-(4-bromophenyl)pyridine (120 mg, 0.5 mmol), compound 4-B pyrrolidin-2-one (60 mg, 0.75 mmol), copper(I) iodide (10 mg, 0.05 mmol), potassium phosphate (212 mg, 1 mmol), and (1R,2R)-N,N'-dimethyl-1,2-diaminocyclohexane (14 mg, 0.1 mmol) were dispersed in 1,4-dioxane (1 mL). The reaction mixture was stirred at 100°C for 16 h. After complete reaction of the raw material and the target mass were detected by LCMS, and formation of the major spot was detected by TLC, the reaction solution was evaporated under reduced pressure, and dichloromethane (20 mL) was added. The reaction solution was washed with water (15 mL), saturated ammonium chloride solution (20 mL) and saturated saline (20 mL) in sequence, dried over anhydrous sodium sulfate, filtered and concentrated. The resulting crude product was purified by preparative silica gel plate (DCM:MeOH = 20:1), and dried in vacuum to obtain compound 4-C 1-(4-(pyridin-4-yl)phenyl)pyrrolidin-2-one as a white solid (80 mg).

LC-MS: (ES+): m/z 239.11 [M+H] +.

### Step 2: Compound 4 3-(1-(4-chlorophenyl)ethyl)-1-(4-(pyridin-4-yl)phenyl)pyrrolidin-2-one

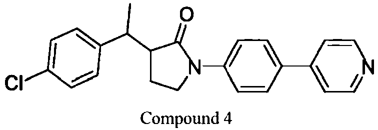

At -78°C, compound 4-C 1-(4-(pyridin-4-yl)phenyl)pyrrolidin-2-one (26 mg, 0.1 mmol) was dissolved in 2 ml of tetrahydrofuran, and lithium diisopropylamide (0.1 ml, 2N) was added dropwise. After 0.5 h, a solution of compound 4-D 1-(1-bromoethyl)-4-chlorobenzene (26 mg, 0.12 mmol) in tetrahydrofuran was added dropwise. The reaction solution was stirred at low temperature for 1 h. After complete reaction of the raw material and the target mass were detected by LCMS, and formation of the major spot was detected by TLC, the saturated ammonium chloride solution (10 mL) was added to quench the reaction. The reaction solution was extracted with dichloromethane (10 mL×3), washed with water (15 mL), saturated ammonium chloride solution (20 mL) and saturated saline (20 mL) in sequence, and then evaporated under reduced pressure to remove the reaction solvent. The resulting crude product was purified by using preparative silica gel plate (DCM:MeOH = 20:1), and dried in vacuum to obtain compound 4 3-(1-(4-chlorophenyl)ethyl)-1-(4-(pyridin-4-yl)phenyl)pyrrolidin-2-one as a yellow solid (10 mg).

LC-MS: (ES+): m/z 377.13 [M+H] +.

### Example 5: Synthesis route of compound 5

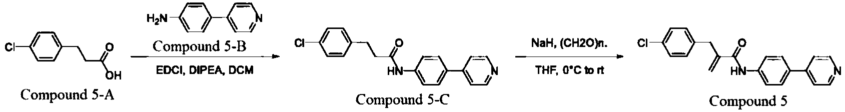

### Step 1: Compound 5-C 3-(4-chlorophenyl)-N-(4-(pyridin-4-yl)phenyl)propanamide

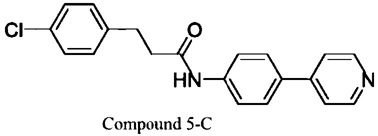

A mixture of compound 5-A 3-(4-chlorophenyl)propionic acid (184 mg, 1 mmol), 1-(3-dimethylaminopropyl)-3-ethylcarbodiimide (210 mg, 1.1 mmol) and N,N-diisopropylethylamine (387 mg, 3 mmol) was dissolved in 5 ml of dichloromethane in an ice bath and stirred for 15 min. Compound 5-B 4-(4-pyridinyl)aniline (170 mg, 1 mmol) was then added. The reaction mixture was stirred at room temperature for 2 h. After complete reaction of the raw material and the target mass were detected by LCMS, and formation of the major spot was detected by TLC, dichloromethane (20 mL) was added. The reaction solution was washed with water (15 mL), saturated ammonium chloride solution (20 mL) and saturated saline (20 mL) in sequence, dried over anhydrous sodium sulfate, filtered, and concentrated. The resulting crude product was purified through a column and dried in vacuum to obtain compound 5-C 3-(4-chlorophenyl)-N-(4-(pyridin-4-yl)phenyl)propanamide as a white solid (250 mg).

LC-MS: (ES+): m/z 337.1 [M+H] +.

### Step 2: Compound 5 2-(4-chlorobenzyl)-3-hydroxy-N-(4-pyridin-4-yl)phenyl)propanamide

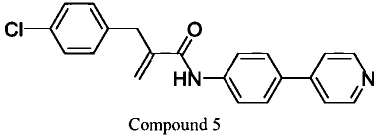

Compound 5-C 3-(4-chlorobenzyl)-N-(4-(pyridin-4-yl)phenyl)propanamide (34 mg, 0.1 mmol) was dissolved in 1 ml of tetrahydrofuran in an ice bath, and sodium hydride (60% wt, 9 mg, 0.22 mmol) was added carefully and stirred for 0.5 h. An excess amount of paraformaldehyde was then added. The reaction mixture was heated to room temperature and stirred for 2 h. After a product Ms was formed in reaction system, as detected by LCMS, the saturated ammonium chloride solution (10 mL) was added to quench the reaction. The reaction solution was extracted with dichloromethane (10 mL×3), washed with water (15 mL), saturated ammonium chloride solution (20 mL) and saturated saline (20 mL) in sequence, and evaporated under reduced pressure to remove the reaction solvent. The resulting crude product was purified by preparative HPLC to obtain compound 5 2-(4-chlorobenzyl)-3-hydroxy-N-(4-pyridin-4-phenyl)propanamide as a yellow solid (1 mg).

LC-MS: (ES+): m/z 349.05 [M+H] +.

### Example 6: Synthesis route of compound 6

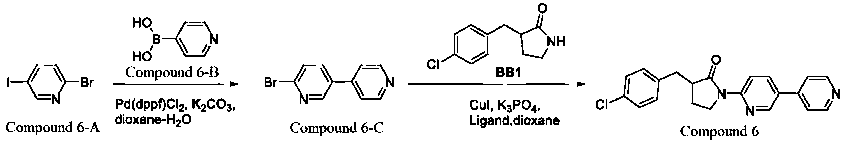

### Step 1: Compound 6-C 2-bromo-5-(pyridin-4-yl)pyridine

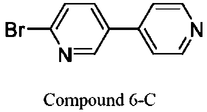

Under nitrogen atmosphere, to a suspension of compound 6-A 2-bromo-5-iodopyridine (1.0 g, 3.53 mmol), compound 6-B pyridin-4-boronic acid (434 mg, 3.53 mmol) and potassium carbonate (976 mg, 7.06 mmol) in 1,4-dioxane (15 ml)-water (3 mL), [1,1-bis(diphenylphosphine)ferrocene]palladium dichloride (25.9 mg, 0.035 mmol) was added at room temperature. The reaction mixture was subjected to nitrogen replacement 3 times and then stirred at 85°C for 12 h. After the reaction was completed, as determined by TLC, the reaction mixture was cooled to room temperature, partitioned into water (20 mL) and extracted with ethyl acetate (10 mL×3). The organic layers were combined, washed with saturated saline (20 mL), dried over anhydrous sodium sulfate, and concentrated under reduced pressure. The resulting crude product was purified by preparative TLC (eluent: a solution of 5% methanol in dichloromethane) to obtain compound 6-C 2-bromo-5-(pyridin-4-yl)pyridine as a white solid (490 mg).

LC_MS: (ES⁺): m/z 234.95, 236.95 [M+H] ⁺.

¹H NMR (400 MHz, DMSO) δ 8.88 - 8.85 (m, 1H), 8.70 (dd, *J=* 4.5, 1.7 Hz, 2H), 8.20 (dd, *J* = 8.3, 2.7 Hz, 1H), 7.85 - 7.82 (m, 1H), 7.82 - 7.79 (m, 2H).

### Step 2: Compound 6 1-([3,4'-bipyridin]-6-yl)-3-(4-chlorobenzyl)pyrrolin-2-one

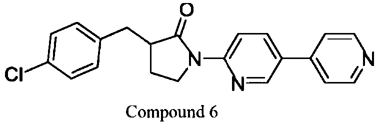

Under nitrogen atmosphere, to a suspension of compound 6-C 2-bromo-5-(pyridin-4-yl)pyridine (170 mg, 0.723 mmol), compound 6-D (BB1) 3-(4-chlorobenzyl)pyrrolin-2-one (150 mg, 0.723 mmol), (1R,2R)-N,N'-dimethyl-1,2-diaminocyclohexane (21 mg, 0.145 mmol) and potassium phosphate (307 mg, 1.446 mmol) in 1,4-dioxane (5 ml), copper(I) iodide (14 mg, 0.073 mmol) was added at room temperature. The reaction mixture was subjected to nitrogen replacement 3 times and then stirred at 100°C for 12 h. After the reaction was completed, as determined by TLC, the reaction mixture was cooled to room temperature, partitioned into water (20 mL) and extracted with ethyl acetate (10 mL×3). The organic layers were combined, washed with saturated saline (20 mL), dried over anhydrous sodium sulfate, and concentrated under reduced pressure. The resulting crude product was purified by preparative TLC (eluent: a solution of 9% methanol in dichloromethane) to obtain compound 6 1-([3,4'-bipyridin]-6-yl)-3-(4-chlorobenzyl)pyrrolin-2-one as a white solid (209 mg)

LC_MS: (ES⁺): m/z 364.10 [M+H] ⁺.

¹H NMR (400 MHz, CDCl₃) δ 8.87 (d, *J* = 2.5 Hz, 1H), 8.75 (m, 2H), 8.45 (dd, *J* = 8.7, 2.7 Hz, 1H), 8.01 (d, *J* = 4.4 Hz, 2H), 7.89 (d, *J* = 8.8 Hz, 1H), 7.30 (t, *J* = 5.4 Hz, 2H), 7.21 (d, *J* = 8.4 Hz, 2H), 3.83 (dd, *J* = 16.8, 8.6 Hz, 1H), 3.75 (td, *J* = 9.1, 3.1 Hz, 1H), 3.29 (dd, *J* = 13.7, 4.2 Hz, 1H), 2.99 (qd, *J* = 8.9, 4.2 Hz, 1H), 2.87 (dd, *J* = 13.7, 8.9 Hz, 1H), 2.35 - 2.26 (m, 1H), 1.95 (ddd, *J* = 18.0, 12.8, 8.8 Hz, 1H).

### Example 7: Synthesis route of compound 7

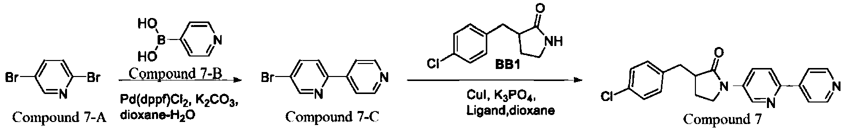

### Step 1: Compound 7-C 5-bromo-2,4'-bipyridine

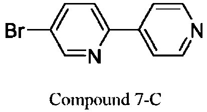

This step was carried out using compound 7-A 2,5-dibromopyridine as the raw material, referring to the synthesis procedure described in step 1 in Example 6.

LC_MS: (ES⁺): m/z 234.95, 236.95 [M+H] ⁺.

¹H NMR (400 MHz, CDCl₃) δ 8.82 (d, J= 2.3 Hz, 1H), 8.76 (d, *J* = 6.1 Hz, 2H), 7.98 (dd, *J* = 8.4, 2.4 Hz, 1H), 7.91 (dd, *J* = 4.7, 1.5 Hz, 2H), 7.74 (d, *J* = 8.4 Hz, 1H).

### Step 2: Compound 7 1-([2,4'-bipyridin]-5-)-3-(4-chlorobenzyl)pyrrolin-2-one

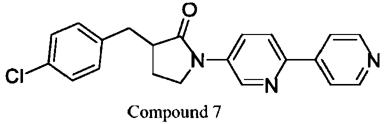

This step was carried out referring to the synthesis procedure described in step 2 in Example 6.

LC_MS: (ES⁺): m/z 364.10 [M+H] ⁺.

¹H NMR (400 MHz, CDCl₃) δ 8.74 (m, 2H), 8.67 (d, J= 2.1 Hz, 1H), 8.61 (d, *J* = 8.8 Hz, 1H), 8.00 (dd, *J* = 8.8, 2.5 Hz, 1H), 7.56 (m, 2H), 7.32 - 7.29 (m, 2H), 7.21 (d, *J* = 8.4 Hz, 2H), 4.13 (ddd, *J* = 11.4, 8.8, 2.8 Hz, 1H), 3.92 (ddd, *J* = 11.2, 8.9, 7.4 Hz, 1H), 3.31 (dd,*J* = 13.8, 4.3 Hz, 1H), 3.02 (ddd, *J* = 18.3, 9.3, 4.4 Hz, 1H), 2.83 (dd, *J* = 13.8, 9.2 Hz, 1H), 2.23 (tdd, *J* = 10.3, 7.5, 2.8 Hz, 1H), 1.92 - 1.83 (m, 1H).

### Example 8: Synthesis route of compound 8

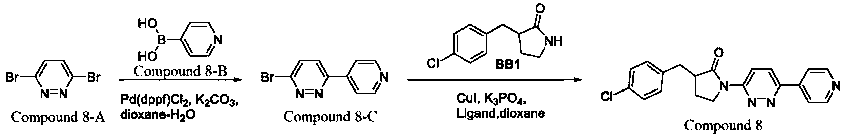

### Step 1: Compound 8-C 3-bromo-6-(pyridin-4-yl)pyridazine

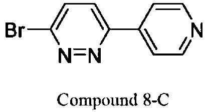

This step was carried out using compound 8-A 3,6-dibromopyridazine as the raw material, referring to the synthesis procedure described in step 1 in Example 6.

LC_MS: (ES⁺): m/z 235.90 [M+H] ⁺.

### Step 2: Compound 8 3-(4-chlorobenzyl)-1-(6-(pyridin-4-yl)pyridazin-3-yl)pyrrolidin-2-one

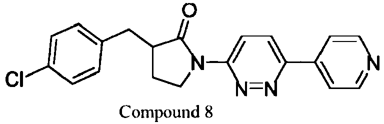

This step was carried out referring to the synthesis procedure described in step 2 in Example 6.

LC_MS: (ES⁺): m/z 241.10 [M+H] ⁺.

¹H NMR (400 MHz, CDCl₃) δ 8.87 (d, 9.4 Hz, 2H), 7.96 (d, J = 9.4 Hz, 2H), 7.32 - 7.29 (m, 2H), 7.23 - 7.18 (m, 4H), 4.32 (ddd, J = 11.6, 8.7, 2.9 Hz, 1H), 4.08 (ddd, J = 11.5, 9.0, 7.4 Hz, 1H), 3.30 (dd, J = 13.8, 4.5 Hz, 1H), 3.04 (qd, J = 9.1, 4.5 Hz, 1H), 2.84 (dd, J = 13.9, 9.1 Hz, 1H), 2.36 - 2.24 (m, 1H), 1.93 (dq, J = 12.8, 9.1 Hz, 1H).

### Example 9: Synthesis route of compound 9

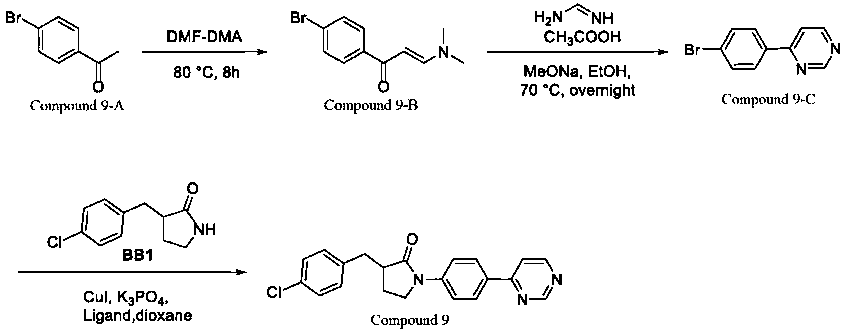

### Step 1: Compound 9-B (E)-1-(4-bromophenyl)-3-(dimethylamino)-2-propen-1-one

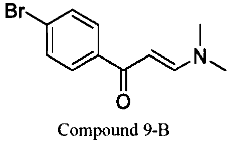

Compound 9-A (1 g, 5.02 mmol) was weighed into a 25 mL reaction tube, and 2 mL of DMF-DMA solvent was added. The reaction solution was heated to 80°C for 8 h. After the reaction was completed, the reaction solution was cooled to room temperature, and then poured into ice water. After precipitation of a solid, the reaction mixture was filtered to collect the filter cake. The filter cake was recrystallized from petroleum ether, filtered, and dried to obtain compound 9-B (E)-1-(4-bromophenyl)-3-(dimethylamino)-2-propen-1-one as a yellow solid (1.0 g), which was used directly in the next step.

### Step 2: Compound 9-C 4-(4-bromophenyl)pyrimidine

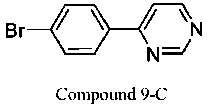

Compound 9-B (E)-1-(4-bromophenyl)-3-(dimethylamino)-2-propen-1-one (100 mg, 393.50 µ mol) was placed in a 10 mL reaction tube, and formamidine acetate (49.2 mg, 472.20 µmol), sodium methoxide (150 µL, 5.4 M in methanol), and ethanol (1.5 mL) were added in sequence at room temperature. The reaction system was heated to 70°C for overnight reaction. After the reaction was completed, the reaction system was cooled to room temperature, and an appropriate amount of water was added. A large amount of organic solvent was removed under vacuum, and then the system was extracted with ethyl acetate (10 mL×3), dried over anhydrous sodium sulfate, evaporated under reduced pressure, and separated and purified by preparative silica gel thin layer chromatography (eluent: a solution of 5% methanol in dichloromethane) to obtain yellow compound 9-C 4-(4-bromophenyl)pyrimidine (50 mg).

LC_MS: (ES⁺): m/z 236.85 [M+H] ⁺.

### Step 3: Compound 9 3-(4-chlorobenzyl)-1-(4-(pyrimidin-4-yl)phenyl)pyrrolidin-2-one

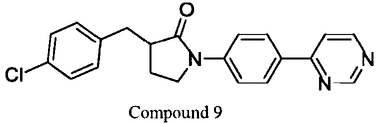

This step was carried out referring to the synthesis procedure described in step 2 in Example 6.

LC_MS: (ES⁺): m/z 364.10 [M+H] ⁺.

¹H NMR (400 MHz, DMSO-*d*₆) δ 9.22 (d, *J* = 1.3 Hz, 1H), 8.84 (*d*, *J* = 5.4 Hz, 1H), 8.28-8.24 (*m,* 2H), 8.09 (dd, *J =* 5.4, 1.4 Hz, 1H), 7.91-7.86 (*m,* 2H), 7.39-7.30 *(m,* 4H), 3.81-3.75 (*m,* 2H), 3.16-3.11 (*m,* 1H), 3.02-2.97 (*m,* 1H), 2.76 (*dd*, *J* = 13.6, 9.3 Hz, 1H), 2.12-2.06 (*m,* 1H), 1.82-1.76 (*m,* 1H).

### Example 10: Synthesis route of compound 10

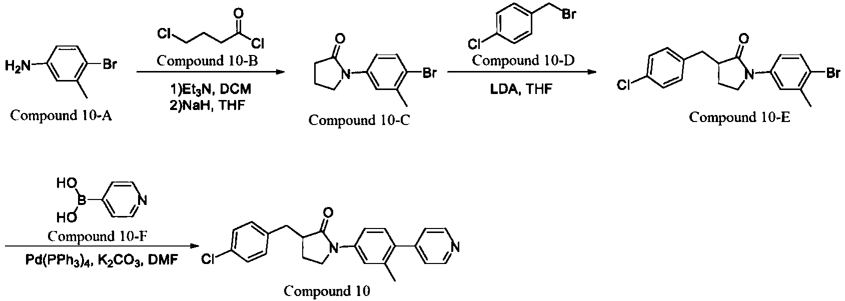

### Step 1: Compound 10-C 1-(4-bromo-3-methylphenyl)pyrrolidin-2-one

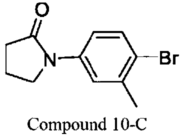

To a solution of compound 10-A 4-bromo-3-methylaniline (500 mg, 2.72 mmol) and triethylamine (554 mg, 5.54 mmol) in dichloromethane (10 mL) in an ice bath, compound 10-B 4-chlorobutyryl chloride (383 mg, 2.72 mmol) was added dropwise and stirred for 1 h. After the reaction was completed, the reaction solution was washed with water (20 mL×2). The organic layer was concentrated and dissolved in tetrahydrofuran (10 mL). Sodium hydride (163 mg, 4.08 mmol) was added in ice bath, and stirred for 2 h. After the reaction was completed, the reaction solution was poured into saturated ammonium chloride solution (20 mL), and extracted with ethyl acetate (15 mL×2). The organic layers were combined, washed with saturated saline (20 mL), dried over anhydrous sodium sulfate, and concentrated under reduced pressure. The residue was purified by silica gel column chromatography (eluent: a solution of 25% ethyl acetate in petroleum) to obtain compound 10-C 1-(4-bromo-3-methylphenyl)pyrrolidin-2-one as a white solid (330 mg).

LC_MS: (ES⁺): m/z 254.1 [M+H] ⁺.

### Step 2: Compound 10-E 1-(4-bromo-3-methylphenyl)-3-(4-chlorobenzyl)pyrrolidin-2-one

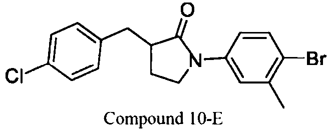

To a solution of compound 10-C 1-(4-bromo-3-methylphenyl)pyrrolidin-2-one (100 mg, 0.418 mmol) in tetrahydrofuran (2 mL), lithium diisopropylamide (0.418 ml, 0.836 mmol) was added at -78°C. The reaction mixture was stirred at -78°C for 1 h. A solution of compound 10-D 4-chlorobenzyl bromide (86 mg, 0.418 mmol) in tetrahydrofuran was added dropwise and the reaction mixture was stirred at -78°C for 2 h . After the reaction was completed, the reaction solution was poured into saturated ammonium chloride solution (20 mL), and left to stand for phase separation. The organic phase was collected and concentrated under reduced pressure, and the residue was purified by silica gel column chromatography (eluent: a solution of 25% ethyl acetate in petroleum ether) to obtain compound 10-E 1-(4-bromo-3-methylphenyl)-3-(4-chlorobenzyl)pyrrolidin-2-one as a pale yellow solid (80 mg, 50%).

LC_MS: (ES⁺): m/z 378.1 [M+H] ⁺.

### Step 3: Compound 10 3-(4-chlorobenzyl)-1-(3-methyl-4-(pyridin-4-yl)phenyl)pyrrolidin-2-one

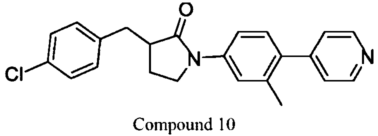

A solution of compound 10-E 1-(4-bromo-3-methylphenyl)-3-(4-chlorobenzyl)pyrrolidin-2-one (80 mg, 0.212 mmol), compound 10-F pyridine-4-boronic acid (52 mg, 0.424 mmol), tetrakis(triphenylphosphine)palladium (2.5 mg, 2 µmol) and potassium carbonate (50 mg, 0.424 mmol) in N,N-dimethylformamide (2 mL) was heated to 85°C and stirred under nitrogen protection for 16 h. After the reaction was completed, the reaction was diluted with water (20 mL), and extracted with ethyl acetate (10 mL×2). The organic layers were combined, washed with saturated saline (20 mL), dried over anhydrous sodium sulfate, and concentrated under reduced pressure. The residue was purified by silica gel column chromatography (eluent: a solution of 25% ethyl acetate in petroleum ether) to obtain compound 10 3-(4-chlorobenzyl)-1-(3-methyl-4-(pyridin-4-yl)phenyl)-4-yl)phenyl)pyrrolidin-2-one as a white solid (20 mg).

LC_MS: (ES⁺): m/z 377.1 [M+H] ⁺.

### Example 11: Synthesis route of compound 11

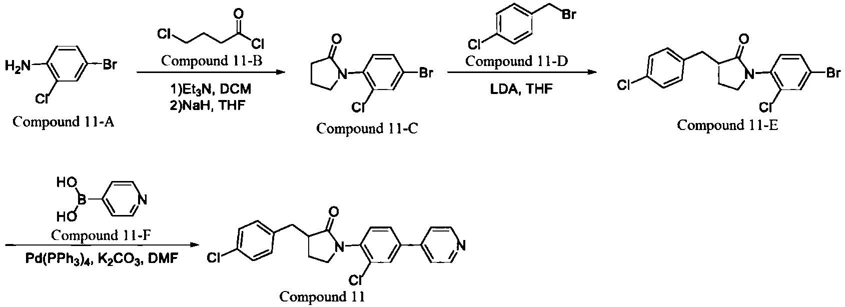

### Step 1: Compound 11-C 1-(4-bromo-2-chlorophenyl)pyrrolidin-2-one

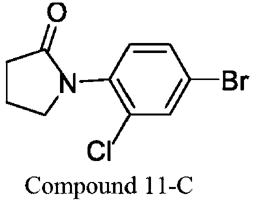

This step was carried out using compound 10-A 4-bromo-2-chloroaniline as the raw material, referring to the synthesis procedure described in step 1 in Example 10.

LC_MS: (ES⁺): m/z 274.02 [M+H] ⁺.

### Step 2: Compound 11-E 1-(4-bromo-2-chlorophenyl)-3-(4-chlorobenzyl)pyrrolidin-2-one

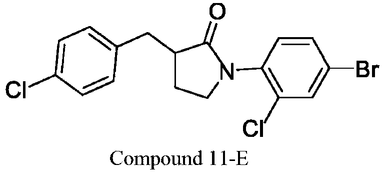

This step was carried out referring to the synthesis procedure described in step 2 in Example 10.

LC_MS: (ES⁺): m/z 398.10 [M+H] ⁺.

### Step 3: Compound 11 3-(4-chlorobenzyl)-1-(2-chloro-4-(pyridin-4-yl)phenyl)pyrrolidin-2-one

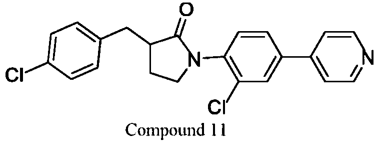

This step was carried out referring to the synthesis procedure described in step 3 in Example 10.

LC_MS: (ES⁺): m/z 397.10 [M+H] ⁺.

¹H NMR (400 MHz, DMSO-*d*₆) δ 8.71-8.66 *(m,* 2H), 8.02 (d, *J* = 2.1 Hz, 1H), 7.85 (dd, *J= 8.2, 2.1* Hz, 1H), 7.80-7.76 *(m,* 2H), 7.53 (*d*, *J* = 8.2 Hz, 1H), 7.44-7.31 *(m,* 4H), 3.73-3.65 *(m,* 1H), 3.56 (*td*, *J* = 8.8, 3.0 Hz, 1H), 3.10 (*dd*, *J* = 13.7, 4.4 Hz, 1H), 2.92 (*qd*, *J* = 9.0, 4.4 Hz, 1H), *2.76 (dd, J* = 13.6, 9.2 Hz, 1H), 2.23-2.10 (*m,* 1H), 1.86 (*dq*, *J* = 12.4, 8.6 Hz, 1H).

### Example 12: Synthesis route of compound 12

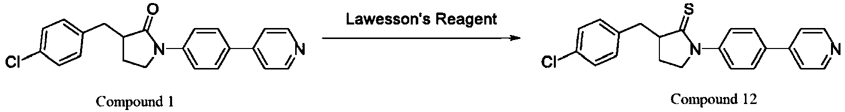

### Step 1: compound 12 3-(4-chlorophenyl)-1-(4-(pyridin-4-yl)phenyl)pyrrolidin-2-thione

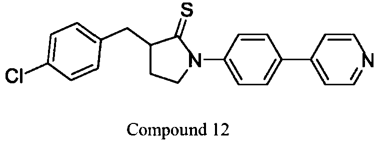

To a solution of compound 1 3-(4-chlorophenyl)-1-(4-(pyridin-4-yl)phenyl)pyrrolidin-2-one (50 mg, 137.80 µmol) in toluene (2 mL), Lawesson's reagent (167.2 mg, 413.39 µmol) was added. The reaction solution was stirred at 100°C for 12 h. After the reaction was completed, as monitored by TLC, the reaction solution was concentrated, diluted with ethyl acetate, filtered and concentrated to obtain a residue, which was purified by preparative thin layer chromatography (eluent: a solution of 5% methanol in dichloromethane) to obtain compound 12 3-(4-chlorophenyl)-1-(4-(pyridin-4-yl)phenyl) pyrrolidin-2-thione (16.1 mg).

LC_MS: (ES⁺): m/z 379.10 [M+H] ⁺.

### Example 13: Synthesis route of compound 13

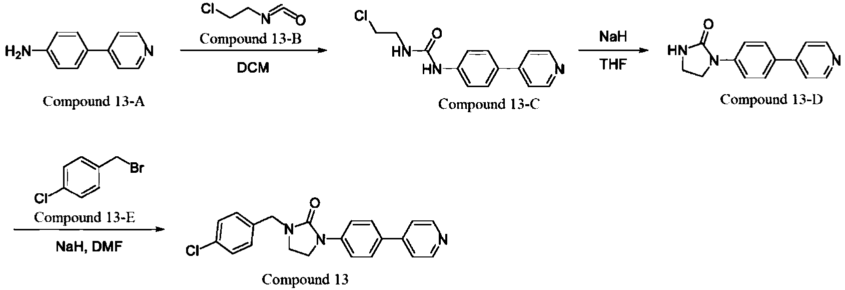

### Step 1: Compound 13-C 1-(2-chloroethyl)-3-(4-(pyridin-4-yl)phenyl)urea

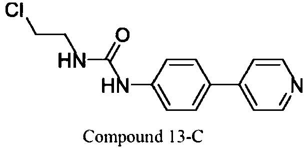

To a solution of compound 13-A 4-(pyridin-4-yl)aniline (500 mg, 2.94 mmol) in dichloromethane (20 mL) in an ice bath, compound 13-B chloroethyl isocyanate (308 mg, 2.94 mmol) was added dropwise. The reaction mixture was stirred in an ice bath for 1 h. After the reaction was completed, the reaction solution was diluted with water (20 mL) and extracted with dichloromethane (20 mL×2). The organic layers were combined and concentrated under reduced pressure. The residue was purified by column chromatography (eluent: a solution of 50% ethyl acetate in petroleum ether) to obtain compound 13-C 1-(2-chloroethyl)-3-(4-(pyridin-4-yl)phenyl)urea as a white solid (360 mg).

LC_MS: (ES⁺): m/z 276.1 [M+H] ⁺.

### Step 2: Compound 13-D 1-(4-(pyridin-4-yl)phenyl)imidazolin-2-one

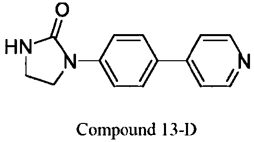

To a solution of compound 13-C 1-(2-chloroethyl)-3-(4-(pyridin-4-yl)phenyl)urea (360 mg, 1.31 mmol) in tetrahydrofuran (5 mL) in an ice bath, sodium hydride (104 mg, 2.62 mmol) was added. The reaction mixture was stirred in an ice bath for 3 h of reaction. After the reaction was completed, the reaction mixture was filtered to collect a filter cake. The filter cake was dried to obtain compound 13-D 1-(4-(pyridin-4-yl)phenyl)imidazolin-2-one as a pale yellow solid (300 mg).

LC_MS: (ES⁺): m/z 240.1 [M+H] ⁺.

### Step 3: Compound 13 1-(4-chlorobenzyl)-3-(4-(pyridin-4-yl)phenyl)imidazolin-2-one

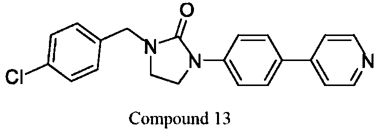

To a solution of compound 13-D 1-(4-(pyridin-4-yl)phenyl)imidazolin-2-one (50 mg, 209 µmol) in N,N-dimethylformamide (1 mL) in an ice bath, sodium hydride (17.5 mg, 314 µmol) was added and stirred for 30 min. Compound 13-E 4-chlorobenzyl bromide (47 mg, 230 µmol) was then added. The reaction mixture was stirred in an ice bath for 3 h. After the reaction was completed, the reaction solution was diluted with water (10 mL) and filtered to collect a filter cake. The filter cake was triturated in tetrahydrofuran (1 mL) for 2 h and filtered. The collected filter cake was dried to obtain compound 13 1-(4-chlorobenzyl)-3-(4-(pyridin-4-yl)phenyl)imidazolin-2-one as a yellow solid (30 mg).

LC_MS: (ES⁺): m/z 364.1 [M+H] ⁺.

¹H NMR (400 MHz, Chloroform-d) δ 8.66 - 8.60 (m, 2H), 7.75 - 7.69 (m, 2H), 7.69 - 7.62 (m, 2H), 7.55 - 7.49 (m, 2H), 7.37 - 7.30 (m, 2H), 7.28 (d, J = 2.3 Hz, 2H), 4.47 (s, 2H), 3.91 - 3.82 (m, 2H), 3.44 - 3.36 (m, 2H).

### Example 14: Synthesis route of compound 14

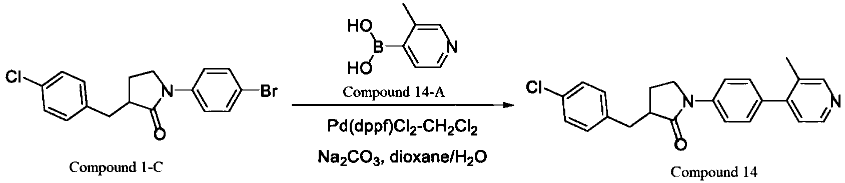

### Step 1: Compound 14 3-(4-chlorobenzyl)-1-(4-(3-methylpyridin-4-yl)phenyl)pyrrolidin-2-one

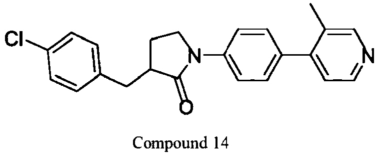

This step was carried out using compound 14-A 3-methylpyridin-4-boronic acid as the raw material, referring to the synthesis procedure described in step 2 in Example 1.

LC_MS: (ES+): m/z 377.15 [M+H] +.

¹H NMR (400 MHz, CDCl3) δ 8.52 (s, 1H), 8.48 (d, J = 5.0 Hz, 1H), 7.76 - 7.71 (m, 2H), 7.40 - 7.35 (m, 2H), 7.33 - 7.29 (m, 2H), 7.22 (d, J = 8.4 Hz, 2H), 7.16 (d, J = 5.0 Hz, 1H), 3.80 (dd, J = 16.8, 8.5 Hz, 1H), 3.70 (td, J = 9.1, 3.1 Hz, 1H), 3.28 (dd, J = 13.7, 4.1 Hz, 1H), 2.96 (ddd, J = 17.6, 8.9, 4.1 Hz, 1H), 2.86 (dd, J = 13.7, 8.9 Hz, 1H), 2.32 (s, 3H), 2.27 - 2.21 (m, 1H), 1.93 - 1.85 (m, 1H).

### Example 15: Synthesis route of compound 15

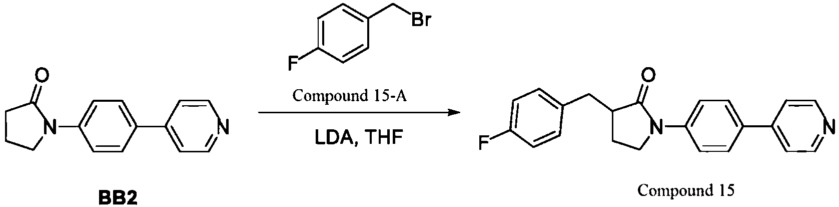

### Step 1: Compound 15 3-(4-fluorobenzyl)-1-(4-(pyridin-4-yl)phenyl)pyrrolidin-2-one

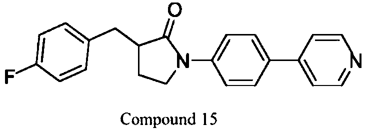

This step was carried out using compound 15-A 4-fluorobenzyl bromide as the raw material, referring to the synthesis procedure described in step 4 in Example 3.

LC_MS: (ES⁺): m/z 347.45 [M+H] ⁺.

¹H NMR (400 MHz, CDCl₃) δ 8.67 (d, *J* = 5.1 Hz, 2H), 7.79 (d, J= 8.8 Hz, 2H), 7.69 (d, *J= 8.8* Hz, 2H), 7.54 (d, *J* = 6.0 Hz, 2H), 7.23 (dd, *J* = 8.5, 5.5 Hz, 2H), 7.02 (t, *J* = 8.7 Hz, 2H), 3.79 (dt, *J* = 15.9, 8.0 Hz, 1 H), 3.68 (td, *J* = 9.1, 3.3 Hz, 1H), 3.28 (dd, *J* = 13.6, 4.0 Hz, 1H), 2.96 (ddd, *J* = 17.5, 8.8, 4.1 Hz, 1H), 2.86 (dd, *J* = 13.6, 8.9 Hz, 1H), 2.30 - 2.19 (m, 1H), 1.89 (dd, J= 8.7, 4.1 Hz, 1H).

### Example 16: Synthesis route of compound 16

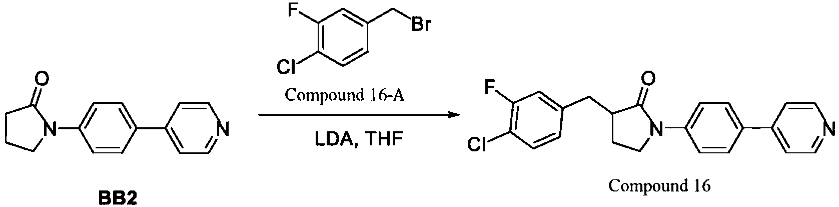

### Step 1: Compound 16 3-(4-chloro-3-fluorobenzyl)-1-(4-(pyridin-4-yl)phenyl)pyrrolidin-2-one

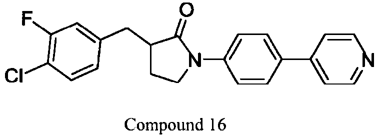

This step was carried out using compound 16-A 3- fluoro-4-chlorobenzyl bromide as the raw material, referring to the synthesis procedure described in step 4 in Example 3.

LC_MS: (ES⁺): m/z 381.05 [M+H] ⁺.

¹H NMR (400 MHz, CDCl₃) δ 8.67 (d, *J* = 5.7 Hz, 2H), 7.79 (d, *J* = 8.7 Hz, 2H), 7.69 (d, *J* = 8.7 Hz, 2H), 7.53 (d, *J* = 5.9 Hz, 2H), 7.35 (t, J= 7.9 Hz, 1H), 7.09 (dd, *J* = 9.9, 1.5 Hz, 1H), 7.01 (d, *J =* 8.2 Hz, 1H), 3.82 (dd, *J =* 16.7, 8.9 Hz, 1H), 3.74 (td, *J* = 9.1, 2.7 Hz, 1H), 3.29 (dd, *J* = 13.8, 4.2 Hz, 1H), 3.01 - 2.92 (m, 1H), 2.85 (dd, *J* = 13.8, 9.0 Hz, 1H), 2.31 - 2.23 (m, 1H), 1.92 - 1.83 (m, 1H).

### Example 17: Synthesis route of compound 17

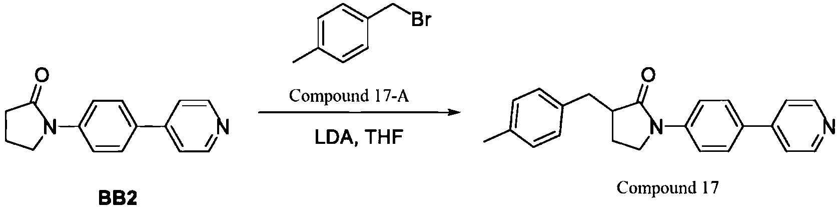

### Step 1: Compound 17 3-(4-methylbenzyl)-1-(4-(pyridin-4-yl)phenyl)pyrrolidin-2-one

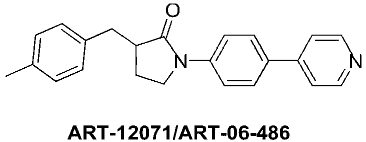

This step was carried out using compound 17-A 4-methylbenzyl bromide as the raw material, referring to the synthesis procedure described in step 4 in Example 3.

LC_MS: (ES⁺): m/z 343.50 [M+H] ⁺.

¹H NMR (400 MHz, CDCl₃) δ 8.67 (d, *J* = 6.0 Hz, 2H), 7.79 (d, *J* = 8.8 Hz, 2H), 7.69 (d, *J* = 8.8 Hz, 2H), 7.54 (dd, *J* = 4.7, 1.5 Hz, 2H), 7.20 - 7.10 (m, 4H), 3.77 (dt, *J* = 15.8, 7.9 Hz, 1H), 3.69 (td, *J* = 9.1, 3.4 Hz, 1H), 3.30 (dd, *J* = 13.7, 4.1 Hz, 1H), 2.96 (ddd, *J* = 17.8, 9.0, 4.1 Hz, 1H), 2.81 (dd, *J* = 13.7, 9.3 Hz, 1H), 2.35 (s, 3H), 2.28 - 2.19 (m, 1H), 1.91 - 1.86 (m, 1H).

### Example 18: Synthesis route of compound 18

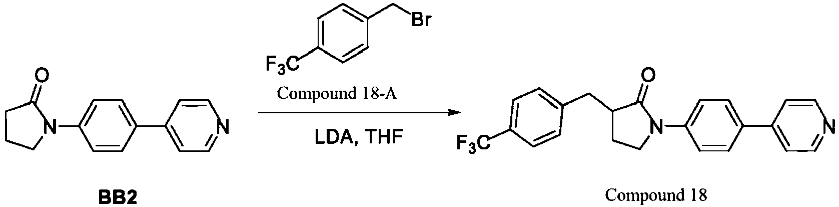

### Step 1: Compound 18 3-(4-trifluoromethylbenzyl)-1-(4-(pyridin-4-yl)phenyl)pyrrolidin-2-one

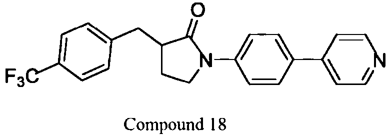

This step was carried out using compound 18-A 4-trifluoromethylbenzyl bromide as the raw material, referring to the synthesis procedure described in step 4 in Example 3.

LC_MS: (ES⁺): m/z 397.50 [M+H] ⁺.

### Example 19: Synthesis route of compound 19

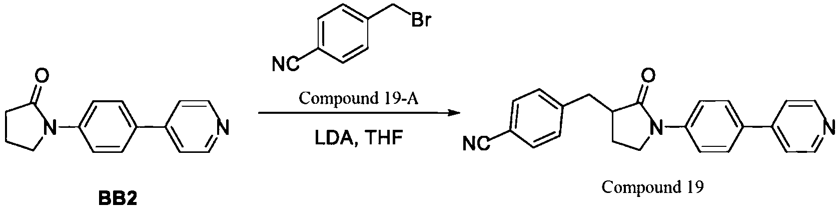

### Step 1: Compound 19 3-(4-cyanobenzyl)-1-(4-(pyridin-4-yl)phenyl)pyrrolidin-2-one

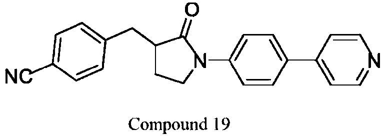

This step was carried out using compound 19-A 4-cyanobenzyl bromide as the raw material, referring to the synthesis procedure described in step 4 in Example 3.

LC_MS: (ES⁺): m/z 354.20 [M+H] ⁺.

¹H NMR (400 MHz, CDCl₃) δ 8.68 (d, *J* = 5.1 Hz, 2H), 7.79 (d, *J* = 8.6 Hz, 2H), 7.70 (d, *J* = 8.6 Hz, 2H), 7.64 (d, *J* = 8.0 Hz, 2H), 7.56 (d, *J* = 5.5 Hz, 2H), 7.40 (d, *J* = 8.1 Hz, 2H), 3.82 (dd, *J* = 16.7, 8.8 Hz, 1H), 3.73 (td, *J* = 9.1, 2.6 Hz, 1H), 3.38 (dd, *J* = 13.1, 3.7 Hz, 1H), 3.06 - 2.90 (m, 2H), 2.31 - 2.23 (m, 1H), 1.91 (dd, *J* = 10.7, 7.2 Hz, 1H).

### Example 20: Synthesis route of compound 20

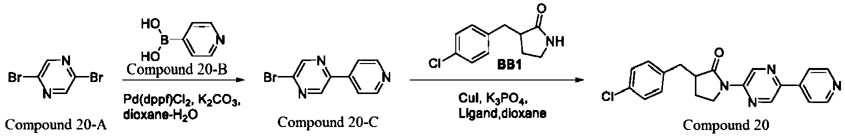

### Step 1: Compound 20-C 2-bromo-5-(pyridin-4-yl)pyrazine

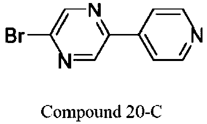

This step was carried out using compound 20-A 2,5-dibromopyrazine as the raw material, referring to the synthesis procedure described in step 1 in Example 6.

LC_MS: (ES⁺): m/z 235.95 [M+H] ⁺.

### Step 2: Compound 20 3-(4-chlorobenzyl)-1-(5-(pyridin-4-yl)pyrazin-2-yl)pyrrolidin-2-one

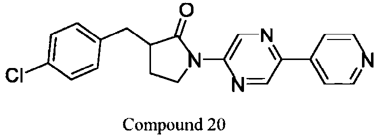

This step was carried out referring to the synthesis procedure described in step 2 in Example 6.

LC_MS: (ES⁺): m/z 365.10 [M+H] ⁺.

¹H NMR (400 MHz, CDCl₃) δ 9.88 (s, 1H), 8.83 (s, 1H), 7.94 (s, 2H), 7.42 - 7.12 (m, 6H), 4.13 - 4.01 (m, 1H), 3.95 - 3.81 (m, 1H), 3.31 (dd, J = 13.9, 4.3 Hz, 1H), 3.04 (qd, J = 9.3, 4.1 Hz, 1H), 2.85 (dd, J = 13.9, 9.1 Hz, 1H), 2.27 (dt, J = 14.8, 6.0 Hz, 1H), 1.96 - 1.88 (m, 1H).

### Example 21: Synthesis route of compound 21

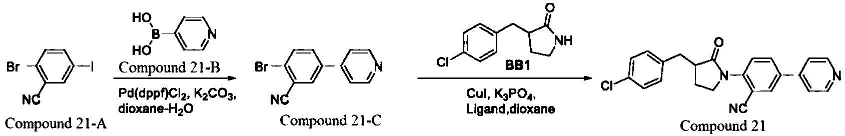

### Step 1: Compound 21-C 2-bromo-5-(pyridin-4-yl)benzonitrile

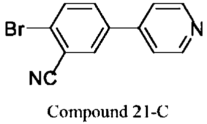

This step was carried out using compound 21-A 2-bromo-5-iodobenzonitrile as the raw material, referring to the synthesis procedure described in step 1 in Example 6.

LC_MS: (ES⁺): m/z 259.02 [M+H] ⁺.

### Step 2: Compound 21 2-(3-(4-chlorobenzyl)-2-pyrrolidinone- 1-yl)-5-(pyridin-4-yl)benzonitrile

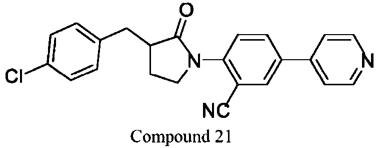

This step was carried out referring to the synthesis procedure described in step 2 in Example 6.

LC_MS: (ES⁺): m/z 388.20 [M+H] ⁺.

### Example 22: Synthesis route of compound 22

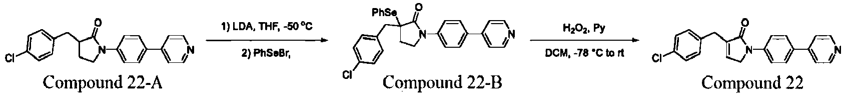

### Step 1: Compound 22-B 3-(4-chlorobenzyl)-3-phenylselenyl-1-(4-(4-pyridin-4-yl)phenyl)pyrrolidin-2-one

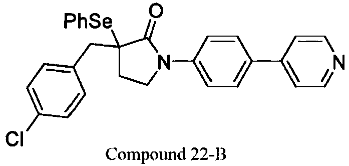

Under nitrogen atmosphere, compound 22-A 3-(4-chlorobenzyl)-1-(4-(pyridin-4-yl)phenyl)pyrrolidin-2-one (180 mg, 0.5 mmol) was dispersed in 2 mL of tetrahydrofuran, and lithium diisopropylamide (0.4 ml, 2N) was added dropwise at -50°C. After 0.5 h, a solution of compound benzeneselenenyl bromide (140 mg, 0.6 mmol) in tetrahydrofuran was added dropwise. The reaction solution was stirred at low temperature for 1 h. After the target mass was detected by LCMS, and the major spot was detected by TLC, the saturated ammonium chloride solution (20 mL) was added to quench the reaction. The reaction solution was extracted with dichloromethane (10 mL×3), washed with water (15 mL), saturated ammonium chloride solution (20 mL) and saturated saline (20 mL) in sequence, and then subjected to rotary evaporation to remove the reaction solvent. The resulting crude product was purified by using preparative silica gel plate (DCM:MeOH = 20:1), and dried in vacuum to obtain compound 22-B 3-(4-chlorobenzyl)-3-phenylselenyl-1-(4-(4-pyridin-4-yl)phenyl)pyrrolidin-2-one as a white solid (40 mg).

LC_MS: (ES⁺): m/z 519.05 [M+H] ⁺.

### Step 2: Compound 22 3-(4-chlorobenzyl)-1-(4-pyridin-4-yl)phenyl)-1,5-dihydro-2H-pyrrolidin-2-one

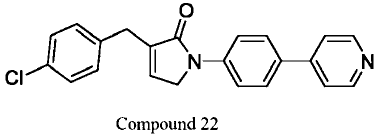

Compound 22-B 3-(4-chlorobenzyl)-3-phenylselenyl-1-(4-(4-pyridin-4-yl)phenyl)pyrrolidin-2-one (40 mg, 0.1 mmol) was dissolved in 2 mL of dichloromethane at -78°C, and hydrogen peroxide (0.1 ml, 30%wt) was added dropwise. After 0.5 h, a drop of pyridine (15 mg, 0.2 mmol) was added. The reaction solution was stirred at low temperature for 0.5 h, and then stirred at room temperature. After the target mass was detected by LCMS, the saturated ammonium chloride solution (10 mL) was added to quench the reaction. The reaction solution was extracted with dichloromethane (10 mL×3), washed with water (15 mL), saturated ammonium chloride solution (20 mL) and saturated saline (20 mL) in sequence, and then subjected to rotary evaporation to remove the reaction solvent. The resulting crude product was purified by using a preparative reverse phase column, and lyophilized to obtain compound 22 3-(4-chlorobenzyl)-1-(4-pyridin-4-yl)phenyl)-1,5-dihydro-2H-pyrrolidin-2-one as a white solid (5 mg).

LC_MS: (ES⁺): m/z 361.50 [M+H] ⁺.

### Example 23: Synthesis route of compound 23

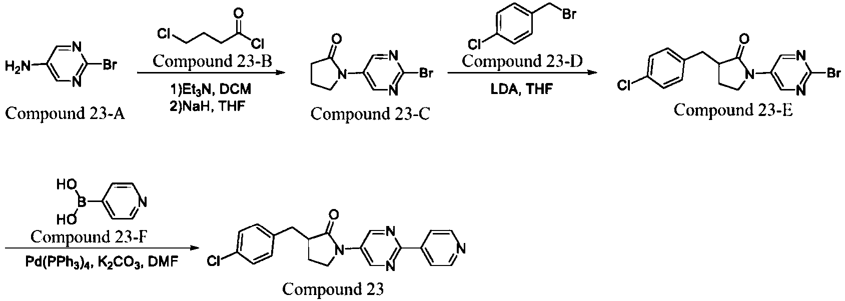

### Step 1: Compound 23-C 1-(2-bromopyrimidin-5-yl)pyrrolidin-2-one

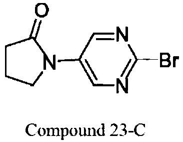

This step was carried out using compound 23-A 2-bromopyrimidin-5-amine as the raw material, referring to the synthesis procedure described in step 1 in Example 10.

LC_MS: (ES⁺): m/z 241.90 [M+H] ⁺.

### Step 2: Compound 23-E 1-(2-bromopyrimidin-5-yl)-3-(4-chlorobenzyl)pyrrolidin-2-one

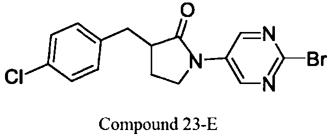

This step was carried out referring to the synthesis procedure described in step 2 in Example 10.

LC_MS: (ES⁺): m/z 241.05 [M+H] ⁺.

### Step 3: Compound 23 3-(4-chlorobenzyl)-1-(2-(pyridin-4-yl)pyrimidin-5-yl)pyrrolidin-2-one

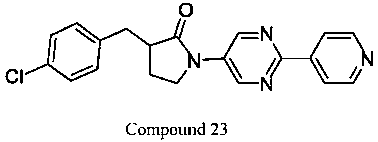

This step was carried out referring to the synthesis procedure described in step 3 in Example 10.

LC_MS: (ES⁺): m/z 365.10 [M+H] ⁺.

### Example 24: Synthesis route of compound 24

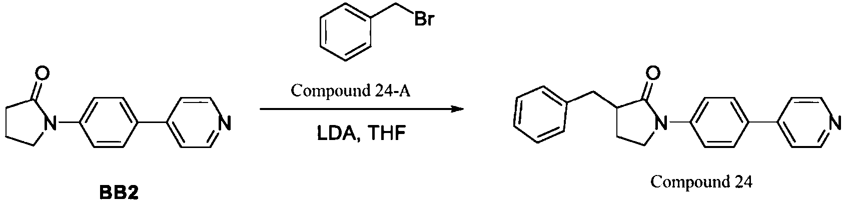

### Step 1: Compound 24 3-benzyl-1-(4-(pyridin-4-yl)phenyl)pyrrolidin-2-one

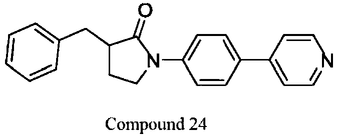

This step was carried out using compound 24-A benzyl bromide as the raw material, referring to the synthesis procedure described in step 4 in Example 3.

LC_MS: (ES⁺): m/z 329.20 [M+H] ⁺.

### Example 25: Synthesis route of compound 25

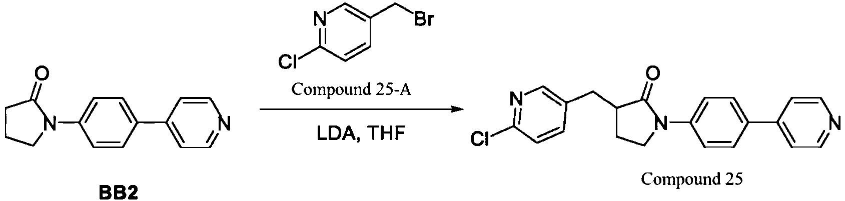

### Step 1: Compound 25 3-((6-chloropyridin-3-yl)methyl)-1-(4-(pyridin-4-yl)phenyl)pyrrolidin-2-one

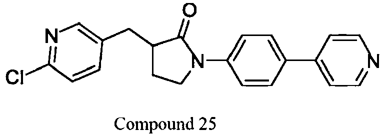

This step was carried out using compound 25-A 5-(bromomethyl)-2-chloropyridine as the raw material, referring to the synthesis procedure described in step 4 in Example 3.

LC_MS: (ES⁺): m/z 364.11 [M+H] ⁺.

### Example 26: Synthesis route of compound 26

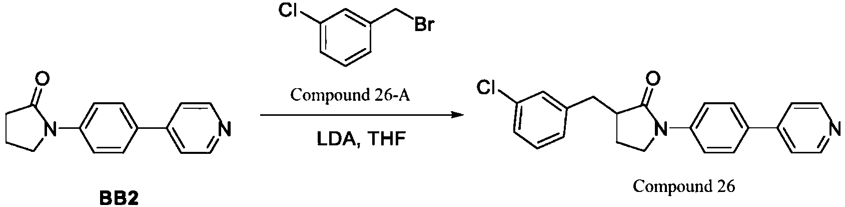

### Step 1: Compound 26 3-(3-chlorobenzyl)-1-(4-(pyridin-4-yl)phenyl)pyrrolidin-2-one

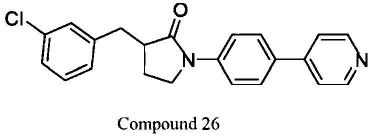

This step was carried out using compound 26-A 3-chlorobenzyl bromide as the raw material, referring to the synthesis procedure described in step 4 in Example 3.

LC_MS: (ES+): m/z 363.11 [M+H] +.

### Example 27: Synthesis route of compound 27

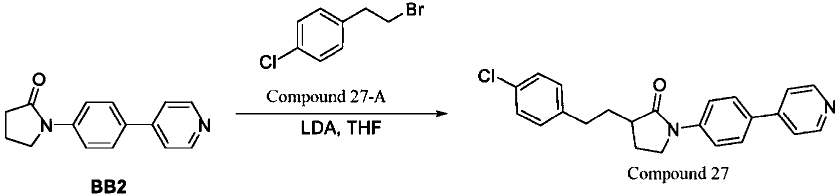

### Step 1: Compound 27 3-(4-chlorophenethyl)-1-(4-(pyridin-4-yl)phenyl)pyrrolidin-2-one

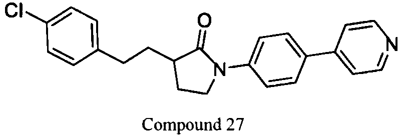

This step was carried out using compound 27-A 1-(2-bromoethyl)-4-chlorobenzene as the raw material, referring to the synthesis procedure described in step 4 in Example 3.

LC_MS: (ES⁺): m/z 377.13 [M+H] ⁺.

### Example 28: Synthesis route of compound 28

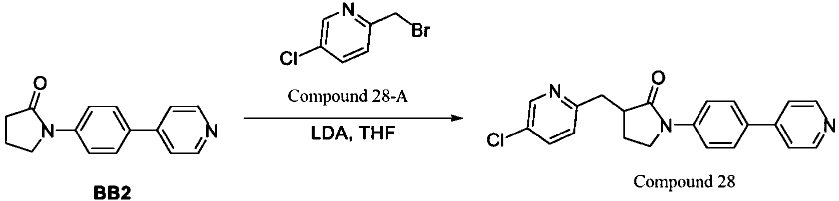

### Step 1: Compound 28 3-((5-chloropyridin-2-yl)methyl)-1-(4-(pyridin-4-ylphenyl)pyrrolidin-2-one

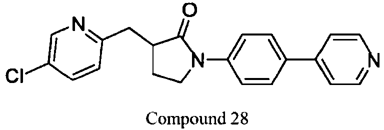

This step was carried out using compound 28-A2-bromomethyl-5-chloropyridine as the raw material, referring to the synthesis procedure described in step 4 in Example 3.

LC_MS: (ES⁺): m/z 364.11 [M+H] ⁺.

### Example 29: Synthesis route of compound 29

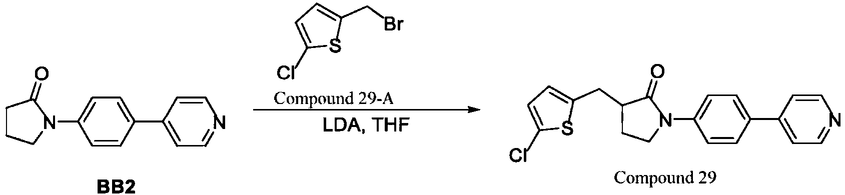

### Step 1: Compound 29 3-((5-chlorothiophen-2-yl)methyl)-1-(4-(pyridin-4-yl)phenyl)pyrrolidin-2-one

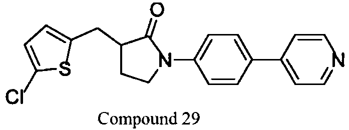

This step was carried out using compound 29-A 2-(bromomethyl)-5-chlorothiophene as the raw material, referring to the synthesis procedure described in step 4 in Example 3.

LC_MS: (ES⁺): m/z 369.10 [M+H] ⁺.

### Example 30: Synthesis route of compound 30

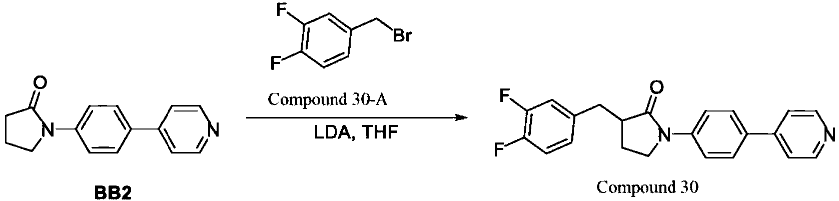

### Step 1: Compound 30 3-(3,4-difluorobenzyl)-1-(4-(pyridin-4-yl)phenyl)pyrrolidin-2-one

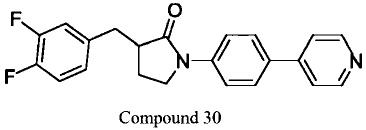

This step was carried out using compound 30-A 3,4-difluorobenzyl bromide as the raw material, referring to the synthesis procedure described in step 4 in Example 3.

LC_MS: (ES⁺): m/z 364.15 [M+H] ⁺.

### Example 31: Synthesis route of compound 31

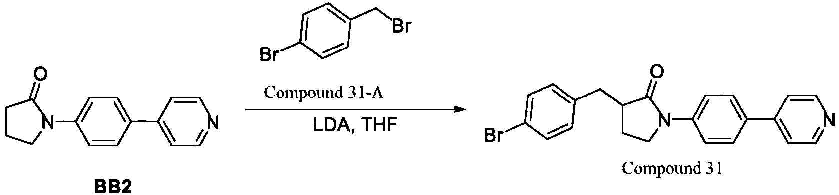

### Step 1: Compound 31 3-(4-bromobenzyl)-1-(4-(pyridin-4-yl)phenyl)pyrrolidin-2-one

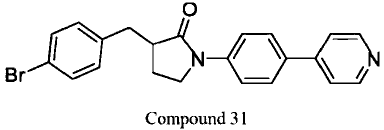

This step was carried out using compound 31-A 4-bromobenzyl bromide as the raw material, referring to the synthesis procedure described in step 4 in Example 3.

LC_MS: (ES⁺): m/z 407.08 [M+H] ⁺.

### Example 32: Synthesis route of compound 32

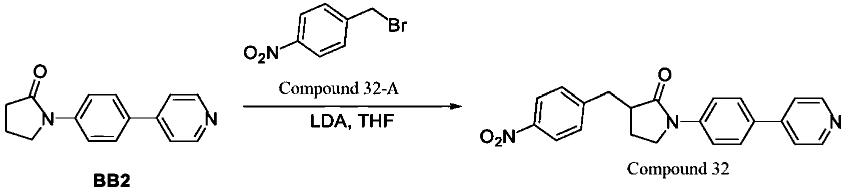

### Step 1: Compound 32 3-(4-nitrobenzyl)-1-(4-(pyridin-4-yl)phenyl)pyrrolidin-2-one

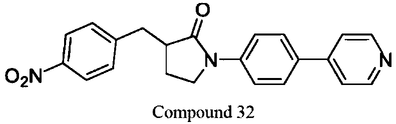

This step was carried out using compound 32-A 4-nitrobenzyl bromide as the raw material, referring to the synthesis procedure described in step 4 in Example 3.

LC_MS: (ES⁺): m/z 374.15 [M+H] ⁺.

### Example 33: Synthesis route of compound 33

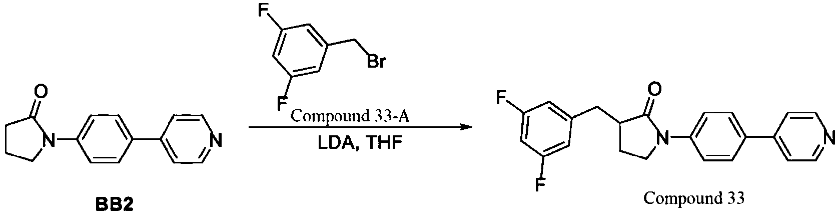

### Step 1: Compound 33 3-(3,5-difluorobenzyl)-1-(4-(pyridin-4-yl)phenyl)pyrrolidin-2-one

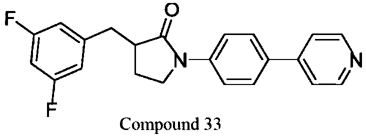

This step was carried out using compound 33-A 3,5-difluorobenzyl bromide as the raw material, referring to the synthesis procedure described in step 4 in Example 3.

LC_MS: (ES⁺): m/z 365.15 [M+H] ⁺.

### Example 34: Synthesis route of compound 34

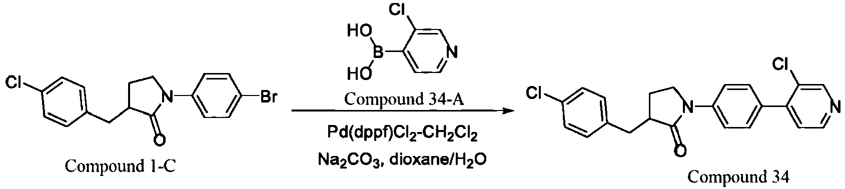

### Step 1: Compound 34 3-(4-chlorobenzyl)-1-(4-(3-chloropyridin-4-yl)phenyl)pyrrolidin-2-one

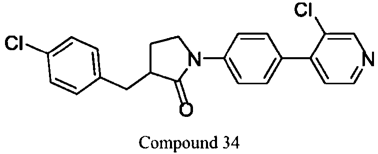

This step was carried out using compound 34-A 3-chloropyridin-4-boronic acid as the raw material, referring to the synthesis procedure described in step 2 in Example 1.

LC_MS: (ES+): m/z 397.12 [M+H] +.

### Example 35: Synthesis route of compound 35

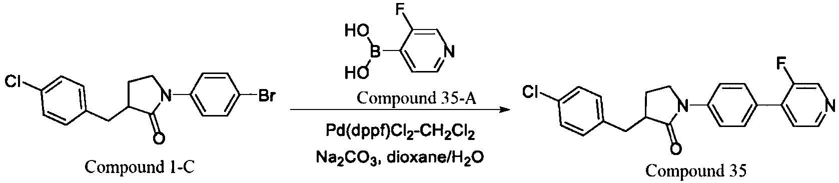

### Step 1: Compound 35 3-(4-chlorobenzyl)-1-(4-(3-fluoropyridin-4-yl)phenyl)pyrrolidin-2-one

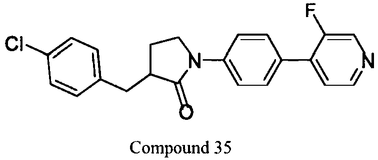

This step was carried out using compound 35-A 3-fluoropyridin-4-boronic acid as the raw material, referring to the synthesis procedure described in step 2 in Example 1.

LC_MS: (ES+): m/z 381.11 [M+H] +.

### Example 36: Synthesis route of compound 36

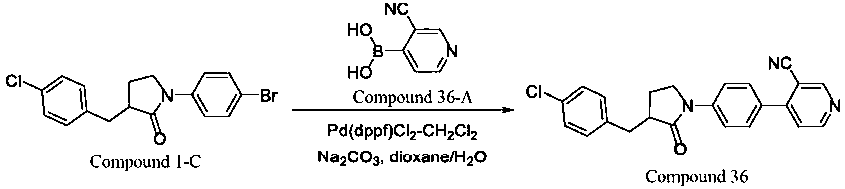

### Step 1: Compound 36 3-(4-chlorobenzyl)- 1-(4-(3-cyanopyridin-4-yl)phenyl)pyrrolidin-2-one

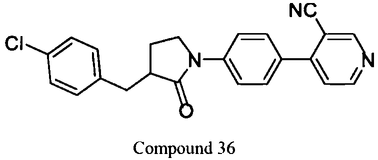

This step was carried out using compound 36-A 3-cyanopyridine-4-boronic acid acid as the raw material, referring to the synthesis procedure described in step 2 in Example 1.

LC_MS: (ES+): m/z 388.11 [M+H] +.

### Example 37: Synthesis route of compound 37

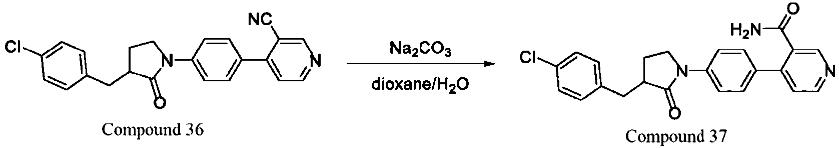

### Step 1: Compound 37 3-(4-chlorobenzyl)-1-(4-(nicotinamidopyridin-4-yl)phenyl)pyrrolidin-2-one

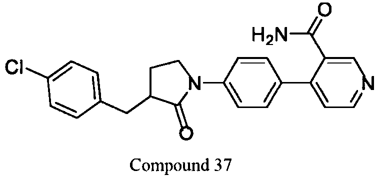

To a solution of compound 36 3-(4-chlorobenzyl)-1-(4-(3-cyanopyridin-4-yl)phenyl)pyrrolidin-2-one (50 mg, 128.91 µmol) in 1,4-dioxane (1 mL)/water (1 mL), sodium carbonate (40.99 mg, 386.73 µmol) was added. The reaction was carried out at 80°C for 3 h. After the reaction was completed, as monitored by LCMS, the reaction solution was cooled to room temperature and concentrated under reduced pressure. The residue was purified by preparative thin layer chromatography (eluent: a solution of 10% methanol in dichloromethane) to obtain compound 37 3-(4-chlorobenzyl)-1-(4-(nicotinamidopyridin-4-yl)phenyl)pyrrolidin-2-one as a yellow solid (20 mg).

LC_MS: (ES+): m/z 406.12 [M+H] +.

### Example 38: Synthesis route of compound 38

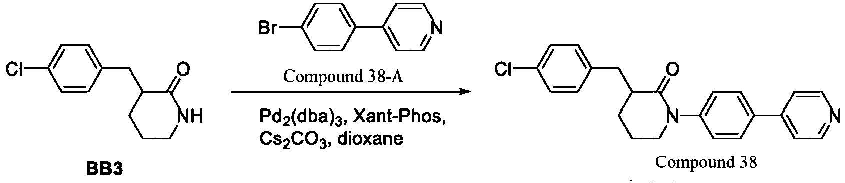

### Step 1: Compound 38 3-(4-chlorobenzyl)-1-(4-(pyridin-4-yl)phenyl)piperidin-2-one

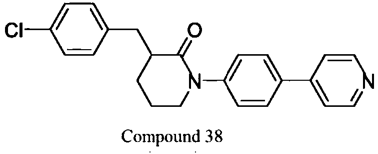

To a solution of intermediate BB3 3-(4-chlorobenzyl)piperidin-2-one (47.78 mg, 213.59 µmol) and compound 38-A4-(4-bromophenyl)pyridine (50 mg, 213.59 µmol) in dioxane (3 mL), Pd₂(dba)₃ (19.6 mg, 213.59 µmol), Xant-Phos (12.4 mg, 21.359 µmol) and cesium carbonate (208.77 mg, 640.76 µmol) were added. The reaction was carried out at 100°C for 3 h. After the reaction was completed, as monitored by LCMS, the reaction solution was cooled to room temperature, filtered and concentrated under reduced pressure. The residue was purified by preparative thin layer chromatography (eluent: a solution of 5% methanol in dichloromethane) to obtain compound 38 3-(4-chlorobenzyl)-1-(4-(pyridin-4-yl)phenyl)piperidin-2-one as a yellow solid (60 mg).

LC_MS: (ES+): m/z 377.12 [M+H] +.

### Example 39: Synthesis route of compound 39

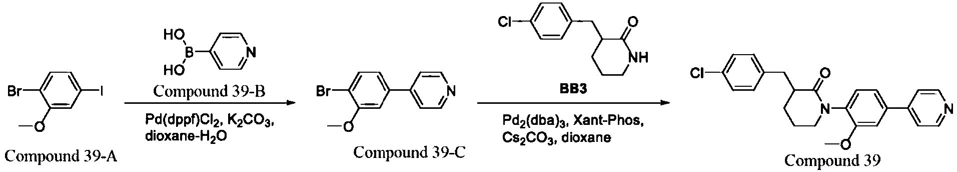

### Step 1: Compound 39-C 4-(4-bromo-3-methoxyphenyl)pyridine

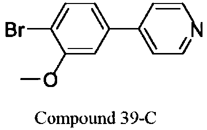

This step was carried out using compound 39-A 1-bromo-4-iodo-2-methoxybenzene as the raw material, referring to the synthesis procedure described in step 2 in Example 1.

LC_MS: (ES+): m/z 264.00 [M+H] +.

### Step 2: Compound 39 3-(4-chlorobenzyl)-1-(2-methoxy-4-(pyridin-4-yl)phenyl)piperidin-2-one

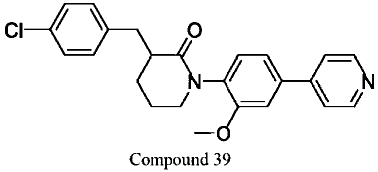

This step was carried out using compound 39-C 4-(4-bromo-3-methoxyphenyl)pyridine as the raw material, referring to the synthesis procedure described in step 1 in Example 38.

LC_MS: (ES+): m/z 407.14 [M+H] +.

### Example 40: Synthesis route of compound 40

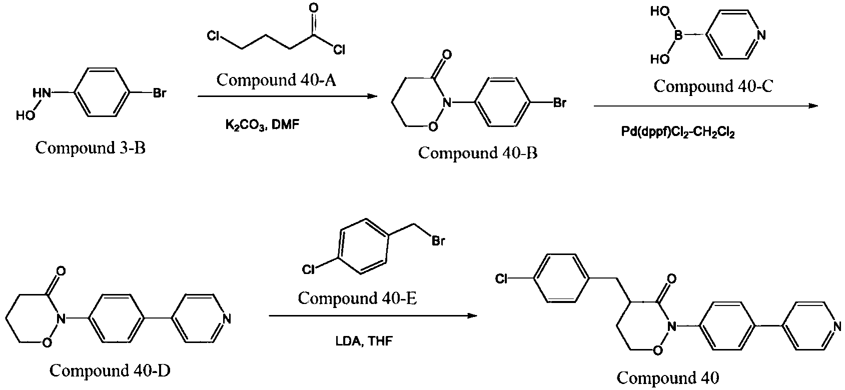

### Step 1: Compound 40-B 2-(4-bromophenyl)-1,2-Oxynitrogen-3-one

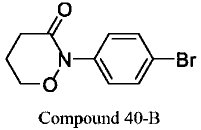

This step was carried out using compound 40-A 4-chlorobutyryl chloride as the raw material, referring to the synthesis procedure described in step 2 in Example 3.

LC_MS: (ES+): m/z 256.02 [M+H] +.

### Step 2: Compound 40-D 2-(4-(pyridin-4-yl)phenyl)-1,2-oxaza-3-one

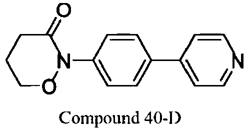

This step was carried out using compound 40-B 2-(4-bromophenyl)-1,2-oxaza-3-one as the raw material, referring to the synthesis procedure described in step 3 in Example 3.

LC_MS: (ES+): m/z 255.11 [M+H] +.

### Step 3: Compound 40 4-(4-chlorobenzyl)-2-(4-(pyridin-4-yl)phenyl)-1,2-oxaza-3-one

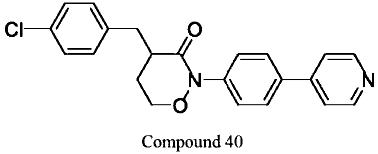

This step was carried out using compound 40-D 2-(4-(pyridin-4-yl)phenyl)-1,2-oxaza-3-one as the raw material, referring to the synthesis procedure described in step 4 in Example 3.

LC_MS: (ES+): m/z 379.11 [M+H] +.

### Example 41: Synthesis route of compound 41

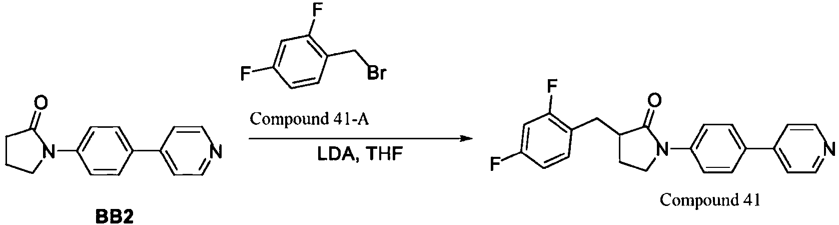

### Step 1: Compound 41 3-(2,4-difluorobenzyl)-1-(4-(pyridin-4-yl)phenyl)pyrrolidin-2-one

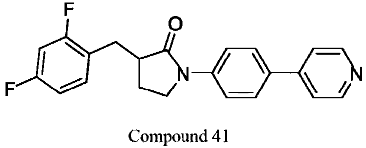

This step was carried out using compound 41-A 2,4-difluorobenzyl bromide as the raw material, referring to the synthesis procedure described in step 4 in Example 3.

LC_MS: (ES⁺): m/z 365.15 [M+H] ⁺.

### Example 42: Synthesis route of compound 42

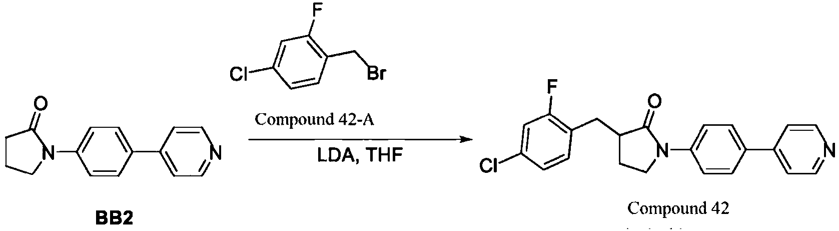

### Step 1: Compound 42 3-(4-chloro-2-fluorobenzyl)-1-(4-(pyridin-4-yl)phenyl)pyrrolidin-2-one

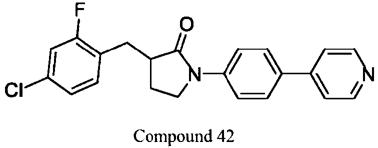

This step was carried out using compound 42-A4-chloro-2-fluorobenzyl bromide as the raw material, referring to the synthesis procedure described in step 4 in Example 3.

LC_MS: (ES⁺): m/z 365.15 [M+H] ⁺.

### Example 43: Synthesis route of compound 43

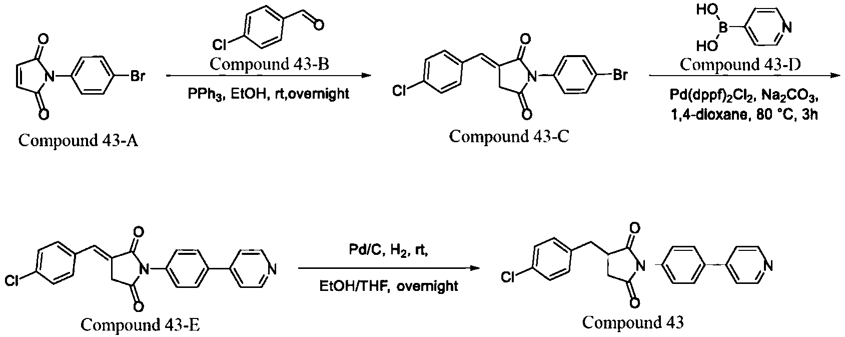

### Step 1: Compound 43-C (E)-1-(4-bromophenyl)-3-(4-chlorobenzylidene)pyrrolidin-2,5-dione

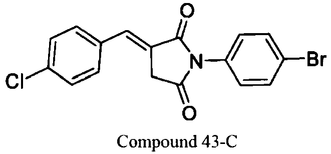

Compound 43-A N-(4-bromophenyl)maleimide (250 mg, 0.99 mmol) and compound 43-B (154 mg, 1.10 mmol) were weighed and dissolved in 10 mL of anhydrous ethanol Triphenylphosphine (274 mg, 1.05 mmol) was added at room temperature. The reaction was carried out overnight at room temperature. After the reaction was completed, the reaction solution was filtered, and washed twice with ethanol The resulting filter cake was dried in vacuum to obtain compound 43-C (E)-1-(4-bromophenyl)-3-(4-chlorobenzylidene)pyrrolidin-2,5-dione (240 mg).

LC_MS: (ES⁺): m/z 377.90 [M+H] +.

### Step 2: Compound 43-E (E)-3-(4-4-chlorobenzylidene)-1-(4-(pyridin-4-yl)phenyl)pyrrolidin-2,5-dione

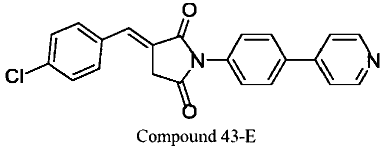

This step was carried out using compound 43-C (E)-1-(4-bromophenyl)-3-(4-chlorobenzylidene)pyrrolidin-2,5-dione as the raw material, referring to the synthesis procedure described in step 3 in Example 3.

LC_MS: (ES⁺): m/z 375.15 [M+H] ⁺.

### Step 3: Compound 43 3-(4-chlorobenzyl)-1-(4-(pyridin-4-yl)phenyl)pyrrolidin-2,5-one

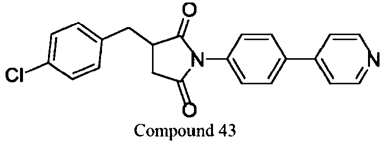

12 mg of compound 43-E (E)-3-(4-4-chlorobenzylidene)-1-(4-(pyridin-4-yl)phenyl)pyrrolidin-2,5-dione was weighed. 2 mL of anhydrous ethanol, 2 mL of tetrahydrofuran, and 2 mg of Pd/C were added. After replacement with hydrogen gas 3 times, the reaction was carried out overnight at room temperature. Following separation by preparative thin layer chromatography, further purification was carried out by pre-HPLC to obtain compound 43 3-(4-chlorobenzyl)-1-(4-(pyridin-4-yl)phenyl)pyrrolidin-2,5-one (3 mg).

LC_MS: (ES⁺): m/z 377.05 [M+H]⁺

¹H NMR (400 MHz, Chloroform-*d*) *δ* 8.71 (d, *J =* 5.1 Hz, 2H), 7.77-7.72 (*m,* 2H), 7.54-7.50 (*m,* 2H), 7.36 (dt, *J* = 8.3, 3.4 Hz, 4H), 7.20 *(d, J* = 8.1 Hz, 2H), 3.37 (*dt, J* = 8.7*,* 4.4 Hz, 1H), 3.26 (*dd, J* = 13.9, 4.6 Hz, 1H), 3.11 (*dd, J* = 14.0, 8.0 Hz, 1H), 2.96 (*dd, J =* 18.5, 9.2 Hz, 1H), 2.66 (*dd, J* = 18.5, 4.9 Hz, 1H).

### Example 44: Synthesis route of compound 53

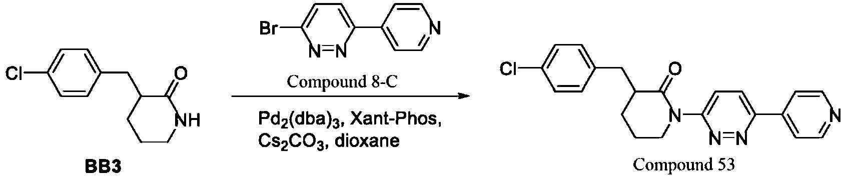

### Step 1: Compound 53 3-(4-chlorobenzyl)-1-(6-(pyridin-4-yl)pyridazin-3-yl)piperidin-2-one

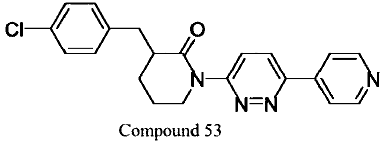

This step was carried out using intermediate BB3 3-(4-chlorobenzyl)piperidin-2-one and compound 8-C 3-bromo-6-(pyridin-4-yl)pyridazine as raw materials, referring to the synthesis procedure described in step 1 in Example 38.

LC_MS: (ES⁺): m/z 379.2 [M+H] ⁺.

¹H NMR (400 MHz, DMSO-d6) δ 8.81 - 8.75 (m, 2H), 8.37 (d, J = 9.3 Hz, 1H), 8.19 - 8.09 (m, 3H), 7.39 - 7.34 (m, 2H), 7.33 - 7.28 (m, 2H), 4.14 (ddd, J = 12.9, 8.3, 4.9 Hz, 1H), 4.06 - 3.94 (m, 1H), 3.25 (dd, J = 13.5, 4.6 Hz, 1H), 2.99 - 2.87 (m, 1H), 2.78 (dd, J = 13.6, 9.0 Hz, 1H), 2.06 - 1.76 (m, 3H), 1.56 (dtd, J = 13.0, 10.0, 5.2 Hz, 1H).

### Example 45: Synthesis route of compound 54

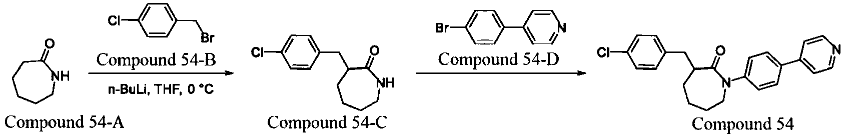

### Step 1: Compound 54-C 3-(4-chlorobenzyl)azacyclopentan-2-one

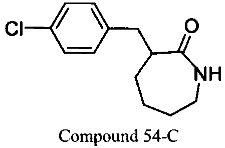

Compound 54-A caprolactam (565 mg, 5 mmol) was dissolved in tetrahydrofuran (50 mL). Under nitrogen protection, the reaction mixture was cooled to 0°C. n-Butyllithium (6.4 mL, 10.25 mmol) was added for 2 h of reaction at 0°C and then compound 54-B 4-chlorobenzyl bromide (1130 mg, 5.5 mmol) in tetrahydrofuran (10 mL) was slowly added for 1 h of reaction at 0°C. The reaction solution was quenched with an excess amount of aqueous saturated ammonium chloride solution. After phase separation, the aqueous phase was extracted with ethyl acetate (30 mL), and the organic phases were combined, washed with aqueous saturated sodium chloride solution (30 mL×2), dried over anhydrous sodium sulfate, subjected to suction filtration, concentrated under reduced pressure, separated and purified by silica gel column chromatography (eluent: a solution of 7.5% methanol in dichloromethane) to obtain compound 54-C 3-(4-chlorobenzyl)azacyclopentan-2-one (142 mg).

LC_MS: (ES⁺): m/z 238.0 [M+H] ⁺.

### Step 2: Compound 54 3-(4-chlorobenzyl)- 1-(4-(pyridin-4-yl)phenyl)azacyclopentan-2-one

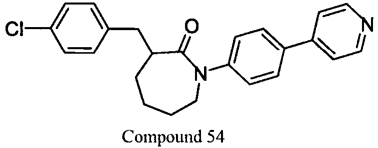

This step was carried out using compound 54-C 3-(4-chlorobenzyl)azacyclopentan-2-one and compound 54-D 4-(4-bromophenyl)pyridine as raw materials, referring to the synthesis procedure described in step 1 in Example 38.

LC_MS: (ES⁺): m/z 391.1 [M+H] ⁺.

¹H NMR (400 MHz, DMSO-d6) δ 8.67 - 8.60 (m, 2H), 7.84 - 7.77 (m, 2H), 7.74 - 7.68 (m, 2H), 7.35 (d, J = 5.7 Hz, 6H), 4.12 (dd, J = 15.4, 9.9 Hz, 1H), 3.59 (dd, J = 15.3, 4.9 Hz, 1H), 3.23 (q, J = 8.1 Hz, 2H), 3.06 (dd, J = 14.0, 6.6 Hz, 1H), 2.00 (q, J = 7.3 Hz, 1H), 1.85 (d, J = 15.0 Hz, 2H), 1.74 - 1.54 (m, 2H), 1.45 (t, J = 11.4 Hz, 1H).

### Example 46: Synthesis route of compound 55

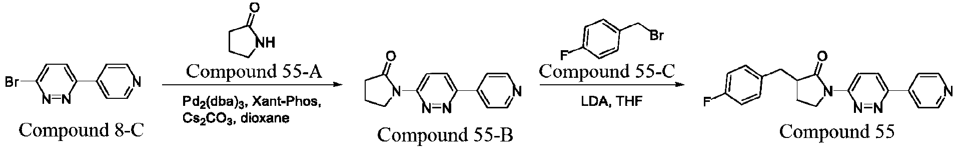

### Step 1: Compound 55-B 1-(6-(pyridin-4-yl)pyridazin-3-yl)pyrrolidin-2-one

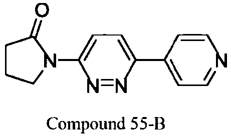

This step was carried out using compound 8-C 3-bromo-6-(pyridin-4-yl)pyridazine and compound 55-A pyrrolidin-2-one as raw materials, referring to the synthesis procedure described in step 1 in Example 38.

LC_MS: (ES⁺): m/z 241.10 [M+H] ⁺.

### Step 2: Compound 55 3-(4-fluorobenzyl)-1-(6-(pyridin-4-yl)pyridazin-3-yl)pyrrolidin-2-one

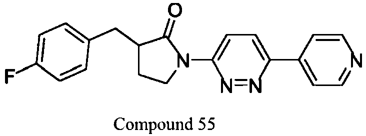

This step was carried out using compound 55-B 1-(6-(pyridin-4-yl)pyridazin-3-yl)pyrrolidin-2-one and compound 55-C 1-(bromomethyl)-4-fluorobenzene as raw materials, referring to the synthesis procedure described in step 4 in Example 3.

LC_MS: (ES⁺): m/z 349.12 [M+H] ⁺.

¹H NMR (400 MHz, DMSO-d6) δ 8.77 (dd, J = 4.5, 1.7 Hz, 2H), 8.73 (d, J = 9.5 Hz, 1H), 8.43 (d, J = 9.5 Hz, 1H), 8.14 - 8.08 (m, 2H), 7.38 - 7.29 (m, 2H), 7.19 - 7.10 (m, 2H), 4.23 - 4.12 (m, 2H), 3.96 (ddd, J = 10.9, 8.9, 7.4 Hz, 1H), 3.13 (ddt, J = 17.9, 9.1, 4.6 Hz, 2H), 2.78 (dd, J = 13.3, 9.0 Hz, 1H), 2.15 (q, J = 8.1, 7.5 Hz, 1H), 1.92 - 1.79 (m, 1H).

### Example 47: Synthesis route of compound 58

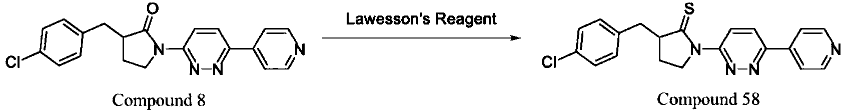

### Step 1: Compound 58 3-(4-chlorobenzyl)-1-(6-(pyridin-4-yl)pyridazin-3-yl)pyrrolidin-2-thione

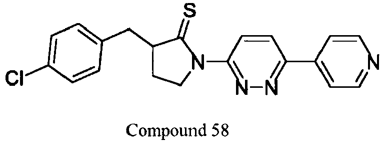

This step was carried out using compound 8 3-(4-chlorobenzyl)-1-(6-(pyridin-4-yl)pyridazin-3-yl)pyrrolidin-2-one as the raw material, referring to the synthesis procedure described in step 1 in Example 12.

LC_MS: (ES⁺): m/z 381.1 [M+H] ⁺.

¹H NMR (400 MHz, CDCl3) δ 9.50 (d, J = 9.3 Hz, 1H), 8.83 (d, J = 3.7 Hz, 2H), 8.02 (d, J = 9.2 Hz, 3H), 7.31 (d, J = 8.3 Hz, 2H), 7.24 (d, J = 8.3 Hz, 2H), 4.50 (dd, J = 8.3, 6.0 Hz, 2H), 3.65 (dd, J = 13.8, 4.1 Hz, 1H), 3.34 (ddd, J = 18.0, 8.5, 4.2 Hz, 1H), 2.86 - 2.78 (m, 1H), 2.31 (dt, J = 13.8, 5.9 Hz, 1H), 2.02 - 1.93 (m, 1H).

### Example 48: Synthesis route of compound 59

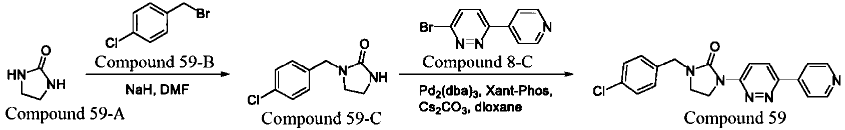

### Step 1: Compound 59-C 1-(4-chlorobenzyl)imidazolidin-2-one

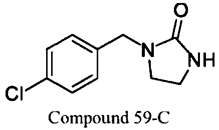

To a solution of compound 59-A imidazolidin-2-one (838 mg, 9.73 mmol) in DMF in an ice bath, sodium hydride (116.8 mg, 4.87 mmol) was added and stirred for 0.5 h. Compound 59-B 1-(bromomethyl)-4-chlorobenzene (1.0 g, 4.87 mmol) was then added dropwise and stirred at room temperature for 12 h. After the reaction was completed, as detected by LCMS, the reaction solution was poured into saturated ammonium chloride solution (20 mL), and extracted with ethyl acetate (10 mL×3). The organic layers were combined, washed with saturated saline (20 mL), dried over anhydrous sodium sulfate, and concentrated under reduced pressure. The residue was purified by silica gel column chromatography (eluent: a solution of 50-70% ethyl acetate in petroleum ether) to obtain compound 59-C 1-(4-chlorobenzyl)imidazolidin-2-one as a yellow solid (300 mg).

LC_MS: (ES⁺): m/z 211.04 [M+H] ⁺.

### Step 2: Compound 59 1-(4-chlorobenzyl)-3-(6-(pyridin-4-yl)pyridazin-3-yl)imidazolidin-2-one

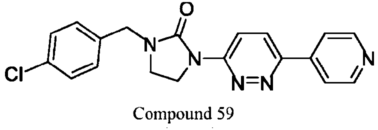

This step was carried out using compound 59-C 1-(4-chlorobenzyl)imidazolidin-2-one and compound 8-C 3-bromo-6-(pyridin-4-yl)pyridazine as raw materials, referring to the synthesis procedure described in step 1 in Example 38.

LC_MS: (ES⁺): m/z 366.10 [M+H] ⁺.

### Example 49: Synthesis route of compound 74

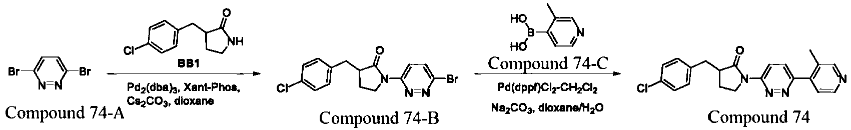

### Step 1: Compound 74-B 1-(6-bromopyridazin-3-yl)-3-(4-chlorobenzyl)pyrrolidin-2-one

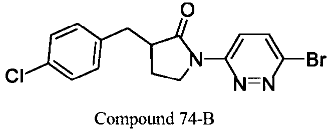

To a solution of compound 74-A 3,6-dibromopyridazine (500 mg, 2.10 mmol) and intermediate BB1 3-(4-chlorobenzyl)pyrrolidin-2-one (440.7 mg, 2.10 mmol) in dioxane (10 mL), Pd₂(dba)₃ (96.2 mg, 105 µmol), Xant-Phos (60.8 mg, 105 µmol) and cesium carbonate (1.71 g, 5.25 mmol) were added. Under nitrogen atmosphere, the reaction was carried out at 100°C for 2 h. After the reaction was completed, as monitored by LCMS, the reaction solution was cooled to room temperature, filtered and concentrated under reduced pressure. The residue was purified by preparative thin layer chromatography (eluent: a solution of 5% methanol in dichloromethane) to obtain compound 74-B 1-(6-bromopyridazin-3-yl)-3-(4-chlorobenzyl)pyrrolidin-2-one as a yellow solid (300 mg).

LC_MS: (ES⁺): m/z 366.02 [M+H] ⁺.

### Step 2: Compound 74 3-(4-chlorobenzyl)-1-(6-(3-methylpyridin-4-yl)pyridazin-3-yl)pyrrolidin-2-one

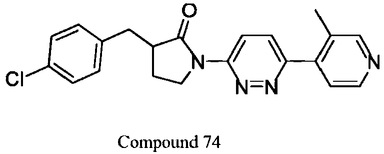

This step was carried out using compound 74-B 1-(6-bromopyridazin-3-yl)-3-(4-chlorobenzyl)pyrrolidin-2-one and compound 74-C 3-methylpyridin-4-boronic acid as raw materials, referring to the synthesis procedure described in step 2 in Example 1.

LC_MS: (ES⁺): m/z 379.10 [M+H] ⁺.

### Example 50: Synthesis route of compound 75

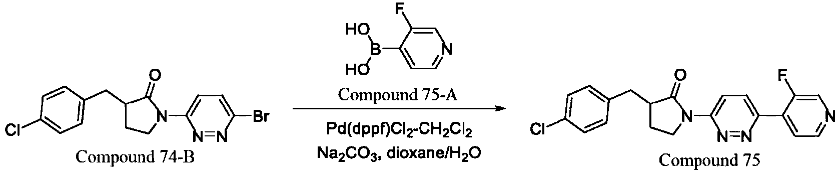

### Step 1: Compound 75 3-(4-chlorobenzyl)-1-(6-(3-fluoropyridin-4-yl)pyridazin-3-yl)pyrrolidin-2-one

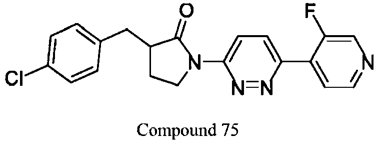

This step was carried out using compound 74-B 1-(6-bromopyridazin-3-yl)-3-(4-chlorobenzyl)pyrrolidin-2-one and compound 75-C 3-fluoropyridin-4-boronic acid as raw materials, referring to the synthesis procedure described in step 2 in Example 1.

LC_MS: (ES⁺): m/z 383.10 [M+H] ⁺.

### Example 51: Synthesis route of compound 76

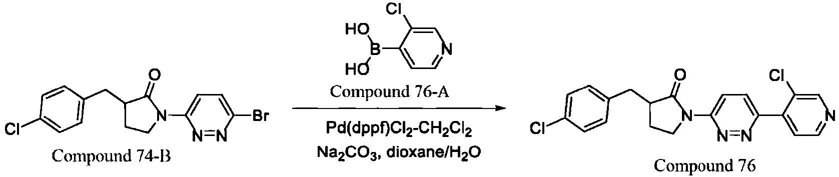

### Step 1: Compound 76 3-(4-chlorobenzyl)-1-(6-(3-chloropyridin-4-yl)pyridazin-3-yl)pyrrolidin-2-one

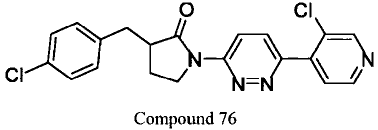

This step was carried out using compound 74-B 1-(6-bromopyridazin-3-yl)-3-(4-chlorobenzyl)pyrrolidin-2-one and compound 76-A 3-chloropyridin-4-boronic acid as raw materials, referring to the synthesis procedure described in step 2 in Example 1.

LC_MS: (ES⁺): m/z 399.05 [M+H] ⁺.

### Example 52: Synthesis route of compound 77

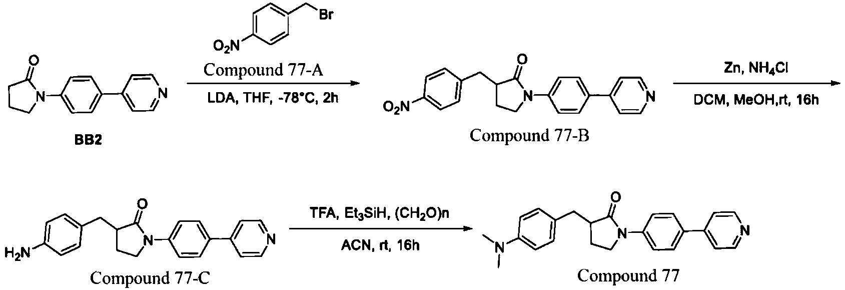

### Step 1: Compound 77-B 3-(4-nitrobenzyl)-1-(4-(pyridin-4-yl)phenyl)pyrrolidin-2-one

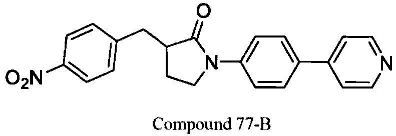

Intermediate BB2 1-(4-(pyridin-4-yl)phenyl)pyrrolidin-2-one (50 mg, 0.21 mmol) was dissolved in anhydrous tetrahydrofuran (4 mL). Under nitrogen protection, the reaction system was stirred at -78°C for 5 min. 2 M LDA (160 µL, 0.32 mmol) was slowly added dropwise, after which, the reaction was carried out for 30 min. Compound 77-A 1-(bromomethyl)-4-nitrobenzene (45 mg, 0.21 mmol) was then added dropwise, after which, the reaction was carried out at -78°C for 1 h. After the reaction was completed, an aqueous saturated ammonium chloride solution (5 mL) was added. After warmed up to room temperature, the reaction solution was diluted with 20 mL of ethyl acetate. Phase separation was performed, and the organic phase was retained, backwashed with saturated saline (20 mL), dried over anhydrous sodium sulfate, subjected to rotary evaporation, purified by preparative thin layer chromatography to obtain compound 77-B 3-(4-nitrobenzyl)-1-(4-(pyridin-4-yl)phenyl)pyrrolidin-2-one as a yellow solid (26 mg).

### Step 2: Compound 77-C 3-(4-aminobenzyl)-1-(4-(pyridin-4-yl)phenyl)pyrrolidin-2-one

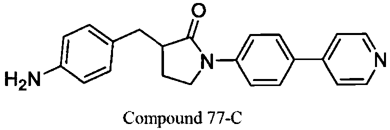

Compound 77-B 3-(4-nitrobenzyl)-1-(4-(pyridin-4-yl)phenyl)pyrrolidin-2-one (26 mg) was dissolved in a mixed solvent of dichloromethane and methanol (4 mL, v/v = 1:1) and placed in an ice bath. Zinc powder (36 mg, 0.56 mmol) and ammonium chloride (37 mg, 0.69 mmol) were added in sequence. The reaction system was slowly heated to room temperature for overnight reaction at room temperature. After the reaction was completed, the reaction system was filtered to remove solid impurities, washed with 20 mL of dichloromethane and methanol in sequence, and purified by preparative thin layer chromatography to obtain ompound 77-C 3-(4-aminobenzyl)-1-(4-(pyridin-4-yl)phenyl)pyrrolidin-2-one as a yellow solid (20 mg).

LC_MS: (ES⁺): m/z 344.2 [M+H] ⁺.

### Step 3: Compound 77 3-(4-(dimethylamino)benzyl)-1-(4-(pyridin-4-yl)phenyl)pyrrolidin-2-one

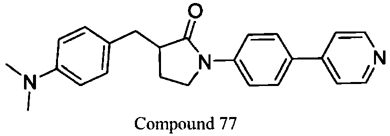

Compound 77-C 3-(4-aminobenzyl)-1-(4-(pyridin-4-yl)phenyl)pyrrolidin-2-one (20 mg) was dissolved in acetonitrile (2 mL) and placed in an ice bath. Paraformaldehyde (52 mg, 0.58 mmol), Et₃SiH (34 mg, 0.29 mmol) and trifluoroacetic acid (33 mg, 0.29 mmol) were added in sequence. The reaction system was warmed up to room temperature and reacted overnight. After the reaction was completed, the organic solvent was removed under vacuum. The residue was redissolved in methanol, adjusted to pH 10-11 with saturated sodium bicarbonate solution, subjected to rotary evaporation, dissolved, and subjected to two rounds of separation and purification by preparative thin layer chromatography to obtain compound 77 3-(4-(dimethylamino)benzyl)-1-(4-(pyridin-4-yl)phenyl)pyrrolidin-2-one (4 mg).

LC_MS: (ES⁺): m/z 372.2 [M+H] ⁺.

¹H NMR (400 MHz, Chloroform-*d*) *δ* 8.68 (*d, J =* 5.3 Hz, 2H), 7.83-7.75 (*m,* 2H), 7.74-7.68 (*m,* 2H), 7.59 (*d, J* = *5.5* Hz, 2H), 7.15 (*d, J* = 8.5 Hz, 2H), 6.76 (*d, J =* 8.1 Hz, 2H), 3.80-3.72 (*m,* 2H), 3.24-3.20 *(m,* 1H), 2.96-2.91 (*m,* 6H), 2.80-2.75 (*m,* 1H), 2.25-2.21 (*m,* 1H), 1.94-1.90 (*m,* 1H).

### Example 53: Synthesis route of compound 78

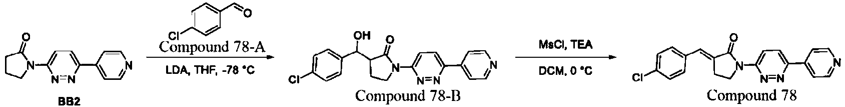

### Step 1: Compound 78-B 3-((4-chlorophenyl)(hydroxy)methyl)-1-(6-(pyridin-4-yl)pyridazin-3-yl)pyrrolidin-2-one

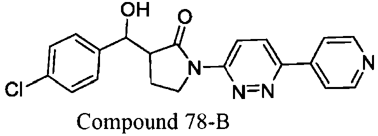

Compound BB2 1-(4-(pyridin-4-yl)phenyl)pyrrolidin-2-one (25 mg, 0.1 mmol) was dissolved in 10 mL of tetrahydrofuran at -78°C, and lithium diisopropylamide (1.5 eq, 0.075 mL, 2N) was added dropwise. After 0.5 h, a solution of compound 78-A4-chlorobenzaldehyde (14 mg, 0.1 mmol) in tetrahydrofuran (10 mL) was added dropwise. The reaction solution was stirred at low temperature for 1 h. After complete reaction of the raw material and target mass were detected by LCMS, and formation of the major spot was detected by TLC, the saturated ammonium chloride solution (10 mL) was added to quench the reaction. The reaction solution was extracted with dichloromethane (10 mL×3), washed with water (15 mL) and saturated saline (20 mL) in sequence, and then subjected to rotary evaporation to remove the reaction solvent. The resulting crude product was purified by using preparative silica gel plate (DCM:MeOH = 20:1), and dried in vacuum to obtain compound 78-B 3-((4-chlorophenyl)(hydroxy)methyl)-1-(6-(pyridin-4-yl)pyridazin-3-yl)pyrrolidin-2-one as a yellow solid (30 mg).

LC_MS: (ES⁺): m/z 381.1 [M+H] ⁺.

### Step 2: Compound 78 (E)-3-(4-chlorobenzylidene)-1-(6-(pyridin-4-yl)pyridazin-3-yl)pyrrolidin-2-one

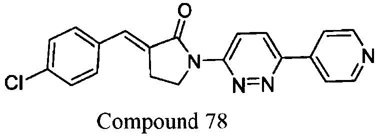

Compound 78-B 3-((4-chlorophenyl)(hydroxy)methyl)-1-(6-(pyridin-4-yl)pyridazin-3-yl)pyrrolidin-2-one (20 mg, 0.05 mmol) was dissolved in 1 mL of dichloromethane at 0°C, and triethylamine (25 mg, 0.25 mmol, 5 eq) was added dropwise. After 0.5 h, a solution of methanesulfonyl chloride (11 mg, 0.1 mmol, 2 eq) in dichloromethane (0.5 mL) was added dropwise. The reaction solution was stirred at low temperature for 1 h. After complete reaction of the raw material and target mass were detected by LCMS, and formation of the major spot was detected by TLC, water (10 mL) was added to quench the reaction. The reaction solution was extracted with dichloromethane (10 mL×3), washed with saturated saline (20 mL), and then subjected to rotary evaporation to remove the reaction solvent. The resulting crude product was purified by using preparative silica gel plate (DCM:MeOH = 20:1), and dried in vacuum to obtain compound 78 (E)-3-(4-chlorobenzylidene)-1-(6-(pyridin-4-yl)pyridazin-3-yl)pyrrolidin-2-one as a white solid (10 mg).

LC_MS: (ES⁺): m/z 363.1 [M+H] ⁺.

### Example 54: Synthesis route of compound 80

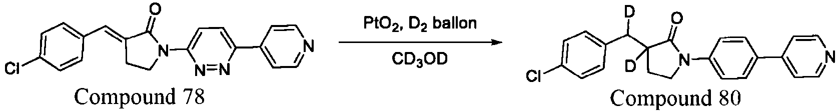

### Step 1: Compound 80 3-((4-chlorophenyl)methyl-deutero)-1-(4-(pyridin-4-yl)phenyl)-3-deutero-pyrrolidin-2-one

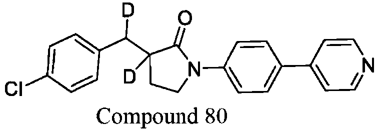

Compound 78 (E)-3-(4-chlorobenzylidene)-1-(6-(pyridin-4-yl)pyridazin-3-yl)pyrrolidin-2-one (10 mg, 0.027 mmol) was dissolved in 1 mL of deuterated methanol at room temperature. A catalytic amount of platinum dioxide was added, and the reaction system was stirred overnight at room temperature under a deuterium balloon for reaction. After complete reaction of the raw material and target mass were detected by LCMS, and formation of the major spot was detected by TLC, the reaction solution was subjected to rotary evaporation to remove the solvent. The residue was diluted with dichloromethane, filtered, and subjected to rotary evaporation to remove the solvent. The resulting crude product was purified using preparative silica gel plate (developing agent: DCMMeOH = 20:1), and dried in vacuum to obtain compound 80 3-((4-chlorophenyl)methyl-deutero)-1-(4-(pyridin-4-yl)phenyl)-3-deutero-pyrrolidin-2-one (1 mg).

LC_MS: (ES⁺): m/z 365.2 [M+H] ⁺.

### Example 55: Preparation of compounds 81 and 82 through chiral separation

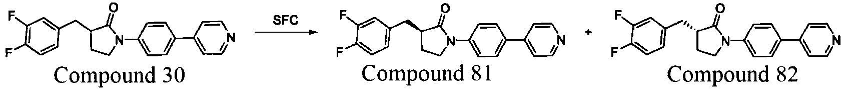

Compound 30 3-(3,4-difluorobenzyl)-1-(4-(pyridin-4-yl)phenyl)pyrrolidin-2-one (50 mg) was dissolved in isopropanol. Using an AD-H chiral column, the chiral separation was carried out under conditions of: detection wavelength: 280 nm, column temperature: 25°C, pressure: 5 MPa, mobile phase: n-hexane:isopropanol = 65:35, and flow rate: 0.75 mL/min. Peak 1 (retention time = 19.52 min) and peak 2 (retention time = 25.78 min) were collected, and concentrated under reduced pressure to obtain compound 81 (R)-3-(3,4-difluorobenzyl)-1-(4-(pyridin-4-yl)phenyl)pyrrolidin-2-one, and compound 82 (R)-3-(3,4-difluorobenzyl)-1-(4-(pyridin-4-yl)phenyl)pyrrolidin-2-one.

### Example 56: Synthesis route of compound 83

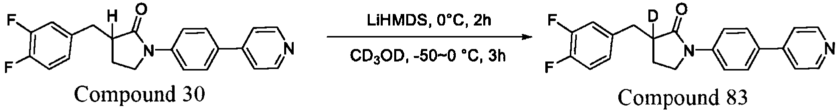

### Step 1: Compound 83 3-(3,4-difluorobenzyl)-1-(4-(pyridin-4-yl)phenyl)-3-deutero-pyrrolidin-2-one

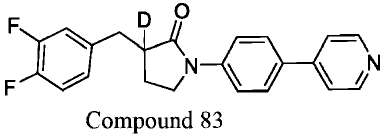

Under nitrogen protection, a solution of compound 30 3-(3,4-difluorobenzyl)-1-(4-(pyridin-4-yl)phenyl)pyrrolidin-2-one (10.92 g, 30 mmol) in tetrahydrofuran (220 mL) was cooled to 0°C, and lithium bis(trimethylsilyl)amide (150 mL, 150 mmol) was added. The reaction was carried out at 0°C for 2 h. To a deuterated methanol-d4 solution (65 g, 1800 mmol) at -50°C, the reaction solution was added, stirred for 10 min, and slowly heated to 0°C for 3 h of reaction. An aqueous saturated ammonium chloride solution (150 mL) was added to quench the reaction, and the reaction solution was adjusted to pH of approximately 8-9 with 1 M dilute hydrochloric acid. After phase separation, the aqueous phase was extracted with ethyl acetate (100 mL×2). The organic phases were combined, washed with saturated saline (100 mL×2), dried over anhydrous sodium sulfate, subjected to suction filtration, concentrated and dried. After the resulting crude product was triturated with ethanol (150 mL) for 1.5 h, the mixture was subjected suction filtration, and the filter cake was washed with ethanol (30 mL×3), and dried to obtain compound 83 3-(3,4-difluorobenzyl)-1-(4-(pyridin-4-yl)phenyl)-3-deutero-pyrrolidin-2-one as a white solid (8.5 g, D%>99%).

LC_MS: (ES⁺): m/z 365.8 [M+H] ⁺.

¹H NMR (400 MHz, Chloroform-*d*) δ 8.70 - 8.64 (m, 2H), 7.81 - 7.76 (m, 2H), 7.72 - 7.66 (m, 2H), 7.55 - 7.49 (m, 2H), 7.10 (ddd, *J* = 11.1, 7.9, 2.4 Hz, 2H), 7.02 - 6.95 (m, 1H), 3.87 - 3.67 (m, 2H), 3.27 (d*, J =* 14.1 Hz, 1H), 2.84 (d, *J* = 14.1 Hz, 1H), 2.26 (ddd, *J* = 10.9, 7.3, 3.0 Hz, 1H), 1.89 (dt, *J =* 12.8, 8.6 Hz, 1H).

### Example 57: Synthesis route of compound 84

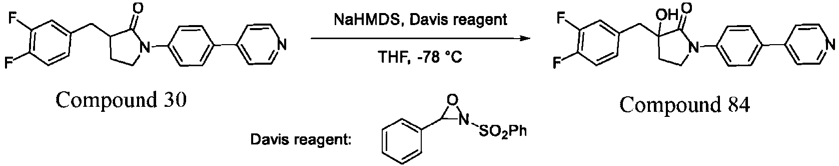

### Step 1: Compound 84 3-(3,4-difluorobenzyl)-3-hydroxy-1-(4-(pyridin-4-yl)phenyl)pyrrolidin-2-one

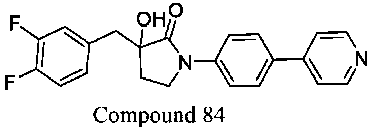

Compound 30 3-(3,4-difluorobenzyl)-1-(4-(pyridin-4-yl)phenyl)pyrrolidin-2-one (20 mg, 0.055 mmol) was dissolved in 10 mL of tetrahydrofuran at -78°C, and sodium bis(trimethylsilyl)amide (1.5 eq., 0.08 mL, 1N) was added dropwise. After 0.5 h, a solution of Davis reagent 3-phenyl-2-phenylsulfonyl-1,2-oxaziridine (21 mg, 0.08 mmol) in tetrahydrofuran (1 mL) was added dropwise. The reaction system was stirred at a low temperature for 2 h. After complete reaction of the raw material and target mass were detected by LCMS, and formation of the major spot was detected by TLC, the saturated ammonium chloride solution (5 mL) was added to quench the reaction. The reaction solution was extracted with dichloromethane (10 mL×3), washed with water (15 mL), saturated ammonium chloride solution (20 mL) and saturated saline (20 mL) in sequence, and then subjected to rotary evaporation to remove the reaction solvent. The resulting crude product was purified by using preparative silica gel plate (DCM:MeOH = 20:1), and dried in vacuum to obtain compound 84 3-(3,4-difluorobenzyl)-3-hydroxy-1-(4-(pyridin-4-yl)phenyl)pyrrolidin-2-one as a white solid (5 mg).

LC_MS: (ES⁺): m/z 381.2 [M+H] ⁺.

### Example 58: Synthesis route of compound 85

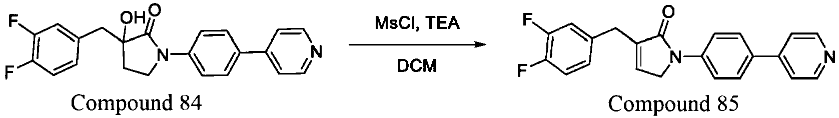

### Step 1: Compound 85 3-(3,4-difluorobenzyl)-1-(4-(pyridin-4-yl)phenyl)-1,5-dihydro-2H-pyrrolidin-2-one

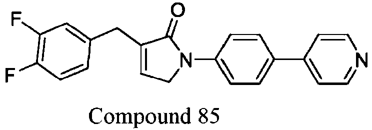

Compound 84 3-(3,4-difluorobenzyl)-3-hydroxy-1-(4-(pyridin-4-yl)phenyl)pyrrolidin-2-one (20 mg, 0.05 mmol) was dissolved in 1 mL of dichloromethane at 0°C, and triethylamine (25 mg, 0.25 mmol, 5 eq) was added dropwise. After 0.5 h, a solution of compound methanesulfonyl chloride (11 mg, 0.1 mmol, 2 eq) in dichloromethane (0.5 mL) was added dropwise. The reaction solution was stirred at low temperature for 1 h. After complete reaction of the raw material and target mass were detected by LCMS, and formation of the major spot was detected by TLC, a saturated ammonium chloride solution (5 mL) was added to quench the reaction. The reaction solution was extracted with dichloromethane (10 mL×3), washed with water (15 mL), saturated ammonium chloride solution (20 mL) and saturated saline (20 mL) in sequence, and then subjected to rotary evaporation to remove the reaction solvent. The resulting crude product was purified by using preparative silica gel plate (developing agent: DCM:MeOH = 20:1), and dried in vacuum to obtain compound 85 3-(3,4-difluorobenzyl)-1-(4-(pyridin-4-yl)phenyl)-1,5-dihydro-2H-pyrrolidin-2-one as a white solid (2 mg).

LC_MS: (ES⁺): m/z 363.0 [M+H] ⁺.

### Example 59: Synthesis route of compounds 86 and 87

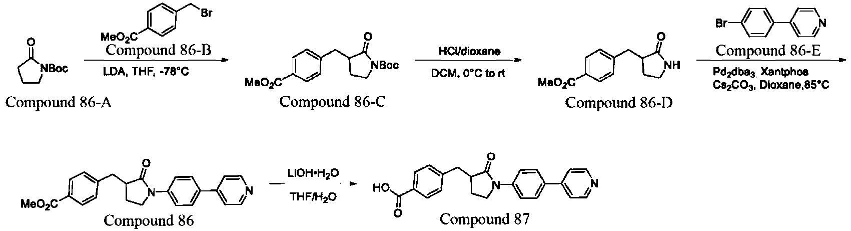

### Step 1: Compound 86-C tert-butyl 3-(4-(methoxycarbonyl) benzyl)-2-oxopyrrolidin-1-carboxylate

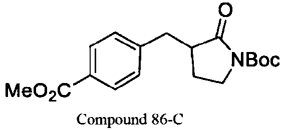

Under nitrogen atmosphere, to a solution of compound 86-A 1-(tert-butoxycarbonyl)-2-pyrrolidone (1.85 g, 10.0 mmol) in tetrahydrofuran (20 mL), 15.0 mL of 1M lithium N,N-diisopropylamide in tetrahydrofuran solution was added dropwise at -78°C. This mixture was stirred at -78°C for 1 h, and then a solution of compound 86-B methyl 4-(bromomethyl)benzoate (2.29 g, 10.0 mmol) in tetrahydrofuran (30 mL) was slowly added dropwise. After 1 h of reaction at -78°C, a saturated ammonium chloride solution (20 mL) was added to quench the reaction. The reaction solution was partitioned into dichloromethane (15 mL×3), and the organic phase was collected and concentrated under reduced pressure. The resulting crude product was purified by silica gel column chromatography (eluent: a solution of 25% ethyl acetate in petroleum ether) to obtain compound 86-C tert-butyl 3-(4-(methoxycarbonyl)benzyl)-2-oxopyrrolidin-1-carboxylate as a white solid (1 g).

LC_MS: (ES⁺): m/z 234.1 [M+H-100] ⁺.

### Step 2: Compound 86-D methyl 4-((2-oxopyrrolidin-3-yl)methyl)benzoate

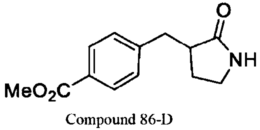

To a solution of compound 86-C tert-butyl 3-(4-(methoxycarbonyl)benzyl)-2-oxopyrrolidin-1-carboxylate (1 g, 3.00 mmol) in dichloromethane (10 mL) in an ice bath, a solution of 4M HCl in 1,4-dioxane (2 mL) was slowly added. The reaction system was stirred in an ice bath for 1.5 h. After the reaction was completed, as monitored by TLC, the reaction solution was concentrated under reduced pressure, added with saturated sodium bicarbonate solution to achieve a neutral pH, and partitioned into dichloromethane (5 mL×3). The organic phases were combined and concentrated under reduced pressure to obtain the crude compound 86-D methyl 4-((2-oxopyrrolidin-3-yl)methyl)benzoate as a white solid (650 mg).

LC_MS: (ES+): m/z 234.1 [M+H] ⁺.

### Step 3: Compound 86 methyl 4-((2-oxo-1-(4-(pyridin-4-yl)phenyl)pyrrolidin-3-yl)methyl)benzoate

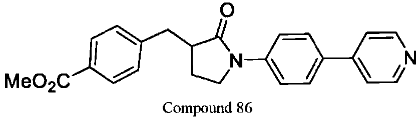

A solution of compound 86-D methyl 4-((2-oxopyrrolidin-3-yl)methyl)benzoate (300 mg, 1.28 mmol), compound 86-E 4-(4-bromophenyl)pyridine (300 mg, 1.28 mmol), tris (dibenzylideneacetone)dipalladium (108 mg, 0.0128 mmol), 4,5-bis(diphenylphosphino)-9,9-dimethylxanthene (74 mg, 0.0128 mmol) and cesium carbonate (834 mg, 2.56 mmol) in 1,4-dioxane (5 mL) was reacted under nitrogen atmosphere at 85°C for 12 h. After the reaction was completed, as monitored by TLC, the mixture was cooled to room temperature and filtered, and the filter residue was washed with dichloromethane (10 mL×2). The filtrates were combined and concentrated under reduced pressure. The resulting crude product was purified by silica gel column chromatography (eluent: a solution of 5% methanol in dichloromethane) to obtain compound 86 methyl 4-((2-oxo-1-(4-(pyridin-4-yl)phenyl)pyrrolidin-3-yl)methyl)benzoate as a white solid (310 mg).

LC_MS: (ES⁺): m/z 387.2 [M+H] ⁺.

¹H NMR (400 MHz, CDCl₃) δ 8.66 (d, *J* = 6.0 Hz, 2H), 7.99 (d, *J* = 8.2 Hz, 2H), 7.77 (d, *J* = 8.8 Hz, 2H), 7.67 (d, *J* = 8.8 Hz, 2H), 7.52 (dd, *J* = 4.6, 1.5 Hz, 2H), 7.33 (d, *J* = 8.2 Hz, 2H), 3.91 (s, 3H), 3.78 (dd, *J* = 16.6, 8.9 Hz, 1H), 3.68 (td, *J =* 9.1, 3.1 Hz, 1H), 3.37 (dd, *J=* 13.5, 4.0 Hz, 1H), 3.03 - 2.94 (m, 1H), 2.90 (dd, *J* = 13.5, 9.2 Hz, 1H), 2.28 - 2.17 (m, 1H), 1.88 (ddd, *J* = 17.9, 12.7, 8.7 Hz, 1H).

### Step 4: Compound 87 4-((2-oxo-1-(4-(pyridin-4-yl)phenyl)pyrrolidin-3-yl)methyl)benzoic acid

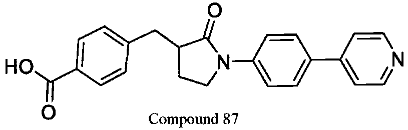

To a solution of compound 86 methyl 4-((2-oxo-1-(4-(pyridin-4-yl)phenyl)pyrrolidin-3-yl)methyl)benzoate (50 mg, 0.129 mmol) in tetrahydrofuran/methanol/water (0.5 mL/0.5 mL/0.5 mL), sodium hydroxide (10.35 mg, 0.259 mmol) was added at room temperature. The reaction solution was heated to 50°C and stirred for 6 h. After the reaction was completed, as monitored by LCMS, the reaction solution was adjusted to pH approximately 3 with dilute hydrochloric acid. After a large amount of solid was precipitated, the solid was collected through filtration to obtain compound 87 4-((2-oxo-1-(4-(pyridin-4-yl)phenyl)pyrrolidin-3-yl)methyl)benzoic acid as a white solid (45 mg).

LC_MS: (ES⁺): m/z 373.1 [M+H] ⁺.

### Example 60: Synthesis route of compound 88

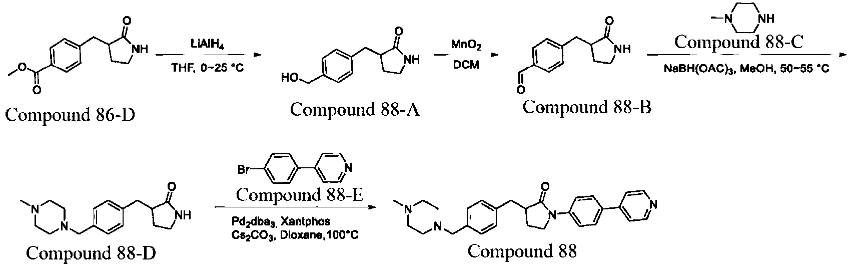

### Step 1: Compound 88-A 3-(4-(hydroxymethyl)benzyl)pyrrolidin-2-one

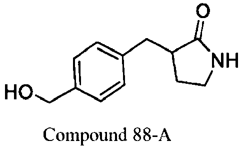

To a solution of compound 86-D methyl 4-((2-oxopyrrolidin-3-yl)methyl)benzoate (200 mg, 0.858 mmol) in tetrahydrofuran (8 mL), lithium aluminum hydride (20 mg, 1.03 mmol) was slowly added at 0°C. The reaction solution was heated to room temperature and stirred for 3 h. After the reaction was completed, as monitored by TLC, water (20 µL) and 10% sodium hydroxide solution (20 µL) were added to quench the reaction. The reaction system was filtered and concentrated under reduced pressure. The resulting crude product was separated and purified by preparative thin layer chromatography (eluent: a solution of 10% methanol in dichloromethane) to obtain compound 88-A 3-(4-(hydroxymethyl)benzyl)pyrrolidin-2-one as a beige solid (70 mg).

LC_MS: (ES⁺): m/z 206.1 [M+H] ⁺.

### Step 2: Compound 88-B 4-(2-oxopyrrolidin-3-yl)methyl)benzaldehyde

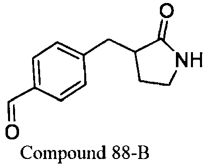

To a solution of compound 88-A 3-(4-(hydroxymethyl)benzyl)pyrrolidin-2-one (70 mg, 0.341 mmol) in dichloromethane (5 mL), manganese dioxide (297 mg, 34.1 mmol) was slowly added. The reaction solution was stirred at room temperature for 6 h. After the reaction was completed, as monitored by TLC, the reaction solution was filtered, the filter cake was washed with dichloromethane, and the filtrate was concentrated. The resulting crude product was purified by column chromatography (eluent: a solution of 0%-5% methanol in dichloromethane) to obtain compound 88-B 4-(2-oxopyrrolidin-3-yl)methyl)benzaldehyde as a beige solid (50 mg).

LC_MS: (ES⁺): m/z 204.1 [M+H] ⁺.

### Step 3: Compound 88-D 3-(4-((4-methylpiperazin-1-yl)methyl)benzyl)pyrrolidin-2-one

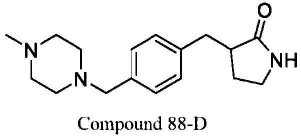

A solution of compound 88-B 4-(2-oxopyrrolidin-3-yl)methyl)benzaldehyde (50 mg, 0.246 mmol) and compound 88-C 1-methylpiperazine (27.1 mg, 0.27 mmol) in methanol (2.5 mL) was heated to 50°C and stirred for 1 h. Sodium triacetoxyborohydride (104.27 mg, 0.492 mmol) was added, and the reaction solution was stirred at 55°C for 24 h. After the reaction was completed, as monitored by TLC, the reaction solution was added with water (5 mL) to quench the reaction, and extracted with dichloromethane (5 mL×2). The organic phases were combined, dried over anhydrous sodium sulfate and concentrated under reduced pressure. The resulting crude product was purified by preparative thin layer chromatography (eluent: a solution of 10% methanol in dichloromethane) to obtain compound 88-D 3-(4-((4-methylpiperazin-1-yl)methyl)benzyl)pyrrolidin-2-one as a light yellow gum (33 mg).

LC_MS: (ES⁺): m/z 288.2 [M+H] ⁺.

### Step 4: Compound 88 3-(4-((4-methylpiperazin-1-yl)methyl)benzyl)-1-(4-(pyridin-4-yl)phenyl)pyrrolidin-2-one

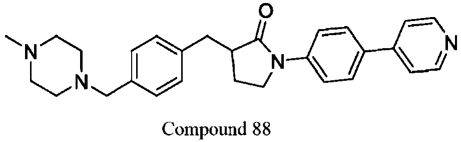

To a solution of compound 88-D 3-(4-((4-methylpiperazin-1-yl)methyl)benzyl)pyrrolidin-2-one (33 mg, 0.115 mmol), compound 88-E 4-(4-bromophenyl)pyridine (26.88 mg, 0.115 mmol), Xant-Phos (6.65 mg, 0.0115 mmol) and cesium carbonate (112.13 mg, 0.345 mmol) in 1,4-dioxane (2 mL), Pd₂(dba)₃ (5.26 mg, 0.0058 mmol) was added under nitrogen protection. The reaction system was subjected to nitrogen replacement 3 times and heated to 100°C, and then the reaction was carried out for 2 h. After the reaction was completed, as monitored by TLC, the reaction solution was cooled to room temperature and filtered. After the filtrate was concentrated, the resulting crude product was separated and purified by preparative thin layer chromatography (eluent: a solution of 10% methanol in dichloromethane) to obtain compound 88 3-(4-((4-methylpiperazin-1-yl)methyl)benzyl)-1-(4-(pyridin-4-yl)phenyl)pyrrolidin-2-one as a white solid (11.1 mg).

LC_MS: (ES⁺): m/z 441.3 [M+H] ⁺.

¹H NMR (400 MHz, CDCl₃) δ 8.67 (dd, *J* = 4.6, 1.6 Hz, 2H), 7.81 - 7.76 (m, 2H), 7.71 - 7.66 (m, 2H), 7.52 (dd, *J* = 4.6, 1.6 Hz, 2H), 7.24 (t, *J =* 9.0 Hz, 4H), 3.79 (dt, *J =* 15.8, 7.9 Hz, 1H), 3.73 - 3.66 (m, 1H), 3.57 (s, 2H), 3.34 - 3.29 (m, 1H), 2.98 (ddd, *J* = 13.8, 9.0, 4.5 Hz, 1H), 2.89 - 2.81 (m, 1H), 2.68 (s, 8H), 2.48 (s, 3H), 2.28 - 2.22 (m, 1H), 1.95 - 1.88 (m, 1H).

### Example 61: Synthesis route of compound 89

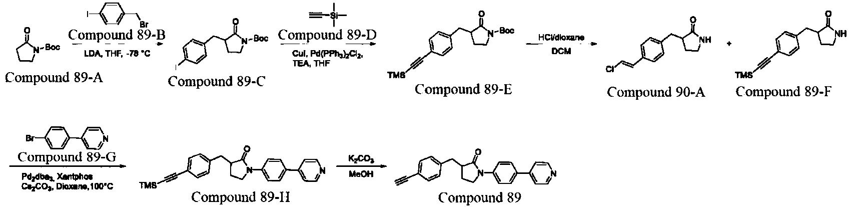

### Step 1: Compound 89-C tert-butyl 3-(4-iodobenzyl)-2-oxopyrrolidin-1-carboxylate

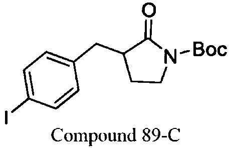

This step was carried out using compound 89-A tert-butyl 2-oxopyrrolidine-1-carboxylate and compound 89-B 1-(bromomethyl)-4-iodobenzene as raw materials, referring to the synthesis procedure described in step 4 in Example 3.

LC_MS: (ES⁺): m/z 402.0 [M+H] ⁺.

### Step 2: compound 89-E tert-butyl 2-oxo-3-(4-((trimethylsilyl)ethynyl)benzyl)pyrrolidin-1-carboxylate

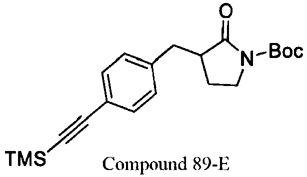

Under nitrogen protection, to a solution of compound 89-C tert-butyl 3-(4-iodobenzyl)-2-oxopyrrolidin-1-carboxylate (200 mg, 0.5 mmol), bis(triphenylphosphine)palladium dichloride (10.5 mg, 0.015 mmol) and copper(I) iodide (3.6 mg, 0.015 mmol) in tetrahydrofuran (4 mL), compound 89-D trimethylsilylacetylene (58.9 mg, 0.6 mmol) and triethylamine (151.8 mg, 1.5 mmol) were added at room temperature. The reaction solution was stirred at room temperature for 4 h. After the reaction was completed, as monitored by TLC, water (10 mL) was added to quench the reaction and the reaction solution was extracted with ethyl acetate (8 mL×2). The organic layers were combined, dried over anhydrous sodium sulfate and concentrated under reduced pressure. The resulting crude product was and purified by silica gel column chromatography to obtain compound 89-E tert-butyl 2-oxo-3-(4-((trimethylsilyl)ethynyl)benzyl)pyrrolidin-1-carboxylate as a yellow solid (176 mg).

LC_MS: (ES⁺): m/z 372.2 [M+H] ⁺.

¹H NMR (400 MHz, CDCl₃) δ 7.41 (d, *J* = 7.9 Hz, 2H), 7.15 (d, *J* = 8.0 Hz, 2H), 3.66 (dd, *J* = 8.7, 2.4 Hz, 1H), 3.54 (dd, *J =* 9.4, 7.5 Hz, 1H), 3.26 (dd, *J* = 13.6, 3.8 Hz, 1H), 2.83 - 2.75 (m, 1H), 2.69 (dd, *J* = 13.6, 9.5 Hz, 1H), 2.00 (m, 1H), 1.73 - 1.65 (m, 1H), 1.58 - 1.54 (m, 9H), 0.26 (d, *J* = 0.6 Hz, 9H).

### Step 3: Compound 90-A (E)-3-(4-(2-chloroethenyl)benzyl)pyrrolidin-2-one and compound 89-F 3-(4-((trimethylsilyl)ethynyl)benzyl)pyrrolidin-2-one

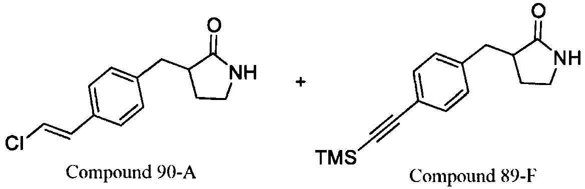

To a solution of compound 89-E tert-butyl 2-oxo-3-(4-((trimethylsilyl)ethynyl)benzyl)pyrrolidin-1-carboxylate (176 mg, 0.474 mmol) in dichloromethane (2 mL), a solution of hydrochloric acid/dioxane (1 mL) was added. The reaction solution was stirred at room temperature for 5 h. After the reaction was completed, as monitored by TLC, the reaction solution was concentrated, and the resulting crude product was purified by preparative thin layer chromatography (eluent: a solution of 8% methanol in dichloromethane) to obtain compound 90-A (E)-3-(4-(2-chloroethenyl)benzyl)pyrrolidin-2-one (40 mg) and compound 89-F 3-(4-((trimethylsilyl)ethynyl)benzyl)pyrrolidin-2-one (60 mg).

**Compound 90-A**_LC_MS: (ES⁺): m/z 236.1 [M+H] ⁺.

**Compound 89-F**_LC_MS: (ES⁺): m/z 272.1 [M+H] ⁺.

### Step 4: Compound 89-H 1-(4-(pyridin-4-yl)phenyl)-3-(4-((trimethylsilyl)ethynyl)benzyl)pyrrolidin-2-one

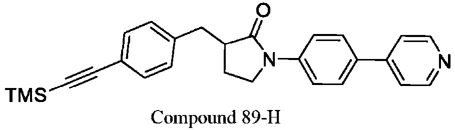

This step was carried out using compound 89-F 3-(4-((trimethylsilyl)ethynyl)benzyl)pyrrolidin-2-one and compound 89-G 4-(4-bromophenyl)pyridine as raw materials, referring to the synthesis procedure described in step 1 in Example 38.

LC_MS: (ES+): m/z 425.2 [M+H] +.

### Step 5: Compound 89 3-(4-ethynylbenzyl)-1-(4-(pyridin-4-yl)phenyl)pyrrolidin-2-one

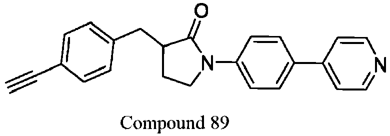

To a solution of compound 89-H 1-(4-(pyridin-4-yl)phenyl)-3-(4-((trimethylsilyl)ethynyl)benzyl)pyrrolidin-2-one (15 mg, 0.353 mmol) in methanol (2 mL), potassium carbonate (9.76 mg, 0.0706 mmol) was added. The reaction solution was stirred at room temperature for 4 h. After the reaction was completed, as monitored by TLC, the reaction solution was concentrated and extracted with dichloromethane (8 mL×2) and water (10 mL). The organic phases were combined, dried over anhydrous sodium sulfate and concentrated under reduced pressure. The resulting crude product was purified by preparative thin layer chromatography (eluent: a solution of 10% methanol in dichloromethane) to obtain compound 89 3-(4-ethynylbenzyl)-1-(4-(pyridin-4- yl)phenyl)pyrrolidin-2-one as a white solid (3.0 mg).

LC_MS: (ES⁺): m/z 353.1 [M+H] ⁺.

¹H NMR (400 MHz, CDCl₃) δ 8.67 (d, *J =* 5.4 Hz, 2H), 7.78 (d, *J* = 8.8 Hz, 2H), 7.69 (d, *J* = 8.7 Hz, 2H), 7.53 (d, *J* = 6.0 Hz, 2H), 7.46 (d, *J* = 8.1 Hz, 2H), 7.24 (d*, J =* 8.1 Hz, 2H), 3.78 (dd, J= 16.8, 8.5 Hz, 1H), 3.69 (td, *J* = 9.1, 3.0 Hz, 1H), 3.33 (dd, *J* = 13.6, 4.1 Hz, 1H), 3.09 (s, 1H), 2.98 (ddd, *J* = 17.7, 8.9, 4.1 Hz, 1H), 2.87 (dd, *J* = 13.6, 9.1 Hz, 1H), 2.29 - 2.19 (m, 1H), 1.95 - 1.86 (m, 1H).

### Example 62: Synthesis route of compound 90

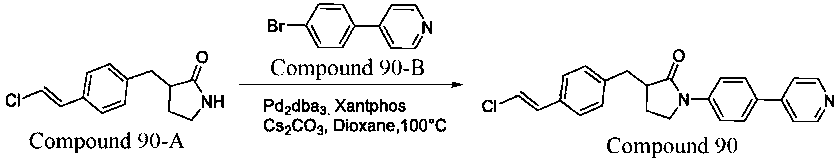

### Step 1: Compound 90 (E)-3-(4-(2-chloroethenyl)benzyl)-1-(4-(pyridin-4-yl)phenyl)pyrrolidin-2-one

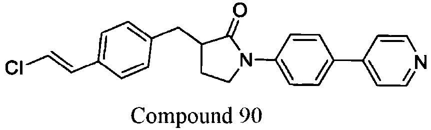

This step was carried out using compound 90-A (E)-3-(4-(2-chloroethenyl)benzyl)pyrrolidin-2-one and compound 90-B 4-(4-bromophenyl)pyridine as raw materials, referring to the synthesis procedure described in step 1 in Example 38.

LC_MS: (ES+): m/z 389.2 [M+H] +.

### Example 63: Synthesis route of compound 91

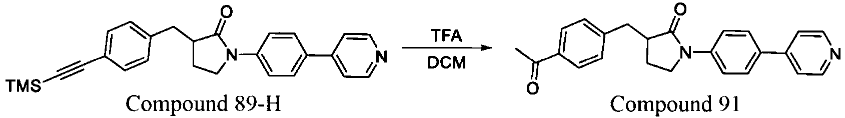

### Step 1: Compound 91 3-(4-acetylbenzyl)-1-(4-(pyridin-4-yl)phenyl)pyrrolidin-2-one

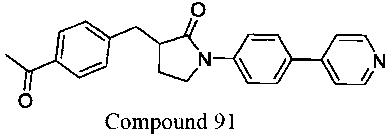

To a solution of compound 89-H 1-(4-(pyridin-4-yl)phenyl)-3-(4-((trimethylsilyl)ethynyl)benzyl)pyrrolidin-2-one (40 mg, 0.0942 mmol) in dichloromethane (1 mL), trifluoroacetic acid (0.5 mL) was added. The reaction solution was stirred at room temperature for 4 h. After the reaction was completed, as monitored by TLC, the reaction solution was concentrated and extracted with saturated aqueous sodium bicarbonate solution (8 mL) and dichloromethane (5 mL×2). The organic phases were combined, dried over anhydrous sodium sulfate and concentrated under reduced pressure. The resulting crude product was purified by preparative thin layer chromatography (eluent: a solution of 10% methanol in dichloromethane) to obtain compound 91 3-(4-acetylbenzyl)-1-(4-(pyridin-4-yl)phenyl)pyrrolidin-2-one as a light yellow solid (10.3 mg).

LC_MS: (ES⁺): m/z 371.6 [M+H] ⁺.

¹H NMR (400 MHz, CDCl₃) δ 8.67 (d, *J* = 5.8 Hz, 2H), 7.94 (d, *J* = 8.2 Hz, 2H), 7.79 (d, *J* = 8.7 Hz, 2H), 7.69 (d, *J* = 8.7 Hz, 2H), 7.53 (d, *J* = 6.1 Hz, 2H), 7.38 (d, *J* = 8.1 Hz, 2H), 3.80 (dd, *J =* 16.9, 8.5 Hz, 1H), 3.72 (td, *J* = 9.1, 3.0 Hz, 1H), 3.39 (dd, *J =* 13.5, 4.0 Hz, 1H), 3.02 (ddd, *J* = 17.6, 8.9, 4.0 Hz, 1H), 2.92 (dd, *J* = 13.5, 9.1 Hz, 1H), 2.62 (s, 3H), 2.30 - 2.20 (m, 1H), 1.97 - 1.86 (m, 1H).

### Synthesis route of compounds 44-52

For the following compounds, the synthesis route is shown below. They were synthesized by using corresponding reagents, referring to the synthesis method described in step 4 in Example 3.

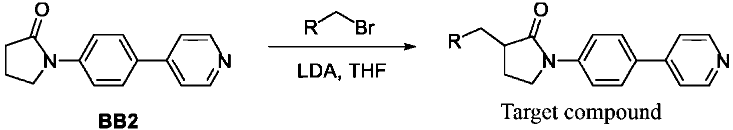

| Number | Structure | Mass spectrometry and NMR data |
|---|---|---|
| Compound 44 | 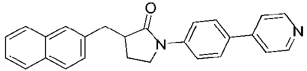 | LC_MS: (ES+): m/z 379.20 [M+H] |
| Compound 45 | 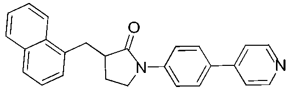 | LC_MS: (ES+): m/z 379.19 [M+H] |
| Compound 46 | 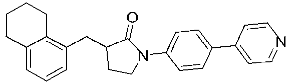 | LC_MS: (ES+): m/z 383.22 [M+H] |
| Compound 47 | 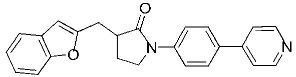 | LC_MS: (ES+): m/z 369.16 [M+H] |
| Compound 48 | 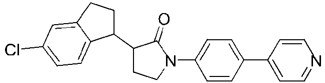 | LC_MS: (ES+): m/z 389.14 [M+H] |
| Compound 49 | 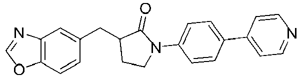 | LC_MS: (ES+): m/z 370.13 [M+H] |
| Compound 50 | 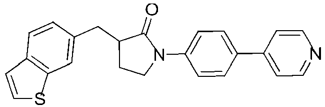 | LC_MS: (ES+): m/z 385.13 [M+H] |
| Compound 51 | 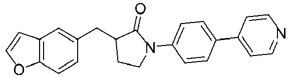 | LC_MS: (ES+): m/z 369.15 [M+H] |
| Compound 52 | 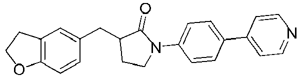 | LC_MS: (ES+): m/z 371.20 [M+H] |
| Compound 56 | 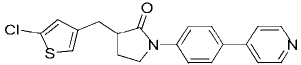 | LC_MS: (ES+): m/z 369.0 [M+H] |
| Compound 57 | 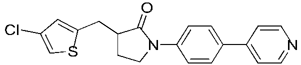 | LC_MS: (ES+): m/z 369.2 [M+H] |
| | | ¹H NMR (400 MHz, Chloroform-*d*) *δ* 8.70 (*d, J* = 5.1 Hz, 2H), 7.86 (d, *J* = 8.5 Hz, 2H), 7.75 (*d, J* = 8.8 Hz, 4H), 6.98 (*d*, *J* = 1.5 Hz, 1H), 6.80 (*s,* 1H), 3.90-3.81 (*m,* 2H), 3.44-3.39 (*m,* 1H), 3.15-3.08 (*m,* 1H), 304-2.97 (*m,* 1H), 2.43-2.37 (*m,* 1H), 1.99-1.95 (*m,* 1H). |
| Compound 72 | 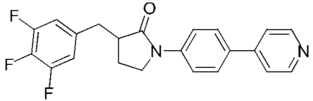 | LC_MS: (ES+): m/z 383.1 [M+H] |
| Compound 73 | 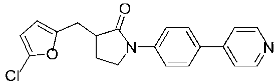 | LC_MS: (ES+): m/z 353.4 [M+H] |
| Compound 79 | 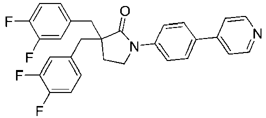 | LC_MS: (ES+): m/z 491.15 [M+H] |
| | | ¹H NMR (400 MHz, CDCl₃) δ 8.62 (dd, *J* = 4.6, 1.5 Hz, 2H), 7.70 - 7.44 (m, 6H), 7.13 - 6.98 (m, 4H), 6.97 - 6.86 (m, 2H), 3.22 (d, *J* = 13.4 Hz, 2H), 2.93 (t, *J* = 7.1 Hz, 2H), 2.68 (d, *J* = 13.5 Hz, 2H), 2.07 (t, *J* = 7.2 Hz, 2H). |

For the following compounds, the synthesis route is shown below. They were synthesized by using corresponding reagents, referring to the synthesis method described in step 2 in Example 46.

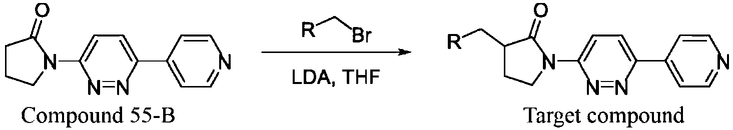

| Number | Structure | Mass spectrometry and NMR data |
|---|---|---|
| Compound 60 | 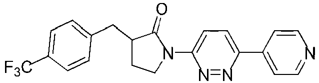 | LC_MS: (ES+): m/z 399.2 [M+H] |
| | | ¹H NMR (400 MHz, DMSO-d6) δ 8.80 - 8.70 (m, 3H), 8.43 (d, J = 9.5 Hz, 1H), 8.14 - 8.08 (m, 2H), 7.69 (d, J = 8.0 Hz, 2H), 7.54 (d, J = 8.0 Hz, 2H), 4.21 (ddd, J = 11.1, 8.7, 2.5 Hz, 1H), 3.97 (ddd, J = 10.8, 9.1, 7.3 Hz, 1H), 3.31 - 3.14 (m, 2H), 2.89 (dd, J = 13.5, 9.2 Hz, 1H), 2.17 (dtd, J = 12.2, 7.5, 2.6 Hz, 1H), 1.87 (dq, J = 12.2, 9.1 Hz, 1H). |
| Compound 61 | 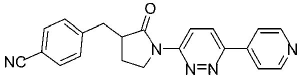 | LC_MS: (ES+): m/z 356.5 [M+H] |
| | | ¹H NMR (400 MHz, DMSO-d6) δ 8.82 - 8.66 (m, 3H), 8.42 (d, J = 9.5 Hz, 1H), 8.15 - 8.06 (m, 2H), 7.80 (d, J = 7.8 Hz, 2H), 7.52 (d, J = 7.9 Hz, 2H), 4.19 (ddd, J = 11.1, 8.7, 2.5 Hz, 1H), 3.96 (ddd, J = 10.8, 9.1, 7.3 Hz, 1H), 3.32 - 3.12 (m, 2H), 2.88 (dd, J = 13.5, 9.1 Hz, 1H), 2.15 (ddt, J = 10.3, 7.5, 4.9 Hz, 1H), 1.85 (dq, J = 12.1, 9.1 Hz, 1H). |
| Compound 62 | 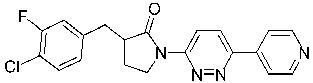 | LC_MS: (ES+): m/z 383.4 [M+H] |
| | | ¹H NMR (400 MHz, DMSO-d6) δ 8.80 - 8.68 (m, 3H), 8.43 (d, J = 9.5 Hz, 1H), 8.14 - 8.08 (m, 2H), 7.53 (t, J = 8.1 Hz, 1H), 7.40 (dd, J = 10.6, 2.0 Hz, 1H), 7.19 (dd, J = 8.3, 2.0 Hz, 1H), 4.20 (ddd, J = 11.1, 8.7, 2.6 Hz, 1H), 3.96 (ddd, J = 10.8, 9.0, 7.3 Hz, 1H), 3.24 - 3.10 (m, 2H), 2.86 - 2.75 (m, 1H), 2.17 (dtd, J = 12.1, 7.8, 2.6 Hz, 1H), 1. 86 (dq, J = 12.2, 9.1 Hz, 1H). |
| Compound 63 | 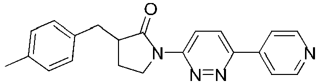 | LC_MS: (ES+): m/z 345.2 [M+H] |
| | | ¹H NMR (400 MHz, DMSO-d6) δ 8.80 - 8.70 (m, 3H), 8.42 (d, J = 9.5 Hz, 1H), 8.15 - 8.06 (m, 2H), 7.21 - 7.08 (m, 4H), 4.13 (ddd, J = 11.3, 8.7, 2.9 Hz, 1H), 3.95 (ddd, J = 10.8, 8.8, 7.4 Hz, 1H), 3.20 - 3.01 (m, 2H), 2.80 - 2.66 (m, 1H), 2.14 (dtd, J = 12.2, 7.6, 2.8 Hz, 1H), 1.84 (dq, J = 12.5, 8.9 Hz, 1H). |
| Compound 64 | 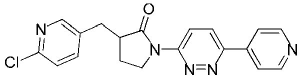 | LC_MS: (ES+): m/z 366.1 [M+H] |
| | | ¹H NMR (400 MHz, DMSO-d6) δ 8.81 - 8.74 (m, 2H), 8.71 (d, J = 9.4 Hz, 1H), 8.42 (d, J = 9.5 Hz, 1H), 8.37 (d, J = 2.5 Hz, 1H), 8.14 - 8.08 (m, 2H), 7.82 (dd, J = 8.2, 2.5 Hz, 1H), 7.48 (d, J = 8.2 Hz, 1H), 4.21 (ddd, J = 11.1, 8.8, 2.6 Hz, 1H), 3.97 (ddd, J = 10.9, 9.1, 7.3 Hz, 1H), 3.23 - 3.11 (m, 2H), 2.88 - 2.79 (m, 1H), 2.28 - 2.14 (m, 1H), 1.86 (dq, J = 12.3, 9.0 Hz, 1H). |
| Compound 65 | 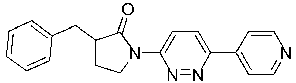 | LC_MS: (ES+): m/z 331.1 [M+H] |
| Compound 66 | 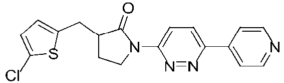 | LC_MS: (ES+): m/z 371.5 [M+H] |
| Compound 67 | 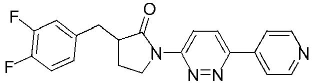 | LC_MS: (ES+): m/z 367.1 [M+H] |
| Compound 68 | 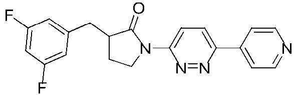 | LC_MS: (ES+): m/z 367.1 [M+H] |
| Compound 69 | 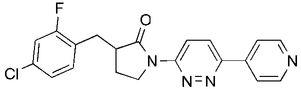 | LC_MS: (ES+): m/z 383.0 [M+H] |
| Compound 70 | 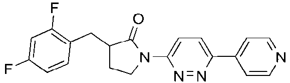 | LC_MS: (ES+): m/z 367.1 [M+H] |
| Compound 71 | 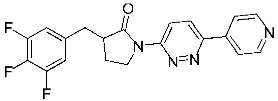 | LC_MS: (ES+): m/z 385.1 [M+H] |

The following compounds were synthesized by using corresponding reagents, referring to the synthesis method described in step 1 in Example 56.

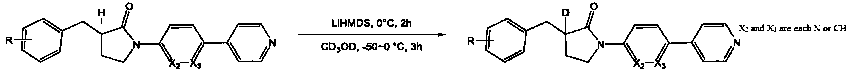

| Number | Structure |
|---|---|
| Compound 92 | 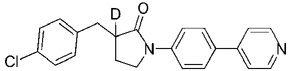 |
| Compound 93 | 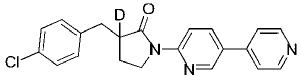 |
| Compound 94 | 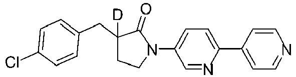 |
| Compound 95 | 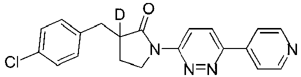 |
| Compound 96 | 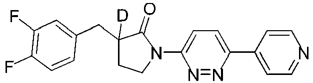 |
| Compound 97 | 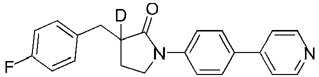 |
| Compound 98 | 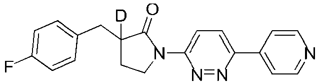 |
| Compound 99 | 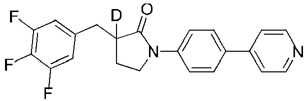 |
| Compound 100 | 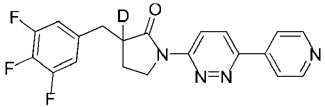 |

### Biological Activity Test Examples:

### Biological Activity Example 1: Preparation of SARM1

### Preparation of Compounds to be Tested:

Compounds to be tested were stocked at 200 µM or 10 mM (in DMSO), and further diluted to the desired compound concentration for the *in vitro* SARM1 enzyme assay and inhibitor screening.

### Expression and Purification of SARM1 Protein

### (1) Plasmid Construction

The gene sequence encoding SARM1 protein was amplified by PCR and the PCR amplification product was constructed into the pcDNA3.1-HIS-C plasmid. This construction was completed by YouBio Biological Technology (Hunan, China).

### (2) Plasmid Transfection

HEK293 cells were cultured in DMEM in a 10 cm dish. The next day, cells were transfected with 15 µg of the pcDNA3.1-SARM1-HIS-C expression plasmid.

### (3) Cell Harvesting and Protein Extraction

Cells were harvested 48 hours after the transfection. The SARM1 protein expressed in cells was obtained through cell lysis by digitonin, which was used for *in vitro* activity assay. The specific process was as follows:

Cells were digested and detached from the dish using trypsin-EDTA, centrifuged at 1000 rpm for 5 minutes, washed once with PBS, then resuspended in PBS containing 100 µM digitonin at 0.6 mL PBS for the cells in 10 cm dish and lysed for 5 min. The cells were added with trypan blue and observed under a microscope. More than 90% of the cells were confirmed to be lysed. The cells were centrifuged at 5000 rpm for 10 min, and the supernatant containing SARM1 protein was collected.

Biological Activity Example 2: *In Vitro* Biochemical Assay for Inhibiting SARM1 Enzymatic Activity (IC₅₀)

The compound was added into a solution of 50 mM Tris-HCl (pH 7.5) containing 0.05 µg/mL SARM1 to achieve a concentration of 200 µM, and then serially diluted 6 times, where each dilution was carried out by taking half of the previous solution and adding an equal volume of 50 mM Tris-HCl (pH 7.5) containing 0.05 µg/mL SARM1, yielding a set of final concentrations of the compound of either 200, 100, 50, 25, 12.5, 6.25 and 3.125 µM, or 200, 50, 12.5, 3.125, 0.78, 0.195 and 0.049 µM. No inhibitor was added in the control group. All mixtures were incubated at room temperature for 10 min.

Subsequently, 50 µM NAD, 50 µM PC6 as substrate and 50 µM NMN as activator were added to the SARM1 protein incubated with the inhibitor and reacted for 30 min at room temperature. Wherein, the aforementioned concentrations of each component represent their respective final concentrations in the reaction system

During the reaction, the fluorescence spectrum dynamics of PC6 was detected by a microplate reader, with excitation and emission wavelengths set at 390 nm and 520 nm, respectively. The activity of the protein was expressed as the reaction rate and half maximal inhibitory concentration was calculated. The higher the reaction rate, the stronger the protein activity, and the lower the inhibitory efficiency of the compound.

Dose-response curves for inhibition of SARM1 enzymatic activity for NAD by the compounds were plotted using the above method.

IC₅₀ ranges of these compounds in the assay are shown in Table 1 below:
IC₅₀ ranges for inhibiting SARM1 enzymatic activity: A<1.0 µM; B: 1-10 µM; C: 10-30 µM

**Table 1**

| Number of compound | IC₅₀ |
|---|---|
| 1 | A |
| 2 | C |
| 3 | A |
| 4 | C |
| 5 | A |
| 6 | B |
| 7 | B |
| 8 | A |
| 9 | B |
| 10 | B |
| 11 | C |
| 12 | A |
| 13 | A |
| 14 | A |
| 15 | B |
| 16 | B |
| 17 | B |
| 18 | B |
| 19 | B |
| 20 | A |
| 21 | C |
| 22 | B |
| 23 | C |
| 24 | B |
| 25 | B |
| 26 | C |
| 27 | C |
| 28 | C |
| 29 | A |
| 30 | A |
| 31 | C |
| 32 | C |
| 33 | B |
| 34 | A |
| 35 | A |
| 36 | C |
| 37 | B |
| 38 | B |
| 39 | B |
| 40 | B |
| 41 | B |
| 42 | B |
| 43 | B |
| 53 | A |
| 55 | A |
| 58 | A |
| 59 | A |
| 60 | A |
| 61 | A |
| 62 | A |
| 63 | A |
| 64 | A |
| 65 | A |
| 66 | A |
| 67 | A |
| 68 | A |
| 69 | A |
| 70 | A |
| 71 | A |
| 72 | A |
| 73 | B |
| 74 | A |
| 75 | A |
| 76 | A |
| 78 | C |
| 80 | B |
| 81 | A |
| 82 | A |
| 83 | A |
| 84 | B |
| 85 | B |
| 86 | B |
| 87 | C |
| 88 | C |
| 89 | C |
| 90 | B |
| 91 | C |

### Biological Activity Example 3: Degeneration Index of Axon

### Mouse DRG Explant Culture

Dorsal root ganglia were dissected from two-month-old male C57BL/6 mice and placed into a 35-mm culture dish containing ice-cold HBSS buffer. Extra tissue surrounding each dorsal ganglion was removed using a scalpel, and the ganglia were cut into 2-3 explant pieces. These explants were seeded into wells of a 96-well plate pre-coated with 100 ng/mL Matrigel (Corning), added with 100 µL of DRG medium (Neurobasal medium (Gibco) containing 2% B-27 (Gibco), 1% GlutaMAX (Gibco) and 10 ng/mL NGF (MCE)), and cultured in a 37°C incubator for one week. The medium was half- replaced on the fourth day and the experiments were counted on the eighth day.

### Wallerian Degeneration Assay of Axon

Refer to methods reported in literatures in the related art (Gerdts J, Sasaki Y, Vohra B, Marasa J, Milbrandt J. Image-based screening identifies novel roles for IkappaB kinase and glycogen synthase kinase 3 in axonal degeneration. J Biol Chem. 2011 Aug 12;286(32):28011-8) for the definition and measurement principle of the degeneration index of axon. In the present disclosure, the measurement method is detaild below.

0.1 µL of 10 mM compound to be tested was added to 100 µL of DRG medium to prepare a new medium containing 10 µM compound to be tested. The medium in the 96-well plate was removed and replaced with the new medium containing the compound to be tested. Explant pieces of dorsal ganglia were incubated with compounds in a 37°C incubator for 2 h of pre-protection. Following this 2-hour pretreatment, axons were transcted near the explants tissue piece using a glass microelectrode. At 0 h and 24 h post axonal transection, brightfield images of axons in the same region were captured using a microscope (Olympus) with 20× objective. Axonal integrity was assessed using an in-house developed script. The results were categorized based on the degree of protection into: (A) >50% and (B) 20-50%. Results are shown in Table 2 below.

The results demonstrate that the following compounds at a concentration of 10 µM exhibited significant neuroprotective effects in the Wallerian Degeneration assay of axon explant.

**Table 2**

| Number of compound | Degree of protection against axonal Wallerian Degeneration |
|---|---|
| 1 | A |
| 3 | B |
| 6 | A |
| 7 | A |
| 8 | A |
| 15 | B |
| 17 | A |
| 20 | B |
| 21 | B |
| 24 | B |
| 25 | A |
| 29 | A |
| 30 | A |
| 33 | A |
| 34 | A |
| 41 | B |
| 53 | B |
| 55 | B |
| 58 | B |
| 59 | B |
| 60 | B |
| 62 | B |
| 64 | A |
| 65 | B |
| 66 | A |
| 67 | A |
| 69 | B |
| 70 | B |
| 71 | A |
| 72 | A |
| 75 | A |
| 76 | B |
| 80 | B |
| 81 | A |
| 82 | A |
| 83 | A |
| 84 | B |

## Claims

1. A compound represented by formula (I), or a racemate, enantiomer, diastereoisomer, deuterated compound or pharmaceutically acceptable salt thereof: 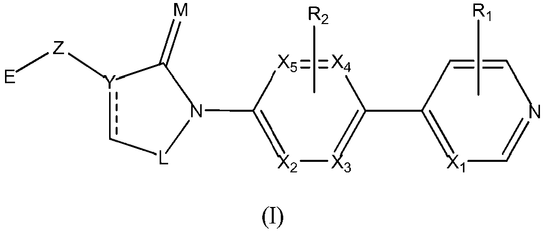 wherein,
X₁, X₂, X₃, X₄ and X₅ are each independently selected from the group consisting of CH and N;
Z represents -CH₂-, -CH₂(CH₃)- or-O-; 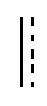 represents a single bond or a double bond;
Y represents a carbon atom or a nitrogen atom;
R₁ is independently selected from the group consisting of H, halogen, C₁-C₃ alkyl, cyano, trifluoromethyl, amino, hydroxy, methoxy and -C(O)NH₂, preferably selected from the group consisting of H, F, Cl, methyl, cyano, and -C(O)NH₂;
R₂ is independently selected from the group consisting of H, halogen, C₁-C₃ alkyl, cyano, trifluoromethyl, amino, hydroxy, methoxy, C₁-C₃ alkyl-OC(O)- and C₁-C₃ alkyl-NHC(O)-, preferably selected from the group consisting of H, cyano, Cl, methyl and methoxy;
M represents O or S;
L is selected from the group consisting of -CH₂-CH₂-, -CH₂-, -O-, -S-, -NH- and -CO-, wherein either -CH₂- moiety in -CH₂-CH₂- is optionally substituted by -O-, -S-, -CO- or -NH-; preferably, L is selected from the group consisting of -CH₂-, -CH₂-CH₂-, -CH-O-, -O- and -CO-; and
E is selected from the group consisting of 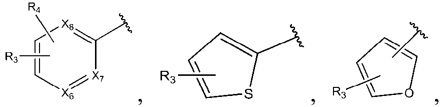 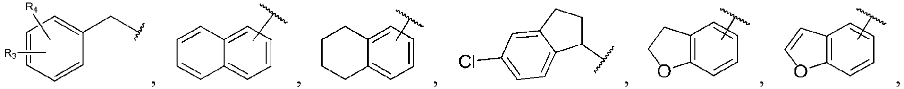 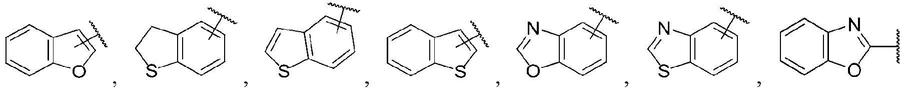 and 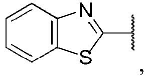
wherein, X₆, X₇ and X₈ are each independently selected from the group consisting of CH and N,
R₃ is independently selected from the group consisting of H, halogen, methyl, cyano, trifluoromethyl and nitro, preferably selected from the group consisting of H, F, Cl, Br, cyano, trifluoromethyl and nitro, and
R₄ is independently selected from the group consisting of H, halogen, methyl, cyano, trifluoromethyl and nitro, preferably selected from the group consisting of H, F, Cl, Br, cyano, trifluoromethyl and nitro.

2. The compound or the racemate, enantiomer, diastereoisomer, deuterated compound or pharmaceutically acceptable salt thereof according to claim 1, wherein E is selected from the group consisting of 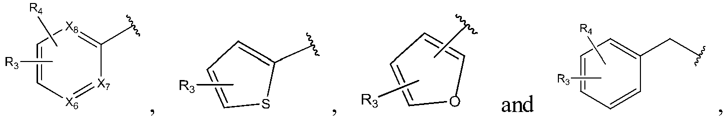 wherein X₆, X₇, X₈, R₃ and R₄ are as defined in claim 1.

3. The compound or the racemate, enantiomer, diastereoisomer, deuterated compound or pharmaceutically acceptable salt thereof according to claim 1 or 2, wherein the compound represented by formula (I) has a structure represented by formula (II): 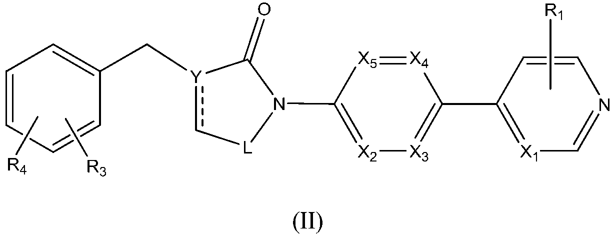 wherein X₁, X₂, X₃, X₄, X₅, L, Y, R₁, R₃ and R₄ are as defined in claim 1.

4. A compound or a racemate, enantiomer, diastereoisomer, deuterated compound or pharmaceutically acceptable salt thereof, wherein the compound is selected from the group consisting of

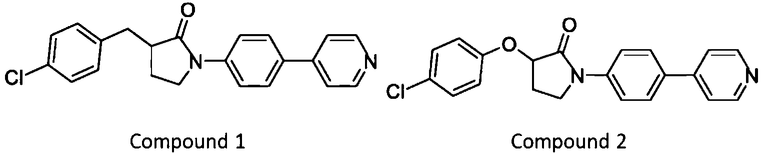

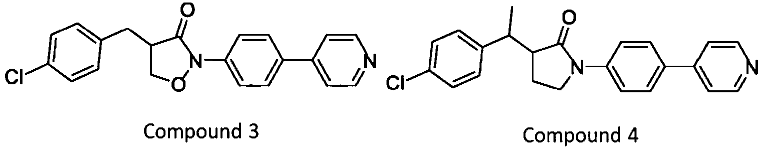

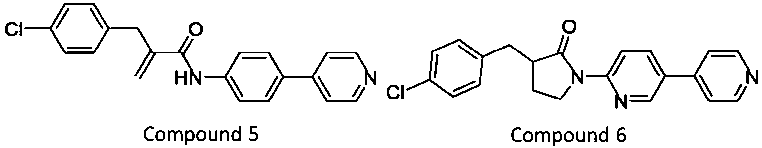

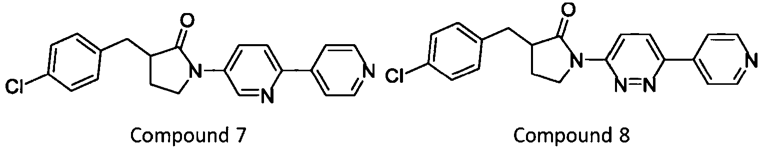

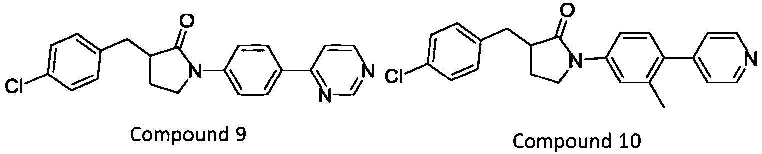

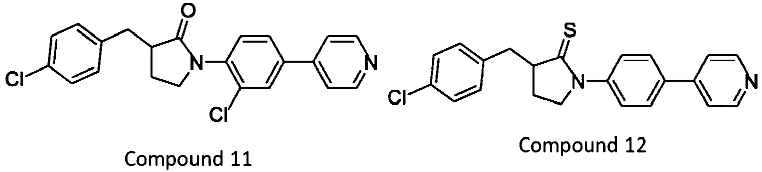

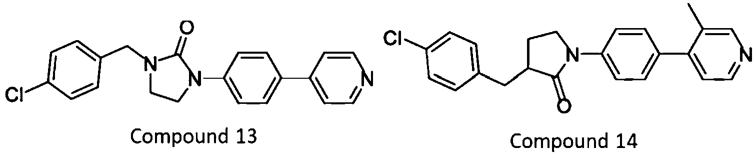

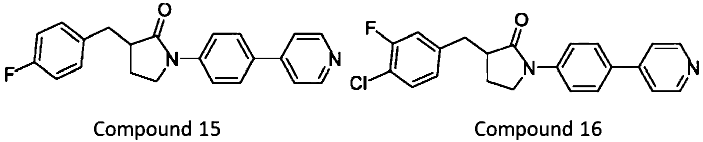

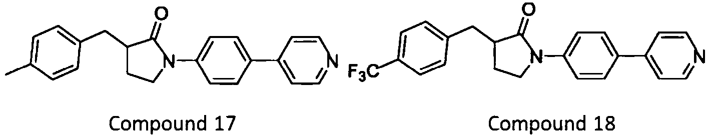

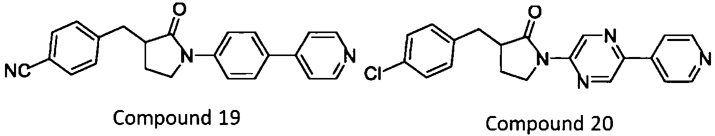

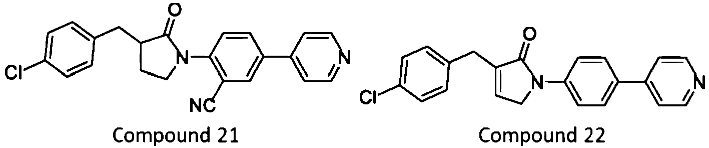

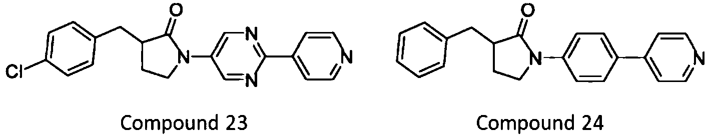

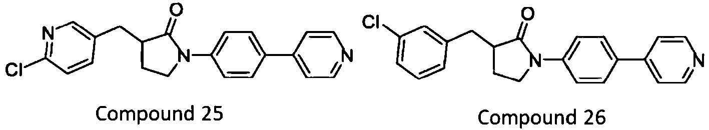

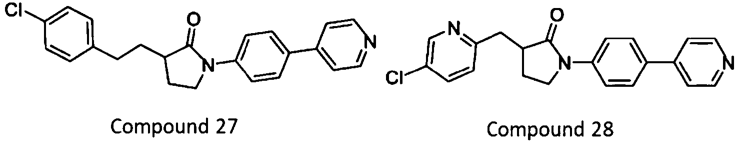

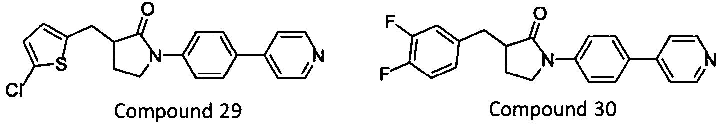

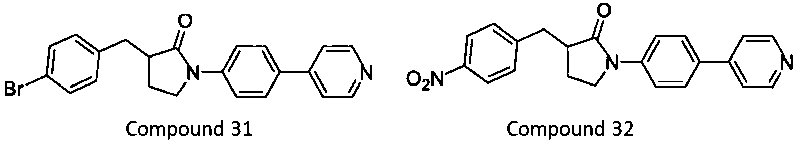

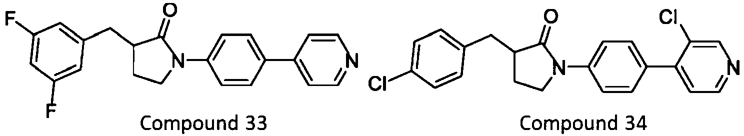

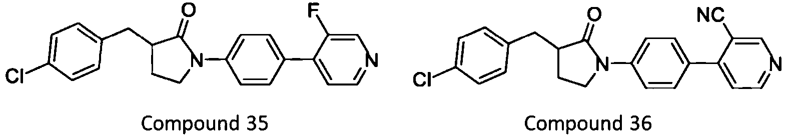

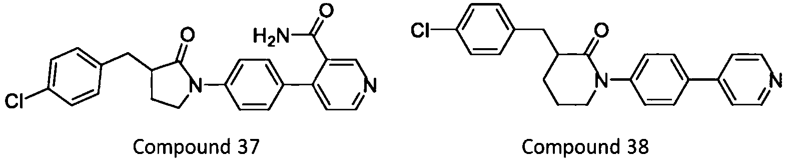

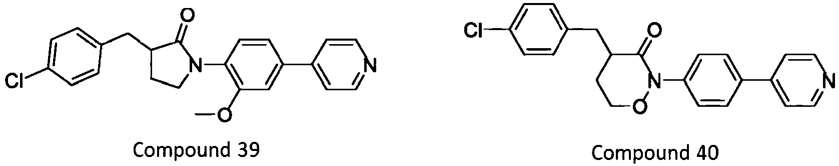

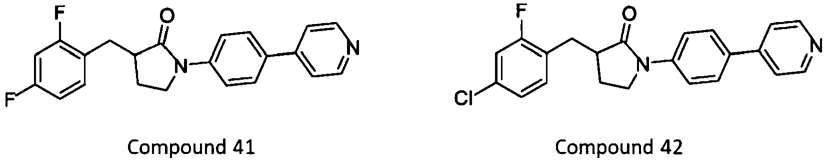

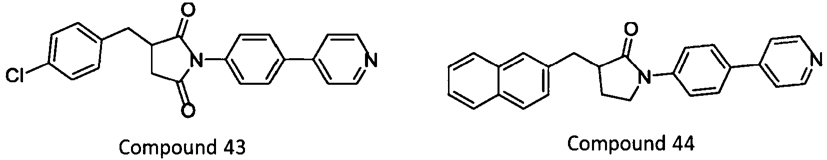

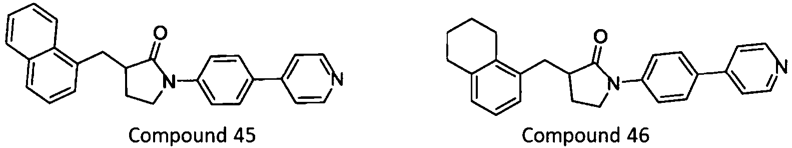

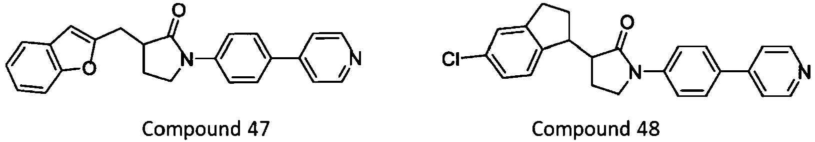

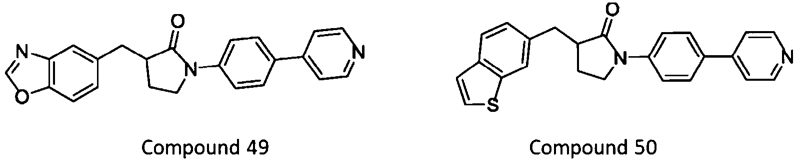

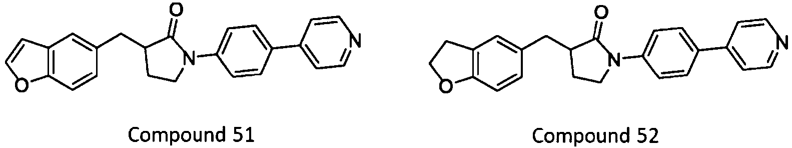

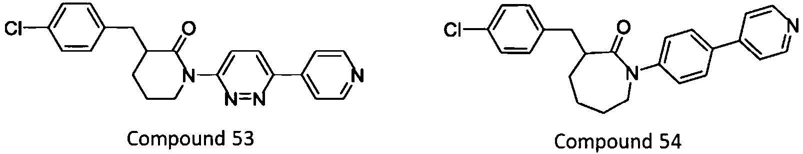

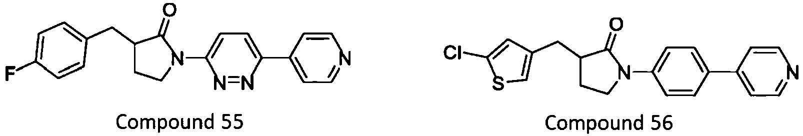

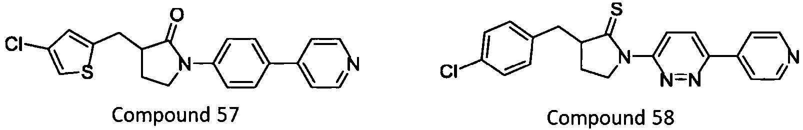

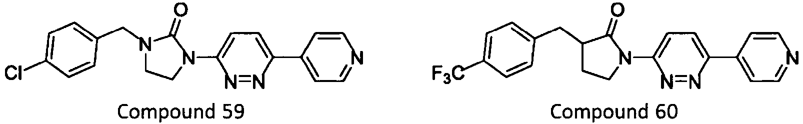

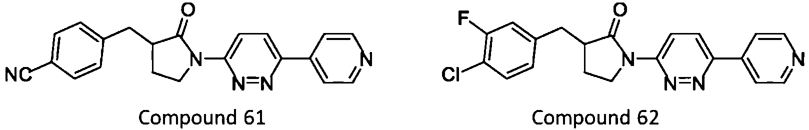

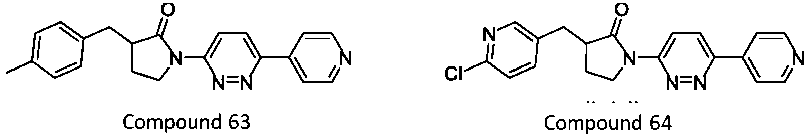

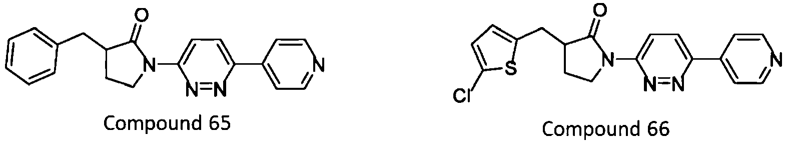

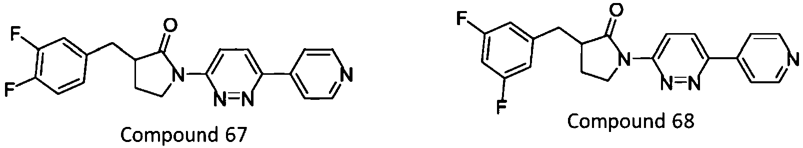

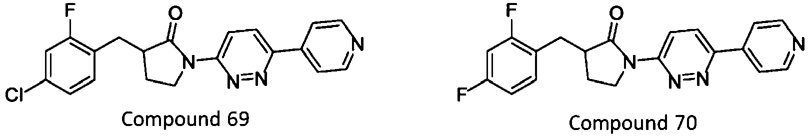

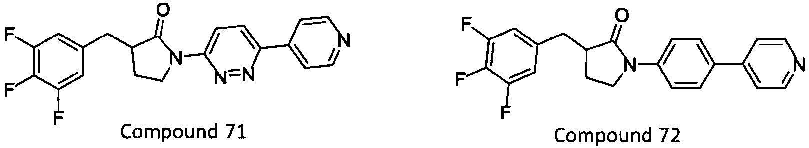

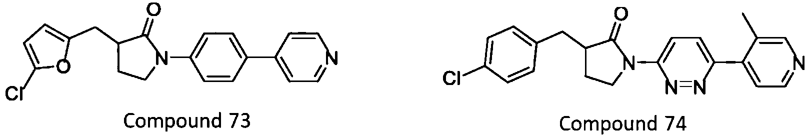

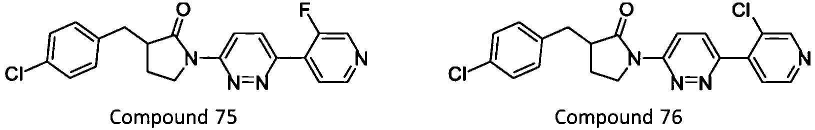

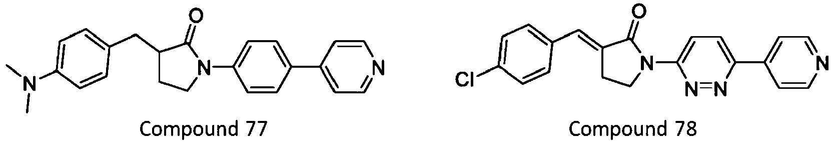

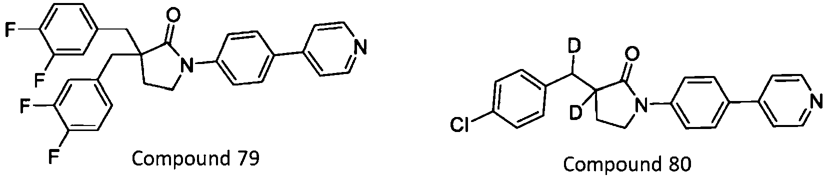

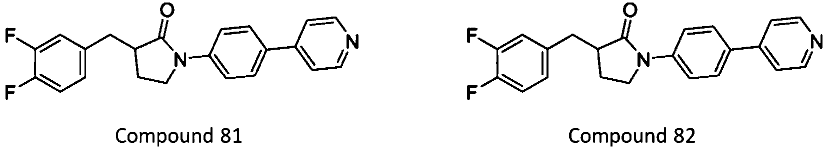

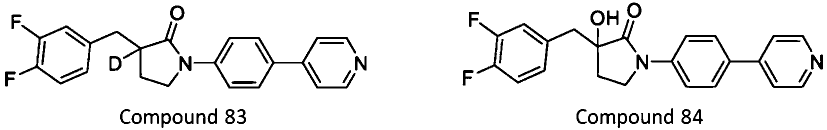

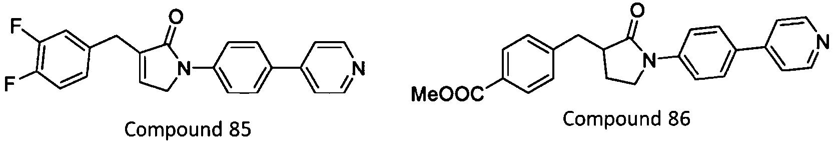

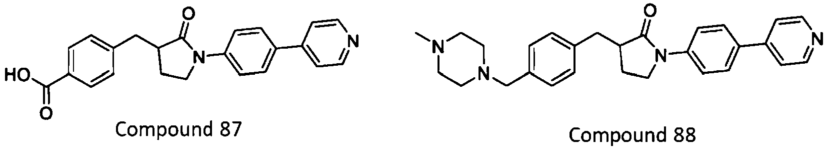

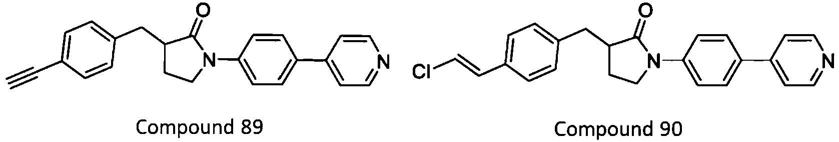

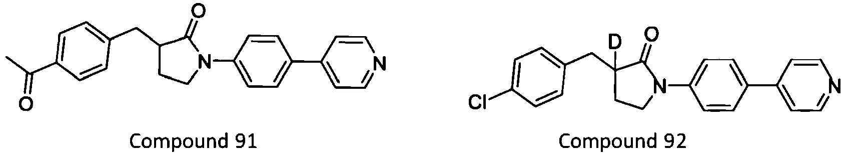

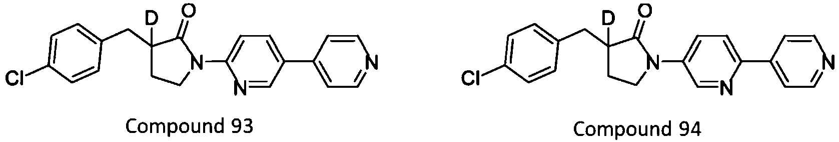

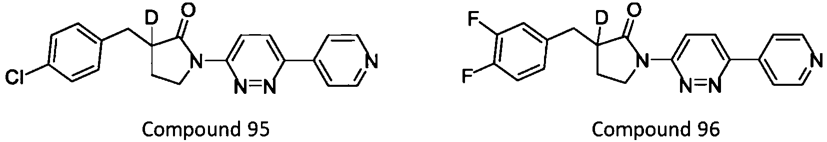

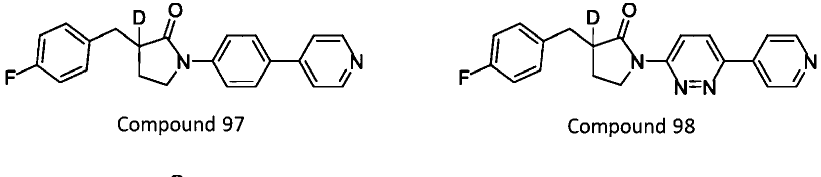

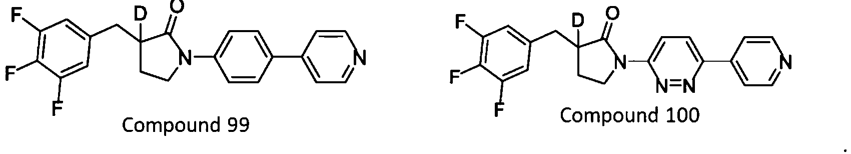

5. Use of the compound or the racemate, enantiomer, diastereoisomer, deuterated compound or pharmaceutically acceptable salt thereof according to any one of claims 1 to 4 in the manufacture of a medicament for treating or preventing a neurodegenerative disease or neurological disease or condition.

6. Use of the compound or the racemate, enantiomer, diastereoisomer, deuterated compound or pharmaceutically acceptable salt thereof according to any one of claims 1 to 5 in the manufacture of a SARM1 enzymatic activity inhibitor.

7. Use of the compound or the racemate, enantiomer, diastereoisomer, deuterated compound or pharmaceutically acceptable salt thereof according to any one of claims 1 to 6 in the manufacture of a medicament for treating or preventing an axonal degeneration-related disease or condition.

8. The use according to any one of claims 5 to 7, wherein the neurodegenerative disease, neurological disease or condition or axonal degeneration-related disease or condition is selected from the group consisting of Alzheimer's disease, Parkinson's disease, multiple sclerosis, amyotrophic lateral sclerosis and peripheral neuropathy.

9. A pharmaceutical composition comprising the compound or the racemate, enantiomer, diastereoisomer, deuterated compound or pharmaceutically acceptable salt thereof according to any one of claims 1 to 4, and a pharmaceutically acceptable carrier.
